# EUROPÄISCHE PATENTSCHRIFT

(11) **EP 4 031 097 B1**
(45) Veröffentlichungstag und Bekanntmachung des Hinweises auf die Patenterteilung: **07.01.2026**
(21) Anmeldenummer: 20737155.0
(22) Anmeldetag: 06.07.2020
(51) Int. Cl.: A61K 8/25, A61K 8/58, A61K 8/81, A61Q 5/00, A61Q 5/06, A61Q 5/08, A61Q 5/10, A61K 8/19, A61K 8/26, A61K 8/02

(54) **VERFAHREN ZUM FÄRBEN VON KERATINISCHEM MATERIAL, UMFASSEND DIE ANWENDUNG VON EINER SILICIUMORGANISCHEN VERBINDUNG, EINES BESCHICHTETEN EFFEKTPIGMENTS UND VERSIEGELUNGSREAGENZ I**
PROCESS FOR COLOURING KERATINOUS MATERIAL, COMPRISING THE USE OF AN ORGANOSILICON COMPOUND, AN EFFECT PIGMENT AND A FILM-FORMING POLYMER IV
PROCEDE DE COLORATION DE MATIERES KERATINIQUES, COMPRENANT L'UTILISATION D'UN COMPOSE ORGANOSILICONE, D'UN PIGMENT A EFFET ET D'UN POLYMERE FILMOGENE IV

(30) Priorität: 19.09.2019 DE 102019214286
(43) Veröffentlichungstag der Anmeldung: 27.07.2022
(73) Patentinhaber: Henkel AG & Co. KGaA, 40589 Düsseldorf (DE)
(72) Erfinder: LECHNER, Torsten, 40764 Langenfeld (DE)
(86) Internationale Anmeldenummer: PCT/EP2020/068978
(87) Internationale Veröffentlichungsnummer: WO 2021/052648

(56) Entgegenhaltungen:
- EP-A1- 3 081 601
- WO-A1-2018/130912
- WO-A2-2005/075578
- WO-A2-2013/068979
- US-A1- 2010 083 446
- US-A1- 2015 313 814

## Beschreibung

Gegenstand der vorliegenden Anmeldung ist ein Verfahren zum Behandeln von keratinischem Material, insbesondere menschlichen Haaren, welches die Anwendung von zwei Mitteln (a) und (b) umfasst. Das Mittel (a) ist durch seinen Gehalt an mindestens zwei organischen Siliciumverbindungen (a1) und mindestens einer ausgewählten farbgebenden Verbindung (a2), umfassend mindestens ein ausgewähltes Effektpigment gekennzeichnet. Das Mittel (b) enthält mindestens ein Versiegelungsreagenz.

Ein weiterer Gegenstand dieser Anmeldung ist eine Mehrkomponenten-Verpackungseinheit (Kit-ofparts) zum Färben von keratinischem Material, insbesondere menschlichen Haaren, welche getrennt voneinander konfektioniert mindestens drei Mittel (a'), (a") und (b) umfasst. Aus den Mitteln (a') und (a") kann das im oben beschriebenen Verfahren angewendete Mittel (a) hergestellt werden.

Die Veränderung von Form und Farbe von keratinischen Fasern, insbesondere von Haaren, stellt einen wichtigen Bereich der modernen Kosmetik dar. Zur Veränderung der Haarfarbe kennt der Fachmann je nach Anforderung an die Färbung diverse Färbesysteme. Für permanente, intensive Färbungen mit guten Echtheitseigenschaften und guter Grauabdeckung werden üblicherweise Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch sehr langanhaltende Färbeergebnisse aus.

Bei dem Einsatz von direktziehenden Farbstoffen diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkeit auf. Färbungen mit direktziehenden Farbstoffen verbleiben üblicherweise für einen Zeitraum zwischen 5 und 20 Haarwäschen auf dem Haar.

Für kurzzeitige Farbveränderungen auf dem Haar und/oder der Haut ist der Einsatz von Farbpigmenten bekannt. Unter Farbpigmenten werden im Allgemeinen unlösliche, farbgebende Substanzen verstanden. Diese liegen ungelöst in Form kleiner Partikel in der Färbeformulierung vor und lagern sich lediglich von außen auf den Haarfasern und/oder der Hautoberfläche ab. Daher lassen sie sich in der Regel durch einige Wäschen mit Tensid-haltigen Reinigungsmitteln wieder rückstandslos entfernen. Unter dem Namen Haar-Mascara sind verschiedene Produkte dieses Typs auf dem Markt erhältlich.

Wünscht sich der Anwender besonders langanhaltende Färbungen, so ist die Verwendung von oxidativen Färbemitteln bislang seine einzige Option. Doch trotz vielfacher Optimierungsversuche lässt sich bei der oxidativen Haarfärbung ein unangenehmer Ammoniakgeruch bzw. Amingeruch nicht vollständig vermeiden. Auch die mit dem Einsatz der oxidativen Färbemittel nach wie vor verbundene Haarschädigung wirkt sich auf das Haar des Anwenders nachteilig aus.

EP 2168633 B1 beschäftigt sich mit der Aufgabenstellung, langanhaltende Haarfärbungen unter Einsatz von Pigmenten zu erzeugen. Die Schrift lehrt, dass sich bei Verwendung der Kombination aus einem Pigment, einer organischer Siliciumverbindung, einem filmbildenden Polymer und einem Lösungsmittel auf Haaren Färbungen erzeugen lassen, die gegenüber Shampoonierungen besonders widerstandsfähig sind.

Aus der WO 2018/130912 A1 ist ein Verfahren zum Beschichten von Haar bekannt, wobei in dem Verfahren eine Öl-in-Wasser-Emulsion auf Haar aufgetragen wird, die (A) eine wässrige Phase und (B) eine Ölphase, die mindestens ein reaktives, kondensationshärtbares, filmbildendes Aminosilikon-Prepolymer enthält, eingesetzt wird
Metallische Glanzpigmente oder Metalleffektpigmente finden breite Anwendung in vielen Bereichen der Technik. Sie werden beispielsweise zur Einfärbung von Lacken, Druckfarben, Tinten, Kunststoffen, Gläsern keramischen Produkten und Zubereitungen der dekorativen Kosmetik wie Nagellack eingesetzt. Sie zeichnen sich vor allem durch ihren reizvollen winkelabhängigen Farbeindruck (Goniochromie) und ihren metallartig wirkenden Glanz aus.

Haare mit einem Metallic Finish oder metallischen Reflexen sind im Trend. Durch den Metallic-Ton wirkt das Haar dicker und glänzender.

Es besteht Bedarf, Haarfärbungen mit Effektpigmenten bereitzustellen, die einerseits eine hohe Wasch- und Reibechtheit und andererseits die Haareigenschaften wie Handhabbarkeit und Haptik nicht negativ beeinträchtigen. Dazu wäre es wünschenswert, wenn die eingesetzten Effektpigmente eine hohe Deckkraft aufwiesen und in dünnen Schichten auf dem Haar aufgebracht werden könnten.

Entsprechend war die Aufgabe der vorliegenden Erfindung, ein Färbesystem mit Effektpigmenten bereitzustellen, das mit der oxidativen Färbung vergleichbare Echtheitseigenschaften besitzt. Insbesondere die Waschechtheiten sollten herausragend sein, hierbei sollte jedoch auf den Einsatz der sonst zu diesem Zweck üblicherweise eingesetzten Oxidationsfarbstoffvorprodukte verzichtet werden.

Überraschenderweise hat sich nun herausgestellt, dass die vorgenannte Aufgabe hervorragend gelöst werden kann, wenn keratinische Materialien, insbesondere menschliche Haare, mit einem Verfahren gefärbt werden, bei welchem mindestens zwei Mittel (a) und (b) auf die keratinischen Materialien (Haare) appliziert werden. Hierbei enthält das erste Mittel (a) mindestens zwei ausgewählte organische Siliciumverbindungen und weiterhin mindestens eine ausgewählte farbgebende Verbindung. Im Mittel (a) sind damit die organische Siliciumverbindung und die farbgebende Verbindung zusammen konfektioniert. Das zweite Mittel (b) enthält mindestens ein Versiegelungsreagenz.

Bei Einsatz der zwei Mittel (a) und (b) in einem Färbeverfahren konnte keratinisches Material in besonders hoher Farbintensität und hohen Echtheiten gefärbt werden.

Ein erster Gegenstand der vorliegenden Erfindung ist ein Verfahren zum Färben von keratinischem Material, insbesondere menschlichen Haaren, umfassend die folgenden Schritte:
- Anwendung eines Mittels (a) auf dem keratinischem Material, wobei das Mittel (a) enthält:
   (a1) mindestens eine organische Siliciumverbindung der Formel (I) und mindestens eine organische Siliciumverbindung der Formel (IV),
   wobei in der organischen Siliciumverbindung der Formel (I)

      R₁R₂N-L-Si(OR₃)ₐ(R₄)_{b} (I),

      - R₁, R₂ unabhängig voneinander für ein Wasserstoffatom oder eine C₁-C₆-Alkylgruppe stehen,
      - L für eine lineare oder verzweigte, zweiwertige C₁-C₂₀-Alkylengruppe steht,
      - R₃ für ein Wasserstoffatom oder für eine C₁-C₆-Alkylgruppe steht,
      - R₄ für eine C₁-C₆-Alkylgruppe steht,
      - a, für eine ganze Zahl von 1 bis 3 steht, und
      - b für die ganze Zahl 3 - a steht, und
   wobei in der organischen Siliciumverbindung der Formel (IV)

      R₉Si(OR₁₀)ₖ(R₁₁)ₘ (IV),

      - R₉ für eine C₁-C₁₈-Alkylgruppe steht,
      - R₁₀ für ein Wasserstoffatom oder eine C₁-C₆-Alkylgruppe steht,
      - R₁₁ für eine C₁-C₆-Alkylgruppe steht
      - k für eine ganze Zahl von 1 bis 3 steht, und
      - m für die ganze Zahl 3 - k steht, und
   (a2) mindestens eine farbgebende Verbindung, enthaltend mindestens ein Effektpigment, umfassend α) ein Substratplättchen und β) eine Beschichtung,
   wobei die Beschichtung mindestens eine Schicht aufweist, die nass-chemisch unter Verwendung eines Metallalkoxids und einer siliciumorganischen Verbindung mit einer basischen Gruppe hergestellt worden ist, und
- Anwendung eines Mittels (b) auf dem keratinischen Material, wobei das Mittel (b) enthält:
   (b1) mindestens ein Versiegelungsreagenz, umfassend eine Verbindung ausgewählt aus der Gruppe bestehend aus filmbildenden Polymeren, Alkalisierungsmitteln, Acidifizierungsmittel und Mischungen daraus.

Bei den zu dieser Erfindung führenden Arbeiten hat sich herausgestellt, dass die bevorzugt sukzessive Anwendung der Mittel (a) und (b) die Erzeugung von sehr stabilen und waschechten Färbungen auf den keratinischen Materialien ermöglicht. Ohne auf diese Theorie eingeschränkt zu sein, wird in diesem Zusammenhang vermutet, dass die gemeinsame Anwendung von organischen Siliciumverbindungen (a1) und farbgebender Verbindung (a2) zur Ausbildung eines besonders resistenten ersten Films auf dem keratinischen Material führt. Mit Applikation des zweiten Mittels (b) wird die erste Schicht versiegelt. So lagert sich beispielsweise auf dieser ersten Schicht nun ein filmbildendes Polymer als Versiegelungsreagenz (b1) in Form eines weiteren Films ab.

Durch diese spezielle Art der Konfektionierung - d.h. die gemeinsame Anwendung von Silanen (a1) und farbgebender Verbindung (a2) und davon separierter Anwendung des Versiegelungsreagenz (b1) - wies das auf diese Weise erzeugte Filmsystem eine verbesserte Widerstandsfähigkeit gegenüber äußeren Einflüssen auf. Die farbgebenden Verbindungen (a2) wurden auf diesem Wege dauerhaft auf dem keratinischen Material fixiert, so dass überaus waschechte Färbungen mit guter Resistenz gegenüber Shampoonierungen erhalten werden konnten.

Durch die spezielle Beschichtung des Effektpigments wird die Affinität des Effektpigments zu dem durch die organischen Siliciumverbindungen (a1) gebildeten ersten Film und dem keratinischen Material deutlich erhöht.

### Keratinisches Material

Unter keratinischem Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter keratinischem Material das menschliche Haar, die menschliche Haut und menschliche Nägel, insbesondere Finger- und Fußnägel, verstanden. Ganz besonders bevorzugt wird unter keratinischem Material das menschliche Haar verstanden.

### Mittel (a) und (b)

Im Rahmen des erfindungsgemäßen Verfahrens werden die Mittel (a) und (b) auf dem keratinischen Material, insbesondere den menschlichen Haaren, appliziert. Die zwei Mittel (a) und (b) sind voneinander verschieden.

Mit anderen Worten ist ein erster Gegenstand der vorliegenden Erfindung ein Verfahren zum Behandeln von keratinischem Material, insbesondere menschlichen Haaren, umfassend die folgenden Schritte:
Anwendung eines Mittels (a) auf dem keratinischem Material, wobei das Mittel (a) enthält:
(a1) mindestens eine organische Siliciumverbindung
der Formel (I) und mindestens eine organische Siliciumverbindung der Formel (IV),
wobei in der organischen Siliciumverbindung der Formel (I)

   R₁R₂N-L-Si(OR₃)ₐ(R₄)_{b} (I),

   - R₁, R₂ unabhängig voneinander für ein Wasserstoffatom oder eine C₁-C₆-Alkylgruppe stehen,
   - L für eine lineare oder verzweigte, zweiwertige C₁-C₂₀-Alkylengruppe steht,
   - R₃ für ein Wasserstoffatom oder für eine C₁-C₆-Alkylgruppe steht,
   - R₄ für eine C₁-C₆-Alkylgruppe steht,
   - a, für eine ganze Zahl von 1 bis 3 steht, und
   - b für die ganze Zahl 3 - a steht, und
wobei in der organischen Siliciumverbindung der Formel (IV)

   R₉Si(OR₁₀)ₖ(R₁₁)ₘ (IV),

   - R₉ für eine C₁-C₁₈-Alkylgruppe steht,
   - R₁₀ für ein Wasserstoffatom oder eine C₁-C₆-Alkylgruppe steht,
   - R₁₁ für eine C₁-C₆-Alkylgruppe steht
   - k für eine ganze Zahl von 1 bis 3 steht, und
   - m für die ganze Zahl 3 - k steht, und
(a2) mindestens eine farbgebende Verbindung, enthaltend mindestens ein Effektpigment, umfassend α) ein Substratplättchen und β) eine Beschichtung,
wobei die Beschichtung mindestens eine Schicht aufweist, die nass-chemisch unter Verwendung eines Metallalkoxids und einer siliciumorganischen Verbindung mit einer basischen Gruppe hergestellt worden ist, und
Anwendung eines Mittels (b) auf dem keratinischen Material, wobei das Mittel (b) enthält:
   (b1) mindestens ein Versiegelungsreagenz, umfassend eine Verbindung ausgewählt aus der Gruppe bestehend aus filmbildenden Polymeren, Alkalisierungsmitteln, Acidifizierungsmittel und Mischungen daraus.

### Mittel (a)

Bevorzugt enthält das Mittel (a) die erfindungswesentlichen Inhaltsstoffe (a1) und (a2) in einem kosmetischen Träger, besonders bevorzugt in einem wässrigen oder wässrig-alkoholischen kosmetischen Träger. Dieser kosmetische Träger kann flüssig, gelartig oder cremeförmig sein. Auch pastöse, feste oder pulverförmige kosmetische Träger können für die Herstellung des Mittels (a) verwendet werden. Zum Zwecke der Haarbehandlung, insbesondere Haarfärbung, sind solche Träger beispielsweise Cremes, Emulsionen, Gele oder auch Tensid-haltige schäumende Lösungen, wie beispielsweise Shampoos, Schaumaerosole, Schaum-formulierungen oder andere Zubereitungen, die für die Anwendung auf dem Haar geeignet sind.

Bevorzugt enthält der kosmetische Träger - bezogen auf sein Gewicht - mindestens 2 Gew.-% Wasser. Weiter bevorzugt liegt der Wassergehalt oberhalb von 10 Gew.-%, noch weiter bevorzugt oberhalb von 20 Gew.-% und besonders bevorzugt oberhalb von 40 Gew.-%. Der kosmetische Träger kann auch wässrig-alkoholisch sein. Unter wässrig-alkoholischen Lösungen sind im Sinne der vorliegenden Erfindung wässrige Lösungen enthaltend 2 bis 70 Gew.-% eines C₁-C₄-Alkohols, insbesondere Ethanol bzw. Isopropanol, zu verstehen. Die erfindungsgemäßen Mittel können zusätzlich weitere organische Lösemittel, wie beispielsweise Methoxybutanol, Benzylalkohol, Ethyldiglykol oder 1,2-Propylenglykol, enthalten. Bevorzugt sind dabei alle wasserlöslichen organischen Lösemittel.

### Organische Siliciumverbindungen aus der Gruppe der Silane (a1)

Als erfindungswesentlichen Inhaltsstoff (a1) enthält das Mittel (a) mindestens zwei ausgewählte organische Siliciumverbindungen.

Diese im Mittel (a) enthaltenen organischen Siliciumverbindungen (a1) bzw. organischen Silane sind reaktive Verbindungen.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das SiliciumAtom geknüpft ist.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die Wasserstoff-Atome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Das Mittel (a) enthält mindestens eine organische Siliciumverbindung (a1) der Formel (I).

In einer weiteren ganz besonders bevorzugten Ausführungsform ist das Verfahren dadurch gekennzeichnet, dass auf dem keratinischen Material (oder den menschlichen Haaren) ein Mittel angewendet wird, wobei das Mittel (a) mindestens eine organische Siliciumverbindung (a) der Formel (I) enthält,

R₁R₂N-L-Si(OR₃)ₐ(R₄)_{b} (I),

wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder eine C₁-C₆-Alkylgruppe steht,
- R₄ für eine C₁-C₆-Alkylgruppe steht
- a, für eine ganze Zahl von 1 bis 3 steht, und
- b für die ganze Zahl 3 - a steht.

Die Substituenten R₁, R₂, R₃, R₄ und Lin den Verbindungen der Formel (I) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweiwertige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweiwertige Alkylengruppen auch verzweigt sein. Beispiele für verzweigte, zweiwertige C₃-C₂₀-Alkylengruppen sind (-CH₂-CH(CH₃)-) und (-CH₂-CH(CH₃)-CH₂-).

In den organischen Siliciumverbindungen der Formel (I)

R₁R₂N-L-Si(OR₃)ₐ(R₄)_{b} (I),

stehen die Reste R₁ und R₂ unabhängig voneinander für ein Wasserstoffatom oder eine C₁-C₆-Alkylgruppe. Ganz besonders bevorzugt stehen die Reste R₁ und R₂ beide für ein Wasserstoffatom.

Im Mittelteil der organischen Siliciumverbindung befindet sich die Struktureinheit oder der Linker -L-der für eine lineare oder verzweigte, zweiwertige C₁-C₂₀-Alkylengruppe steht.

Eine zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung L zwei Bindungen eingehen kann. Eine Bindung erfolgt von der Aminogruppe R1R2N zum Linker L, und die zweite Bindung besteht zwischen dem Linker L und dem Siliciumatom.

Bevorzugt steht -L- für eine lineare, zweiwertige (d.h. divalente) C₁-C₂₀-Alkylengruppe. Weiter bevorzugt steht -L- für eine lineare zweiwertige C₁-C₆-Alkylengruppe. Besonders bevorzugt steht -L-für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt steht L für eine Propylengruppe (-CH₂-CH₂-CH₂-).

Die lineare Propylengruppe (-CH₂-CH₂-CH₂-) kann alternativ auch als Propan-1,3-diylgruppe bezeichnet werden.

Die organischen Siliciumverbindungen der Formel (I)

R₁R₂N-L-Si(OR₃)ₐ(R₄)_{b} (I),

tragen jeweils ein einem Ende die Silicium-haltige Gruppierung -Si(OR₃)ₐ(R₄)_{b}.

In der endständigen Struktureinheit -Si(OR₃)ₐ(R₄)_{b} steht der Rest R₃ für ein Wasserstoffatom oder eine C₁-C₆-Alkylgruppe, und der Rest R₄ steht für eine C₁-C₆-Alkylgruppe. Besonders bevorzugt stehen R₃ und R₄ unabhängig voneinander für eine Methylgruppe oder eine Ethylgruppe.

Hierbei steht a für eine ganze Zahl von 1 bis 3, und b steht für die ganze Zahl 3 - a. Wenn a für die Zahl 3 steht, dann ist b gleich 0. Wenn a für die Zahl 2 steht, dann ist b gleich 1. Wenn a für die Zahl 1 steht, dann ist b gleich 2.

Besonders widerstandsfähige Filme konnten erzeugt werden, wenn das Mittel (a) mindestens eine organische Siliciumverbindung (a1) der Formel (I) enthält, in welcher die Reste R₃, R₄ unabhängig voneinander für eine Methylgruppe oder für eine Ethylgruppe stehen.

Bei Einsatz des Verfahrens zum Färben von keratinischen Material konnten analog dann Färbungen mit den besten Waschechtheiten erhalten werden, wenn das Mittel (a) mindestens eine organische Siliciumverbindung der Formel (I) enthält, in welcher die Reste R₃, R₄ unabhängig voneinander für eine Methylgruppe oder für eine Ethylgruppe stehen.

Weiterhin konnten Färbungen mit den besten Waschechtheiten erhalten werden, wenn das Mittel (a) mindestens eine organische Siliciumverbindung der Formel (I) enthält, in welcher der Rest a für die Zahl 3 steht. In diesem Fall steht der Rest b für die Zahl 0.

In einer weiteren bevorzugten Ausführungsform ist das in dem Verfahren eingesetzte Mittel (a) dadurch gekennzeichnet, dass es mindestens eine organische Siliciumverbindung (a1) der Formel (I) enthält, wobei
- R₃, R₄ unabhängig voneinander für eine Methylgruppe oder für eine Ethylgruppe stehen und
- a für die Zahl 3 steht und
- b für die Zahl 0 steht.

In einer weiteren bevorzugten Ausführungsform ist ein Verfahren dadurch gekennzeichnet, dass das Mittel (a) mindestens eine organische Siliciumverbindung (a1) der Formel (I) enthält,

R₁R₂N-L-Si(OR₃)ₐ(R₄)_{b} (I),

wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃ für ein Wasserstoffatom, eine Ethylgruppe oder eine Methylgruppe steht,
- R₄ für eine Methylgruppe oder für eine Ethylgruppe steht,
- a für die Zahl 3 steht und
- b für die Zahl 0 steht.

Zur Lösung der erfindungsgemäßen Aufgabenstellung besonders gut geeignete organische Siliciumverbindungen der Formel (I) sind
- (3-Aminopropyl)triethoxysilan 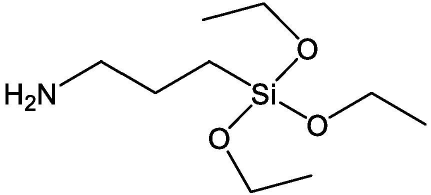
- (3-Aminopropyl)trimethoxysilan 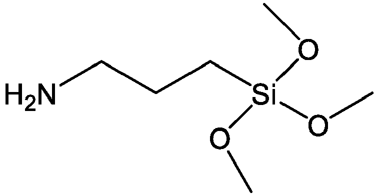
- 1-(3-Aminopropyl)silantriol 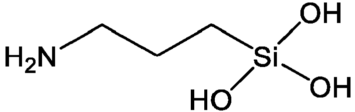
- (2-Aminoethyl)triethoxysilan 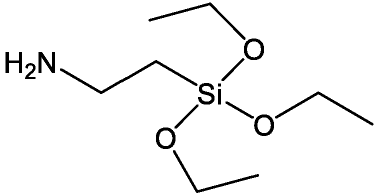
- (2-Aminoethyl)trimethoxysilan 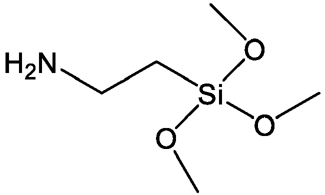 1-(2-Aminoethyl)silantriol 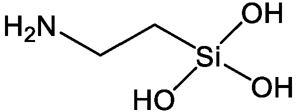
- (3-Dimethylaminopropyl)triethoxysilan 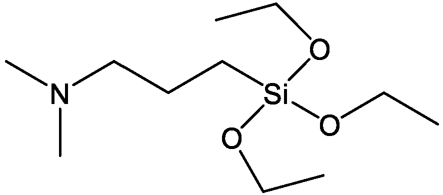
- (3-Dimethylaminopropyl)trimethoxysilan 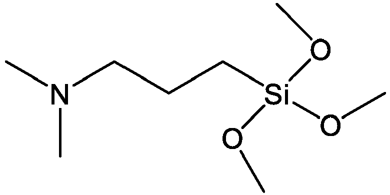 1-(3-Dimethylaminopropyl)silantriol 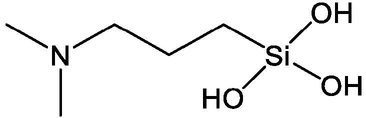
- (2-Dimethylaminoethyl)triethoxysilan. 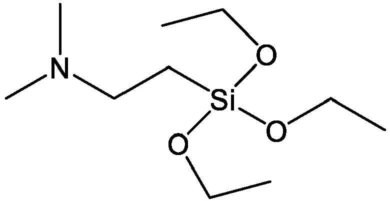
- (2-Dimethylaminoethyl)trimethoxysilan und 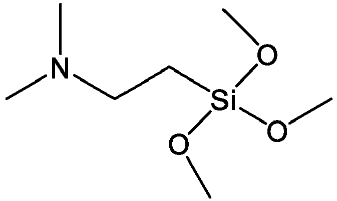 1-(2-Dimethylaminoethyl)silantriol

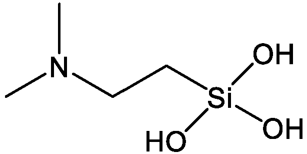

In einer weiteren bevorzugten Ausführungsform ist ein Verfahren dadurch gekennzeichnet, dass das Mittel (a) mindestens eine organische Siliciumverbindung (a1) enthält, die ausgewählt ist aus der Gruppe aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan.
- (2-Dimethylaminoethyl)trimethoxysilan und/oder
- 1-(2-Dimethylaminoethyl)silantriol.

Die vorgenannten organischen Siliciumverbindungen der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erwerblich.

Im Rahmen einer weiteren Ausführungsform enthält das Mittel mindestens eine organische Siliciumverbindung (a1) der Formel (II)

(R₅O)_{c}(R₆)_{d}Si-(A)ₑ-[NR₇-(A')]_{f}-[O-(A")]_{g}-[NR₈-(A‴)]ₕ-Si(Rₑ')_{d'}(OR₅')_{c'} (II).

Die siliciumorganischen Verbindungen der Formel (II) tragen jeweils an ihren beiden Enden die Silicium-haltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NR₈-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organischen Siliciumverbindung der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Filme mit der höchsten Stabilität bzw. Färbungen mit den besten Waschechtheiten konnten erhalten werden, wenn die Reste c und c' beide für die Zahl 3 stehen. In diesem Fall stehen d und d' beide für die Zahl 0.

In einer weiteren bevorzugten Ausführungsform ist ein Verfahren dadurch gekennzeichnet, dass das Mittel (a) mindestens eine organische Siliciumverbindung (a1) der Formel (II) enthält,

(R₅O)_{c}(R₆)_{d}Si-(A)ₑ-[NR₇-(A')]_{f}-[O-(A")]_{g}-[NR₈-(A‴)]ₕ-Si(Rₑ')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die erfindungsgemäßen organischen Siliciumverbindung der Formel (IIa)

(R₅O)₃Si-(A)ₑ-[NR₇-(A')]_{f}-[O-(A")]_{g}-[NR₈-(A‴)]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ-sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der Waschechtheit erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die erfindungsgemäßen organischen Siliciumverbindung der Formel (IIb)

(R₅O)_{c}(R₆)_{d}Si-(A)-[NR₇-(A')]-Si(R₆')_{d'}(OR₅')_{c'} (IIb).

Die Reste A, A', A", A‴ und Aʺʺ stehen unabhängig voneinander für eine lineare oder verzweigte, zweiwertige C₁-C₂₀-Alkylengruppe. Bevorzugt stehen die Reste A, A', A", A‴ und A"" unabhängig voneinander für eine lineare, zweiwertige C₁-C₂₀-Alkylengruppe. Weiter bevorzugt stehen die Reste A, A', A", A‴ und A"" unabhängig voneinander für eine lineare zweiwertige C₁-C₆-Alkylengruppe. Besonders bevorzugt stehen die Reste A, A', A", A‴ und A"" unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und A"" für eine Propylengruppe (-CH₂-CH₂-CH₂-).

Die zweiwertige C₁-C₂₀-Alkylengruppe kann alternativ auch als eine divalente oder zweibindige C₁-C₂₀-Alkylengruppe bezeichnet werden, womit gemeint ist, dass jede Gruppierung A, A', A", A‴ und A"" zwei Bindungen eingehen kann.

Die lineare Propylengruppe (-CH₂-CH₂-CH₂-) kann alternativ auch als Propan-1,3-diylgruppe bezeichnet werden.

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III)

- (Aʺʺ)-Si(R₆'')_{d}''(OR₅'')_{c}'' (III).

Ganz besonders bevorzugt stehen die Reste R7 und R8 unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung die Gruppierung [NR₇-(A')]_{,} aber nicht die Gruppierung -[NR₈-(A‴)]. Steht nun der Rest R7 für eine Gruppierung der Formel (III), so enthält das Mittel (a) eine organische Siliciumverbindung mit 3 reaktiven Silan-Gruppen.

In einer weiteren bevorzugten Ausführungsform ist ein Verfahren dadurch gekennzeichnet, dass das Mittel (a) mindestens eine organische Siliciumverbindung (a1) der Formel (II) enthält,

(R₅O)_{c}(R₆)_{d}Si-(A)ₑ-[NR₇-(A')]_{f}-[O-(A")]_{g}-[NR₈-(A‴)]ₕ-Si(Rₑ')_{d'}(OR₅')_{c'} (II),

wobei
- e und f beide für die Zahl 1 stehen,
- g und h beide für die Zahl 0 stehen,
- A und A' unabhängig voneinander für eine lineare, zweiwertige C₁-C₆-Alkylengruppe stehen und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform ist ein Verfahren dadurch gekennzeichnet, dass das Mittel (a) mindestens eine organische Siliciumverbindung der Formel (II) enthält, wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen,
   und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der erfindungsgemäßen Aufgabenstellung gut geeignete organische Siliciumverbindungen der Formel (II) sind
- 3-(Trimethoxysilyl)-N-[3-(trimethoxysilyl)propyl]-1-propanamin 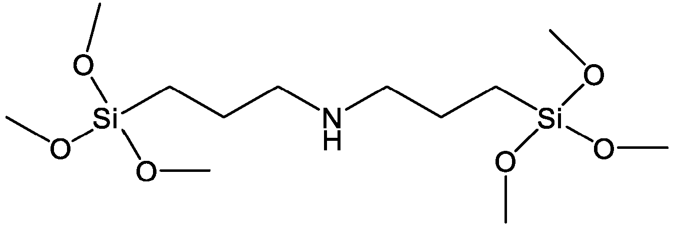
- 3-(Triethoxysilyl)-N-[3-(triethoxysilyl)propyl]-1-propanamin 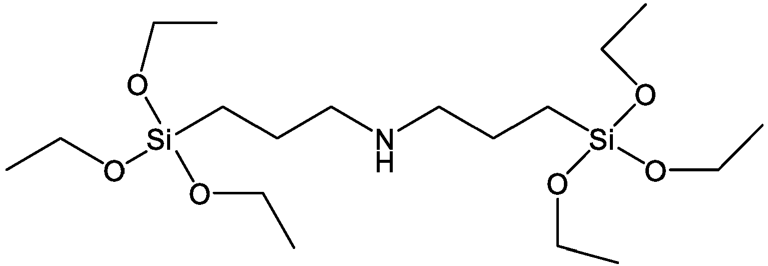
- N-Methyl-3-(trimethoxysilyl)-N-[3-(trimethoxysilyl)propyl]-1-propanamin 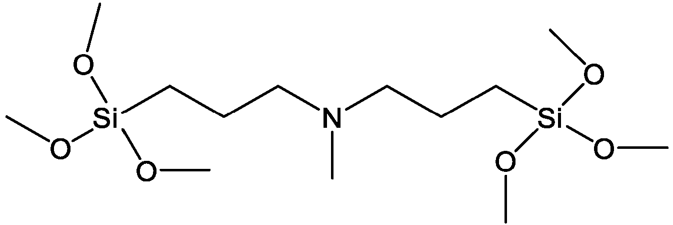
- N-Methyl-3-(triethoxysilyl)-N-[3-(triethoxysilyl)propyl]-1-propanamin 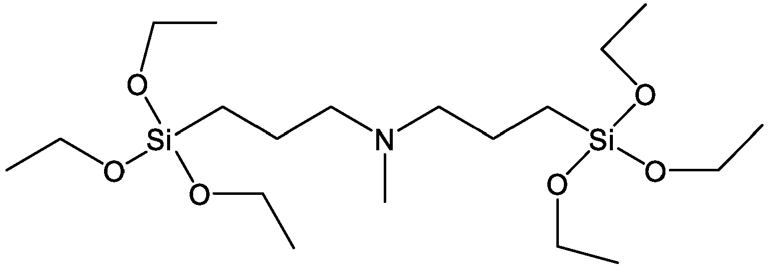
- 2-[Bis[3-(trimethoxysilyl)propyl]amino]-ethanol 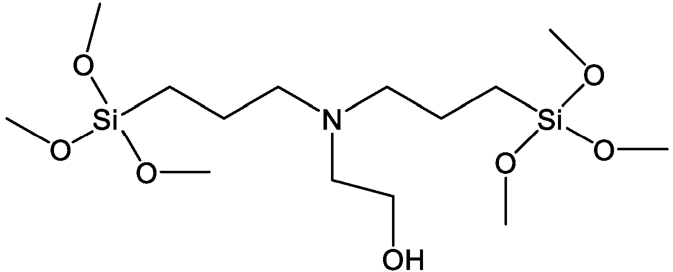
- 2-[Bis[3-(triethoxysilyl)propyl]amino]-ethanol 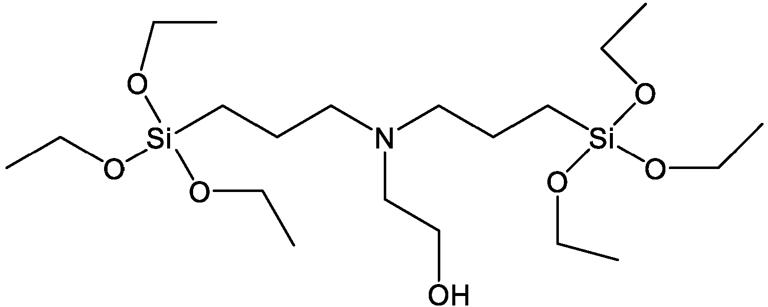
- 3-(Trimethoxysilyl)-N,N-bis[3-(trimethoxysilyl)propyl]-1-propanamin 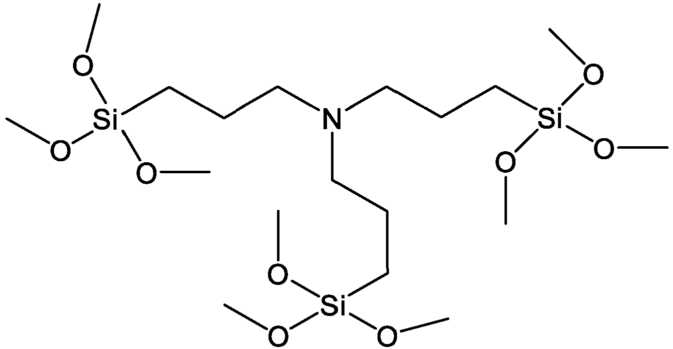
- 3-(Triethoxysilyl)-N,N-bis[3-(triethoxysilyl)propyl]-1-propanamin 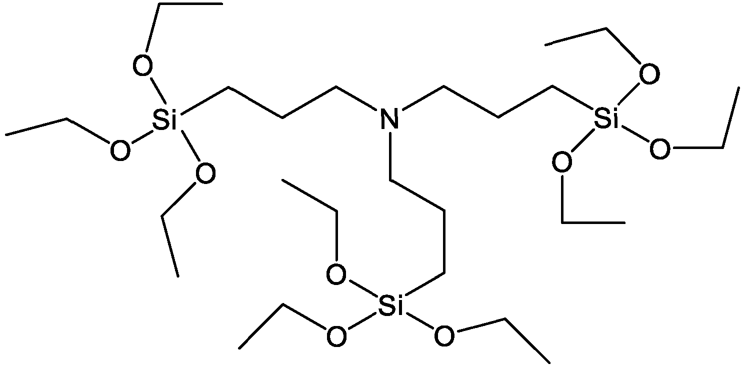
- N1,N1-Bis[3-(trimethoxysilyl)propyl]-1,2-ethanediamin, 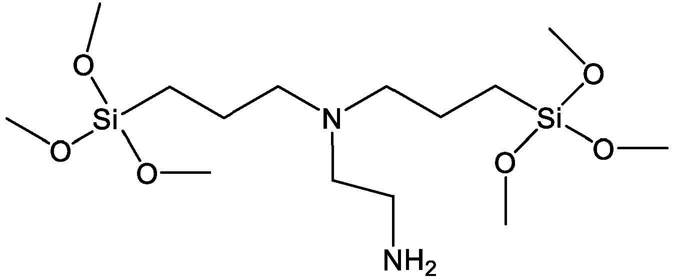
- N1,N1-Bis[3-(triethoxysilyl)propyl]-1,2-ethanediamin, 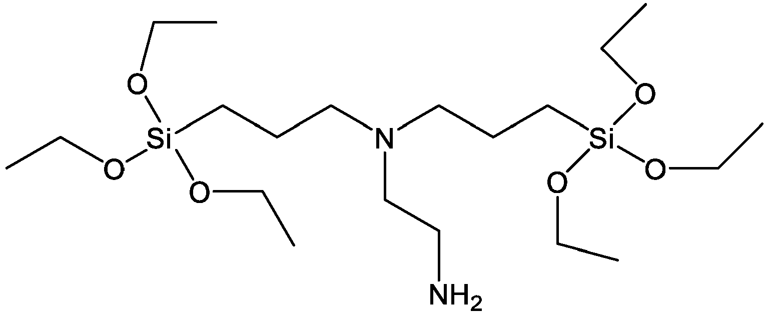
- N,N-Bis[3-(trimethoxysilyl)propyl]-2-propen-1-amin 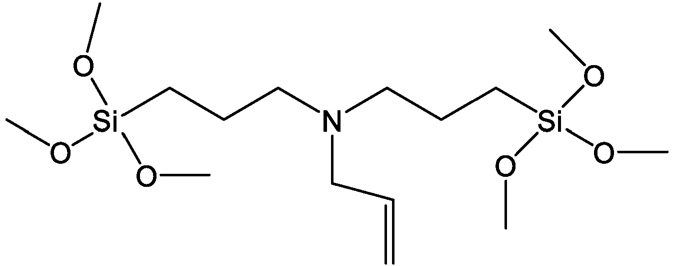
- N,N-Bis[3-(triethoxysilyl)propyl]-2-propen-1-amin

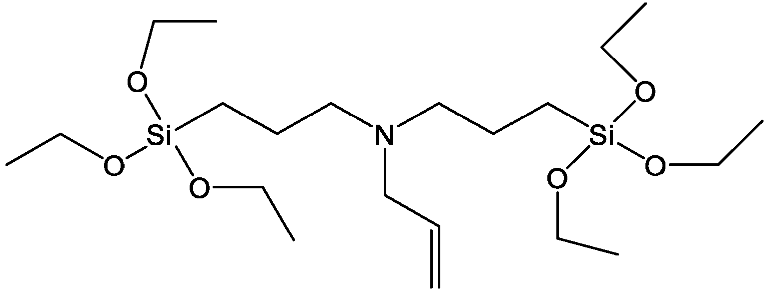

Die vorgenannten organische Siliciumverbindung der Formel (II) sind kommerziell erhältlich. Bis(trimethoxysilylpropyl)amine mit der CAS-Nummer 82985-35-1 kann beispielsweise von Sigma-Aldrich käuflich erworben werden.

Bis[3-(triethoxysilyl)propyl]amine mit der CAS-Nummer 13497-18-2 kann zum Beispiel von Sigma-Aldrich käuflich erworben werden.

N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.

3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

In einer weiteren bevorzugten Ausführungsform ist ein Verfahren dadurch gekennzeichnet, dass das Mittel (a) mindestens eine organische Siliciumverbindung (a1) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1-propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und/oder
- N,N-Bis[3-(triethoxysilyl)propyl]-2-Propen-1-amin.

Das in dem Verfahren auf dem keratinischen Material angewendete Mittel (a) enthält mindestens eine organische Siliciumverbindung der Formel (IV)

R₉Si(OR₁₀)ₖ(R₁₁)ₘ (IV).

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das bzw. die organischen Siliciumverbindungen der Formel (IV) können auch als Silane vom Typ der Alkyl-alkoxy-silane oder der Alkyl-hydroxy-silane bezeichnet werden,

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

Das Verfahren ist dadurch gekennzeichnet, dass das Mittel (a) mindestens eine organische Siliciumverbindung (a1) der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

Somit ist ein Verfahren dadurch gekennzeichnet, dass das Mittel (a) zusätzlich zu der oder den organischen Siliciumverbindungen der Formel (I) mindestens eine weitere organische Siliciumverbindung der Formel (IV) enthält

R₉Si(OR₁₀)ₖ(R₁₁)ₘ (IV),

wobei
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen der Formel (IV) steht der Rest R₉ für eine C₁-C₁₈-Alkylgruppe. Diese C₁-C₁₈-Alkylgruppe ist gesättigt und kann linear oder verzweigt sein. Bevorzugt steht R₉ für eine lineare C₁-C₁₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe, eine n-Dodecylgruppe oder eine n-Octadecylgruppe. Besonders bevorzugt steht R₉ für eine Methylgruppe, eine Ethylgruppe, eine n-Propylgruppe, eine n-Hexylgruppe oder eine n-Octylgruppe.

In den organischen Siliciumverbindungen der Form (IV) steht der Rest R₁₀ für ein Wasserstoffatom oder eine C₁-C₆-Alkylgruppe. Besonders bevorzugt steht R₁₀ für eine Methylgruppe oder für eine Ethylgruppe.

In den organischen Siliciumverbindungen der Form (IV) steht der Rest R₁₁ für eine C₁-C₆-Alkylgruppe. Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Besonders stabile Filme, d.h. Färbungen mit besonders guten Waschechtheiten konnten erhalten werden, wenn im Verfahren ein Mittel (a) eingesetzt wurde, welches mindestens eine organische Siliciumverbindung (a1) der Formel (IV) enthält, in welcher der Rest k für die Zahl 3 steht. In diesem Fall steht der Rest m für die Zahl 0.

Zur Lösung der erfindungsgemäßen Aufgabenstellung besonders gut geeignete organische Siliciumverbindungen der Formel (IV) sind
- Methyltrimethoxysilan 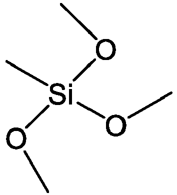
- Methyltriethoxysilan 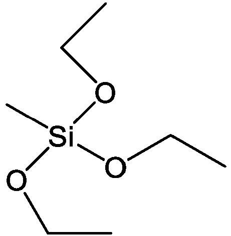
- Ethyltrimethoxysilan 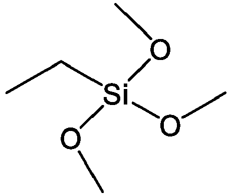
- Ethyltriethoxysilan 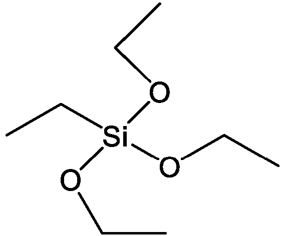
- n-Hexyltrimethoxysilan 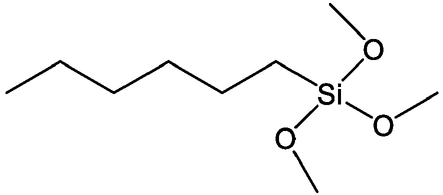
- n-Hexyltriethoxysilan 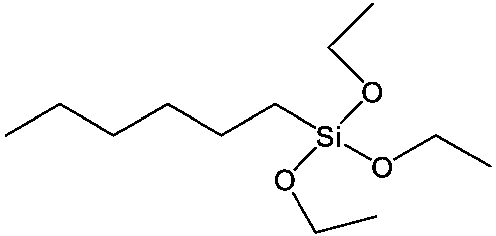
- n-Octyltrimethoxysilan 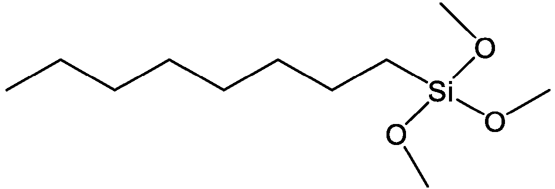
- n-Octyltriethoxysilan 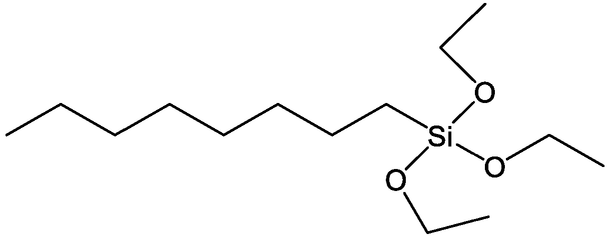
- n-Dodecyltrimethoxysilan und/oder 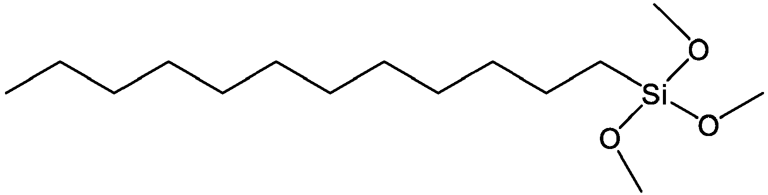
- n-Dodecyltriethoxysilan.

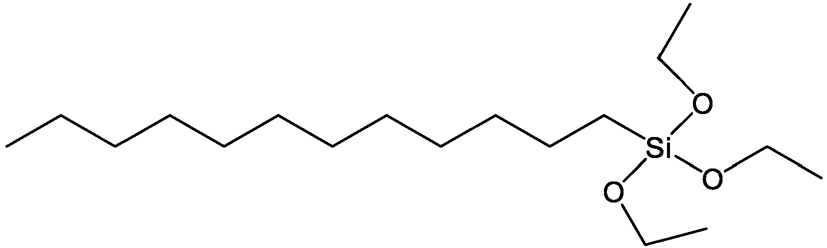

In einer weiteren bevorzugten Ausführungsform ist ein Verfahren dadurch gekennzeichnet, dass das Mittel (a) mindestens eine organische Siliciumverbindung (a1) der Formel (IV) enthält, die ausgewählt ist aus der Gruppe aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan
- Dodecyltriethoxysilan
- Octyldecyltrimethoxysilan und/oder
- Octyldecyltriethoxysilan.

Bei den zuvor beschriebenen organischen Siliciumverbindungen handelt es sich um reaktive Verbindungen. In diesem Zusammenhang hat es sich als bevorzugt herausgestellt, wenn das Mittel (a) - bezogen auf das Gesamtgewicht des Mittels (a) - eine oder mehrere organische Siliciumverbindungen (a1) in einer Gesamtmenge von 0,1 bis 20 Gew.-%, bevorzugt 1 bis 15 Gew.-% und besonders bevorzugt 2 bis 8 Gew.-% enthält.

In einer weiteren bevorzugten Ausführungsform ist ein Verfahren dadurch gekennzeichnet, dass das Mittel (a) - bezogen auf das Gesamtgewicht des Mittels (a) - eine oder mehrere organische Siliciumverbindungen (a1) in einer Gesamtmenge von 0,1 bis 20 Gew.-%, bevorzugt 1 bis 15 Gew.-% und besonders bevorzugt 2 bis 8 Gew.-% enthält.

Zur Erzielung von besonders guten Färbeergebnissen ist es insbesondere von Vorteil, die organische Siliciumverbindungen der Formel (I) in bestimmten Mengenbereichen im Mittel (a) einzusetzen. Besonders bevorzugt enthält das Mittel (a) - bezogen auf das Gesamtgewicht des Mittels (a) - ein oder mehrere organische Siliciumverbindungen der Formel (I) und/oder (II) in einer Gesamtmenge von 0,1 bis 10 Gew.-%, bevorzugt 0,5 bis 5 Gew.-% und besonders bevorzugt 0,5 bis 3 Gew.-%.

In einer weiteren bevorzugten Ausführungsform ist ein Verfahren dadurch gekennzeichnet, dass das Mittel (a) - bezogen auf das Gesamtgewicht des Mittels (a) - eine oder mehrere organische Siliciumverbindungen der Formel (I) und (II) in einer Gesamtmenge von 0,1 bis 10 Gew.-%, bevorzugt 0,5 bis 5 Gew.-% und besonders bevorzugt 0,5 bis 3 Gew.-% enthält.

Weiterhin hat es sich als ganz besonders bevorzugt herausgestellt, wenn auch das oder die organische Siliciumverbindungen der Formel (IV) in bestimmten Mengenbereichen im Mittel (a) enthalten sind. Besonders bevorzugt enthält das Mittel (a) - bezogen auf das Gesamtgewicht des Mittels (a) - eine oder mehrere organische Siliciumverbindungen der Formel (IV) in einer Gesamtmenge von 0,1 bis 20 Gew.-%, bevorzugt 2 bis 15 Gew.-% und besonders bevorzugt 4 bis 9 Gew.-%.

In einer weiteren bevorzugten Ausführungsform ist ein Verfahren dadurch gekennzeichnet, dass das Mittel (a) - bezogen auf das Gesamtgewicht des Mittels (a) - eine oder mehrere organische Siliciumverbindungen der Formel (IV) in einer Gesamtmenge von 0,1 bis 20 Gew.-%, bevorzugt 2 bis 15 Gew.-% und besonders bevorzugt 3,2 bis 10 Gew.-% enthält.

In einer explizit ganz besonders bevorzugten Ausführungsform ist ein Verfahren dadurch gekennzeichnet, dass auf dem keratinischen Material ein Mittel (a) angewendet wird, welches mindestens eine organische Siliciumverbindungen der Formel (I) enthält, welche aus der Gruppe aus (3-Aminopropyl)triethoxysilan und (3-Aminopropyl)trimethoxysilan ausgewählt ist, und zusätzlich mindestens eine organische Siliciumverbindungen der Formel (IV) enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan und Hexyltriethoxysilan ausgewählt ist.

In einer weiteren bevorzugten Ausführungsform ist ein Verfahren dadurch gekennzeichnet, dass das Mittel (a) - bezogen auf das Gesamtgewicht des Mittels (a) - enthält:
- 0,5 bis 5 Gew.-% mindestens einer ersten organischen Siliciumverbindung (a1) die ausgewählt ist aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 10 Gew.-% mindestens einer zweiten organischen Siliciumverbindung (a1) die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

Im Rahmen dieser Ausführungsform enthält das Mittel (a) eine oder mehrere organischen Siliciumverbindungen einer ersten Gruppe in einer Gesamtmenge von 0,5 bis 3 Gew.-%. Die organischen Siliciumverbindungen dieser ersten Gruppe sind ausgewählt aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und/oder (2-Dimethylaminoethyl)triethoxysilan.

Im Rahmen dieser Ausführungsform enthält das Mittel (a) eine oder mehrere organischen Siliciumverbindungen einer zweiten Gruppe in einer Gesamtmenge von 3,2 bis 10 Gew.-%. Die organischen Siliciumverbindungen dieser zweiten Gruppe sind ausgewählt aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

Bereits der Zusatz geringer Wassermengen führt bei organischen Siliciumverbindungen mit mindestens einer hydrolysierbaren Gruppe zur Hydrolyse. Die Hydrolyseprodukte und/oder organischen Siliciumverbindungen mit mindestens einer Hydroxygruppe können in einer Kondensationsreaktion miteinander reagieren. Aus diesem Grund können sowohl die siliciumorganischen Verbindungen mit mindestens einer hydrolysierbaren Gruppe als auch deren Hydrolyse- und/oder Kondensationsprodukte in dem Mittel (a) enthalten sein. Bei Verwendung von siliciumorganischen Verbindungen mit mindestens einer Hydroxylgruppe können sowohl die organischen Siliciumverbindungen mit mindestens einer Hydroxylgruppe als auch deren Kondensationsprodukte in dem Mittel (a) enthalten sein.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Ganz besonders gute Ergebnisse konnten erhalten werden, wenn in dem Verfahren organischen Siliciumverbindungen der Formel (I) und/oder (II) eingesetzt wurden. Da wie bereits zuvor beschrieben bereits bei Spuren von Feuchtigkeit eine Hydrolyse/Kondensation einsetzt, sind auch die Hydrolyse- und/oder Kondensationsprodukte der organischen Siliciumverbindungen (I) und/oder (II) von dieser Ausführungsform mit umfasst.

### Farbgebende Verbindungen (a2)

Bei der Anwendung des Mittels (a) auf dem keratinischen Material werden zunächst die organische(n) Siliciumverbindung(en) (a1), die eine oder mehrere Hydroxylgruppen oder hydrolysierbare Gruppen pro Molekül umfassen, in Anwesenheit des Wassers hydrolysiert und oligomerisiert oder polymerisiert. Die auf diese Weise entstehenden Hydrolyseprodukte oder Oligomere besitzen eine besonders hohe Affinität zur Oberfläche des keratinischen Materials. Durch die gleichzeitige Anwesenheit der farbgebenden Verbindungen (a2) im Mittel (a) werden diese in die entstehenden Oligomere oder Polymere integriert, so dass auf dem keratinischen Material ein gefärbter Film entsteht. Im Anschluss an die Anwendung des Mittels (a) wird nun das Mittel (b). Durch die sukzessive Anwendung der Mittel (a) und (b) entsteht so eine Färbung, die gegenüber äußeren Einflüssen besonders widerstandfähig ist. Die in diesen resistenten Filmen eingeschlossenen farbgebenden Verbindungen weisen eine gute Waschechtheit auf.

Als erfindungswesentlichen Bestandteil (a2) enthält das in dem Färbeverfahren angewendete Mittel (a) daher mindestens eine farbgebende Verbindung. Die mindestens eine farbgebende Verbindung(a2) enthält mindestens ein Effektpigment, umfassend α) ein Substratplättchen und β) eine Beschichtung,
wobei die Beschichtung mindestens eine Schicht aufweist, die nass-chemisch unter Verwendung eines Metallalkoxids und einer siliciumorganischen Verbindung mit einer basischen Gruppe hergestellt worden ist.

Die Effektpigmente zeigen durch die erfindungsgemäße Beschichtung eine besonders hohe Affinität zu dem zu färbenden keratinischen Material und zu dem mit Hilfe der organische(n) Siliciumverbindung(en) (a1) gebildeten Film auf dem keratinischen Material.

Entsprechend können mit Hilfe der erfindungsgemäß beschichteten Effektpigmente keratinische Fasern besonders intensiv und dauerhaft gefärbt werden.

Das Effektpigment weist ein Substratplättchen auf.

Das Substratplättchen weist vorzugsweise eine durchschnittliche Dicke von höchstens 150 nm, vorzugsweise weniger als 50 nm, mehr bevorzugt weniger als 30 nm, besonders bevorzugt höchstens 25 nm, beispielsweise höchstens 20 nm auf. Die durchschnittliche Dicke der Substratplättchen beträgt mindestens 1 nm, vorzugsweise mindestens 2,5 nm, besonders bevorzugt mindestens 5 nm, beispielsweise mindestens 10 nm. Bevorzugte Bereiche für die Dicke des Substratplättchens sind 2,5 bis 50 nm, 5 bis 50 nm, 10 bis 50 nm; 2,5 bis 30 nm, 5 bis 30 nm, 10 bis 30 nm; 2,5 bis 25 nm, 5 bis 25 nm, 10 bis 25 nm, 2,5 bis 20 nm, 5 bis 20 nm und 10 bis 20 nm. Vorzugsweise weist jedes Substratplättchen eine möglichst einheitliche Dicke auf.

Das Substratplättchen ist vorzugsweise monolithisch aufgebaut. Monolithisch bedeutet in diesem Zusammenhang aus einer einzigen abgeschlossenen Einheit ohne Brüche, Schichtungen oder Einschlüsse bestehend, wobei jedoch innerhalb des Substratplättchens Gefügewechsel auftreten können. Das Substratplättchen ist vorzugsweise homogen aufgebaut, d.h. dass innerhalb des Plättchens kein Konzentrationsgradient auftritt. Insbesondere ist das Substratplättchen nicht schichtartig aufgebaut und weist keine darin verteilten Teilchen oder Partikel auf.

Die Größe des Substratplättchens kann auf den jeweiligen Anwendungszweck, beispielsweise dem gewünschten Effekt auf einem keratinischen Material, abgestimmt werden. In der Regel haben die Substratplättchen einen mittleren größten Durchmesser von etwa 2 bis 200 µm, insbesondere etwa 5 bis 100 µm.

In einer bevorzugten Ausführungsform beträgt der Formfaktor (Aspect Ratio), ausgedrückt durch das Verhältnis der mittleren Größe zur durchschnittlichen Dicke, mindestens 80, vorzugsweise mindestens 200, mehr bevorzugt mindestens 500, besonders bevorzugt mehr als 750. Dabei wird als mittlere Größe der unbeschichteten Substratplättchen der d50-Wert der unbeschichteten Substratplättchen verstanden. Der d50-Wert wurde, soweit nicht anders angegeben, mit einem Gerät des Typs Sympatec Helos mit Quixel-Nassdispergierung bestimmt. Dabei wurde zur Probenvorbereitung die zu untersuchende Probe für eine Dauer von 3 Minuten in Isopropanol vordispergiert.

Das Substratplättchen kann aus jedem Material, das in Plättchenform gebracht werden kann, aufgebaut sein.

Sie können natürlichen Ursprungs, aber auch synthetisch hergestellt sein. Materialien, aus denen die Substratplättchen aufgebaut sein können, sind beispielsweise Metalle und Metalllegierungen, Metalloxide, vorzugsweise Aluminiumoxid, anorganische Verbindungen und Mineralien wie Glimmer und (Halb)Edelsteine, sowie Kunststoffe. Vorzugsweise sind die Substratplättchen aus einem Metall oder einer Legierung aufgebaut.

Als Metall kommt jedes, für Effektpigmente geeignete Metall in Betracht. Derartige Metalle sind unter anderem Eisen und Stahl, sowie alle luft- und wasserbeständigen (Halb)metalle wie beispielsweise Platin, Zinn, Zink, Chrom, Molybdän und Silicium, sowie deren Legierungen wie Aluminiumbronzen und Messing. Bevorzugte Metalle sind Aluminium, Kupfer, Silber und Gold. Bevorzugte Substratplättchen sind Aluminiumplättchen und Messingplättchen, wobei Substratplättchen aus Aluminium besonders bevorzugt sind.

Substratplättchen aus Aluminium können unter anderem durch Herausstanzen aus Aluminiumfolie oder nach gängigen Mahl- und Verdüsungstechniken hergestellt werden. So sind beispielsweise Aluminiumplättchen aus dem Hallverfahren, einem Nassmahlverfahren, erhältlich.

Andere Metallplättchen, beispielsweise aus Bronze, können in einem Trockenmahlverfahren wie dem Hametagverfahren erhalten werden.

Die Substratplättchen können verschiedene Formen aufweisen. Als Substratplättchen können beispielsweise lamellare oder lentikulare Metallplättchen oder auch sogenannte *vacuum metallized pigments* (VMP) verwendet werden. Lamellare Substratplättchen zeichnen sich durch einen unregelmäßig strukturierten Rand aus und werden aufgrund ihres Erscheinungsbildes auch als "cornflakes" bezeichnet. Lentikulare Sustratplättchen weisen einen im Wesentlichen regelmäßigen runden Rand auf und werden aufgrund ihres Erscheinungsbildes auch als "silverdollars" bezeichnet.

Die Substratplättchen aus Metall oder Metalllegierung können passiviert sein, beispielsweise durch Eloxieren (Oxidschicht) oder Chromatieren.

Durch eine Beschichtung können die Oberflächeneigenschaften und/oder optischen Eigenschaften des Effektpigments verändert sowie die mechanische und chemische Belastbarkeit der Effektpigmente erhöht werden. Es können beispielsweise lediglich die obere und/oder untere Seite des Substratplättchens beschichtet sein, wobei die Seitenflächen ausgespart sind. Vorzugsweise ist die gesamte Oberfläche der gegebenenfalls passivierten Substratplättchen, einschließlich der Seitenflächen, von der Schicht bedeckt. Die Substratplättchen sind vorzugsweise vollständig von der Beschichtung umhüllt.

Die Beschichtung kann aus einer oder aus mehreren Schichten bestehen. In einer bevorzugten Ausführungsform weist die Beschichtung lediglich eine Schicht A auf. In einer ebenfalls bevorzugten Ausführungsform weist die Beschichtung insgesamt mindestens zwei, vorzugsweise zwei oder drei, Schichten auf. Es kann bevorzugt sein, die Beschichtung zwei Schichten A und B aufweist, wobei die Schicht B von der Schicht A verschieden ist. Vorzugsweise befindet sich Schicht A zwischen der Schicht B und der Oberfläche des Substratplättchens. In noch einer bevorzugten Ausführungsform weist die Beschichtung drei Schichten A, B und C auf. In dieser Ausführungsform befindet sich zwischen der Schicht B und der Oberfläche des Substratplättchens die Schicht A und auf der Schicht B befindet sich eine Schicht C, die von der darunterliegenden Schicht B verschieden ist.

Als Materialien für die mindestens eine Schicht, beispielsweise Schichten A und gegebenenfalls B und C, eignen sich alle Substanzen, die dauerhaft auf die Substratplättchen aufgebracht werden können. Die Materialien sollten mit Vorzug filmartig aufbringbar sein. Vorzugsweise ist die gesamte Oberfläche der gegebenenfalls passivierten Substratplättchen, einschließlich der Seitenflächen, von der mindestens einen Schicht, beispielsweise von Schicht A oder von den Schichten A und B oder von den Schichten A, B und C, umhüllt.

Die mindestens eine Schicht wird nass-chemisch unter Verwendung eines Metallalkoxids und einer siliciumorganischen Verbindung mit einer basischen Gruppe hergestellt.

Es ist erfindungswesentlich, dass bei der Herstellung der mindestens eine Schicht eine siliciumorganischen Verbindung mit einer basischen Gruppe eingesetzt wird.

Bei dieser basischen Gruppe oder basischen chemischen Funktion kann es sich beispielsweise um eine Aminogruppe, eine Alkylaminogruppe, eine Dialkylaminogruppe oder um eine Trialkylaminogruppe handeln, die bevorzugt über einen Linker mit einem Siliciumatom verbunden ist. Bevorzugt handelt es sich bei der basischen Gruppe um eine Aminogruppe, eine C₁-C₆-Alkylaminogruppe oder um eine Di(C₁-C₆)alkylaminogruppe.

Die organische Siliciumverbindung ist vorzugsweise ein Silan mit einem, zwei oder drei Siliciumatomen.

Im Rahmen einer ganz besonders bevorzugten Ausführungsform wird bei der nass-chemischen Herstellung der mindestens einen Schicht der Beschichtung des Effektpigments eine siliciumorganische Verbindung mit einer basischen Gruppe eingesetzt, welche aus Silanen mit einem, zwei oder drei Siliciumatomen ausgewählt ist, wobei die organische Siliciumverbindung außerdem eine oder mehrere basische chemische Gruppen und eine oder mehrere Hydroxylgruppen oder hydrolysierbare Gruppen pro Molekül umfasst.

Geeignete siliciumorganische Verbindung mit einer basischen Gruppe entsprechen dabei den siliciumorganischen Verbindungen, welche bereits oben als geeignete organische Siliciumverbindung (a1) beschrieben wurden.

Dabei ist es insbesondere bevorzugt, dass die siliciumorganischen Verbindung mit einer basischen Gruppe die Formel (I) und/oder (II) aufweist,

R₁R₂N-L-Si(OR₃)ₐ(R₄)_{b} (I),

wobei
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder eine C₁-C₆-Alkylgruppe steht,
- R₄ für eine C₁-C₆-Alkylgruppe steht
- a, für eine ganze Zahl von 1 bis 3 steht, und
- b für die ganze Zahl 3 - a steht,

   (R₅O)_{c}(R₆)_{d}Si-(A)ₑ-[NR₇-(A')]_{f}-[O-(A")]_{g}-[NR₈-(A‴)]ₕ-Si(Rₑ')_{d'}(OR₅')_{c'} (II),
wobei
- R5, R5', R5" unabhängig voneinander für ein Wasserstoffatom oder für eine C₁-C₆-Alkylgruppe stehen,
- R6, R6' und R6" unabhängig voneinander für eine C₁-C₆-Alkylgruppe stehen,
- A, A', A", A‴ und A"" unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III) stehen

   - (Aʺʺ)-Si(R₆")_{d}"(OR₅")_{c}" (III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

Die siliciumorganischen Verbindung mit einer basischen Gruppe ist vorzugsweise ausgewählt aus der Gruppe bestehend aus
- (3-Aminopropyl)triethoxysilan
- (3-Aminopropyl)trimethoxysilan
- -1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- -1-(3-Dimethylaminopropyl)silantriol
- (2-Dimethylaminoethyl)triethoxysilan
- (2-Dimethylaminoethyl)trimethoxysilan
- 1-(2-Dimethylaminoethyl)silantriol und
- Mischungen daraus.

Ganz besonders bevorzugt werden (3-Dimethylaminopropyl)triethoxysilan und/oder (3-Dimethylaminopropyl)trimethoxysilan als siliciumorganischen Verbindung mit einer basischen Gruppe eingesetzt.

Bereits der Zusatz geringer Wassermengen führt bei organischen Siliciumverbindungen mit basischer Gruppe und mit mindestens einer hydrolysierbaren Gruppe zur Hydrolyse. Die Hydrolyseprodukte und/oder organischen Siliciumverbindungen mit einer basischen Gruppe und mit mindestens einer Hydroxygruppe und/oder die Hydrolyseprodukte der Metallalkoxide können in einer Kondensationsreaktion miteinander reagieren. Aus diesem Grund können sowohl die siliciumorganischen Verbindungen mit basischer Gruppe und mit mindestens einer hydrolysierbaren Gruppe als auch deren Hydrolyse- und/oder Kondensationsprodukte sowie die Kondensationsprodukte mit den Hydrolyseprodukten der Metallalkoxide als auch die Kondensationsprodukte der Hydrolyseprodukten der Metallalkoxide in der mindestens einen Schicht enthalten sein.

Unter einem Kondensationsprodukt wird entweder ein Produkt verstanden, dass durch Reaktion von mindestens zwei organischen Siliciumverbindungen mit basischer Gruppe und mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Unter einem Kondensationsprodukt wird auch ein Produkt verstanden, dass durch Reaktion von mindestens einer organischen Siliciumverbindung mit einer basischen Gruppe und mit mindestens einer Hydroxylgruppen oder hydrolysierbaren Gruppen pro Molekül mit einem Hydrolyseprodukt oder einem Kondensationsprodukt der Metallalkoxide unter Abspaltung von Wasser und/oder unter Abspaltung von einem Alkanol entsteht.

Die Kondensationsprodukte der Hydrolyseprodukten der Metallalkoxide sind üblicherweise Metalloxide und/oder Metalloxidhydrate.

Durch Einsatz von Säuren und/oder Basen können die Hydrolyse und/oder die Kondensationsreaktion beeinflusst werden. So kann beispielsweise die Ausbildung der mindestens einen Schicht hinsichtlich Dicke, Kondensationsgrad der Kondensationsprodukte, Vernetzungsgrad der Kondensationsprodukte, Reaktionsgeschwindigkeit beeinflusst und gesteuert werden.

Die mindestens eine Schicht enthält entsprechend vorzugsweise ein Metalloxid und/oder Metalloxidhydrat.

Weist die organische Siliciumverbindung mit einer basischen Gruppe keine Hydroxylgruppe oder hydrolysierbare Gruppen auf, enthält die mindestens eine Schicht neben einem Metalloxid und/oder einem Metalloxidhydrat die organische Siliciumverbindung mit einer basischen Gruppe.

Es ist bevorzugt, dass das Metalloxid und/oder Metalloxidhydrat ausgewählt ist aus der Gruppe bestehend aus Silicium(di)oxid, Siliciumoxidhydrat, Aluminiumoxid, Aluminiumoxidhydrat, Boroxid, Germaniumoxid, Manganoxid, Magnesiumoxid, Eisenoxid, Cobaltoxid, Chromoxid, Titandioxid, Vanadiumoxid, Zirkonoxid, Zinnoxid, Zinkoxid und deren Gemischen.

Die Schicht A weist vorzugsweise mindestens ein niedrigbrechendes Metalloxid und/oder Metalloxidhydrat auf. Vorzugsweise umfasst Schicht A mindestens 95 Gew.-% niedrigbrechendes Metalloxid(hydrat). Niedrigbrechende Materialien weisen einen Brechungsindex von höchstens 1,8, bevorzugt höchstens 1,6 auf.

Zu den niedrigbrechenden Metalloxiden, die für die Schicht A geeignet sind, zählen beispielsweise Silicium(di)oxid, Siliciumoxidhydrat, Aluminiumoxid, Aluminiumoxidhydrat, Boroxid, Germaniumoxid, Manganoxid, Magnesiumoxid und deren Gemische, wobei Siliciumdioxid bevorzugt ist. Die Schicht A weist bevorzugt eine Dicke von 1 bis 100 nm, besonders bevorzugt 5 bis 50 nm, insbesondere bevorzugt 5 bis 20 nm, auf.

Die Schicht B, falls vorhanden, ist von der Schicht A verschieden und kann mindestens ein hochbrechendes Metalloxid enthalten. Hochbrechende Materialien weisen einen Brechungsindex von mindestens 1,9, bevorzugt mindestens 2,0 und besonders bevorzugt mindestens 2,4 auf. Vorzugsweise umfasst die Schicht B mindestens 95 Gew.-%, besonders bevorzugt mindestens 99 Gew.-% an hochbrechenden Metalloxid(en).

Enthält die Schicht B ein (hochbrechendes) Metalloxid, weist sie vorzugsweise eine Dicke von mindestens 50 nm auf. Vorzugsweise beträgt die Dicke von Schicht B nicht mehr als 400 nm, besonders bevorzugt höchstens 300 nm.

Für Schicht B geeignete hochbrechende Metalloxide sind beispielsweise selektiv lichtabsorbierende (d.h. farbige) Metalloxide, wie beispielsweise Eisen(III)oxid (α- und γ-Fe2O3, rot), Cobalt(II)oxid (blau), Chrom(III)oxid (grün),Titan(III)oxid (blau, liegt üblicherweise im Gemisch mit Titanoxynitriden und Titannitriden vor) und Vanadium(V)oxid (orange) sowie deren Gemische. Es eignen sich auch farblose hochbrechende Oxide wie Titandioxid und/oder Zirkonoxid.

Schicht B kann einen selektiv absorbierenden Farbstoff enthalten, vorzugsweise 0,001 bis 5 Gew.-%, besonders bevorzugt 0,01 bis 1 Gew.-%, jeweils bezogen auf die Gesamtmenge der Schicht B. Geeignet sind organische und anorganische Farbstoffe, die sich stabil in eine Metalloxidbeschichtung einbauen lassen. Farbstoffe im Sinne der vorliegenden Erfindung besitzen eine Löslichkeit in Wasser (760 mmHg) bei 25 °C von mehr als 0,5 g/L und sind daher nicht als Pigmente anzusehen.

Alternativ zu einem Metalloxid kann Schicht B eine Metallpartikelträgerschicht mit auf der Oberfläche der Metallpartikelträgerschicht aufgebrachten Metallpartikeln umfassen. In einer bevorzugten Ausführungsform bedecken die Metallpartikel direkt einen Teil der Metallpartikelträgerschicht. In dieser Ausführungsform weist das Effektpigment Bereiche auf, in denen sich keine Metallpartikel befinden, d.h. Bereiche, die nicht mit den Metallpartikeln bedeckt sind.

Die Metallpartikelträgerschicht eine Metallschicht und/oder eine Metalloxidschicht umfassen.

Wenn die Metallpartikelträgerschicht eine Metallschicht und eine Metalloxidschicht umfasst, ist die Anordnung dieser Schichten nicht limitiert.

Es ist bevorzugt, dass die Metallpartikelträgerschicht wenigstens eine Metallschicht umfasst. Es ferner bevorzugt, dass die Metallschicht ein Element ausgewählt aus Zinn (Sn), Palladium (Pd), Platin (Pt) und Gold (Au) aufweist.

Die Metallschicht kann beispielsweise durch Zugabe von Alkali zu einer das Metall enthaltenden Metallsalzlösung gebildet werden.

Enthält die Metallpartikelträgerschicht eine Metalloxidschicht, umfasst diese vorzugsweise kein Siliciumdioxid. Die Metalloxidschicht enthält vorzugsweise ein Oxid von mindestens einem Element, ausgewählt aus der Gruppe bestehend aus von Mg (Magnesium), Sn (Zinn), Zn (Zink), Co (Kobalt), Ni (Nickel), Fe (Eisen), Zr (Zirkonium), Ti (Titan) und Ce (Cer). Besonders bevorzugt enthält die Metallpartikelträgerschicht iii) in Form einer Metalloxidschicht ein Metalloxid von Sn, Zn, Ti und Ce.

Die Herstellung der Metallpartikelträgerschicht in Form einer Metalloxidschicht kann beispielsweise durch Hydrolyse eines Alkoxids eines Metalls, das das Metall des Metalloxids bildet, in einem Sol-Gel Verfahren erfolgen.

Die Dicke der Metallschicht beträgt vorzugsweise nicht mehr als 30 nm.

Die Metallpartikel können wenigstens ein Element umfassen, welches ausgewählt ist aus der Gruppe bestehend aus Aluminium (Al), Titan (Ti), Chrom (Cr), Eisen (Fe), Kobalt (Co), Nickel (Ni), Kupfer (Cu), Zink (Zn), Ruthenium (Ru), Rhodium (Rh), Palladium (Pd), Silber (Ag), Zinn (Sn), Platin (Pt), Gold (Au) und deren Legierungen. Es ist insbesondere bevorzugt, dass die Metallpartikel wenigstens ein Element ausgewählt aus Kupfer (Cu), Nickel (Ni) und Silber (Ag) aufweisen.

Der durchschnittliche Partikeldurchmesser der Metallpartikel beträgt vorzugsweise nicht mehr als 50 nm, mehr bevorzugt nicht mehr als 30 nm. Der Abstand zwischen den Metallpartikeln beträgt vorzugsweise nicht mehr als 10 nm.

Als Verfahren zur Bildung der Metallpartikel eignen sich Vakuumverdampfung, Sputtern, Chemical Vapor Deposition (CVD), stromloses Plattieren oder dergleichen. Von diesen Verfahren ist das stromlose Plattieren besonders bevorzugt.

Gemäß einer bevorzugten Ausführungsform weisen die Effektpigmente eine weitere Schicht C, umfassend ein Metalloxid(hydrat), die von der darunterliegenden Schicht B verschieden ist, auf. Geeignete Metalloxide sind beispielsweise Silicium(di)oxid, Siliciumoxidhydrat, Aluminiumoxid, Aluminiumoxidhydrat, Zinkoxid, Zinnoxid, Titandioxid, Zirkonoxid, Eisen(III)oxid und Chrom(III)oxid. Bevorzugt ist Siliciumdioxid.

Die Schicht C weist vorzugsweise eine Dicke von 10 bis 500 nm, besonders bevorzugt 50 bis 300 nm auf.

Die Beschichtung des Effektpigments weist mindestens eine Schicht auf, die nass-chemisch aus einem Metallalkoxid und einer siliciumorganischen Verbindung mit einer basischen Gruppe hergestellt worden ist.

Die mindestens eine Schicht, die unter Verwendung eines Metallalkoxids und einer siliciumorganischen Verbindung mit einer basischen Gruppe hergestellt worden ist, kann Schicht A, B und/oder C sein. Im Fall, dass die Beschichtung lediglich eine Schicht A aufweist, ist Schicht A unter Verwendung eines Metallalkoxids und einer siliciumorganischen Verbindung mit einer basischen Gruppe hergestellt worden.

Im Fall, dass die Beschichtung des Effektpigments zwei Schichten A und B aufweist, ist Schicht B unter Verwendung eines Metallalkoxids und einer siliciumorganischen Verbindung mit einer basischen Gruppe hergestellt worden.

Im Fall, dass die Beschichtung die Schichten A, B und C aufweist, ist Schicht C unter Verwendung eines Metallalkoxids und einer siliciumorganischen Verbindung mit einer basischen Gruppe hergestellt worden.

Es ist insbesondere bevorzugt, dass das Effektpigment ein Substratplättchen aus Aluminium und eine Schicht A, welche unter Einsatz eines Siliciumalkoxid und der siliciumorganischen Verbindung mit einer basischen Gruppe hergestellt worden ist, umfasst. Weist das Effektpigment auf Basis eines Substratplättchens eine Schicht A und eine Schicht C auf, ist es bevorzugt, dass das Effektpigment ein Substratplättchen aus Aluminium, eine Schicht A, umfassend Siliciumdioxid, und eine Schicht C aufweist, wobei zur Herstellung der Schicht C ein Siliciumalkoxid und die siliciumorganische Verbindung mit einer basischen Gruppe verwendet wurde.

Das in dem nass-chemischen Beschichtungsverfahren eingesetzte Metallalkoxid ist vorzugsweise ein Siliciumalkoxid ausgewählt aus der Gruppe bestehend aus Tetramethylorthosilikat, Tetraethylorthosilikat, Tetraisopropylorthosilikat und Mischungen daraus, wobei Tetraethylorthosilikat bevorzugt ist.

Alternativ können Aluminiumalkoxide wie Aluminiumtriisopropanolat oder Aluminiumtri-sec-butanolat, Zirkonalkoxide wie Zirkoniumpropylat oder Titanalkoxide wie Titantertraethylat (Tetraethylorthotitanat) oder Titantetraisopropanolat (Tetraisopropylorthotitanat) eingesetzt werden.

Alternativ oder zusätzlich einem Tetraalkoxysilan können Alkyltrialkoxysilane in dem nass-chemischen Beschichtungsverfahren zur Herstellung der mindestens einen Schicht, beispielsweise der Schicht A oder C, eingesetzt werden.

Geeignete Alkyltrialkoxysilane umfassen beispielsweise Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und/oder Octadecyltriethoxysilan.

Die mindestens eine Schicht kann entsprechend ferner Hydrolyse- und/oder Kondensationprodukte der Alkyltrialkoxysilane umfassen. Die Kondensationsprodukte können Kondensationsprodukte von zwei oder mehr Alkyltrialkoxysilanen, Kondensationsprodukte von Alkyltrialkoxysilanen mit Hydrolyse- und/oder Kondensationsprodukten der organischen Siliciumverbindung mit einer basischen Gruppe und mit mindestens einer Hydroxylgruppen oder hydrolysierbaren Gruppen pro Molekül und/oder Kondensationsprodukte von Alkyltrialkoxysilanen mit Hydrolyse- und/oder Kondensationsprodukten der Metallalkoxide umfassen.

Weist die organische Siliciumverbindung mit einer basischen Gruppe keine Hydroxylgruppe oder hydrolysierbare Gruppen auf, enthält die mindestens eine Schicht neben einem Metalloxid und/oder einem Metalloxidhydrat und der organische Siliciumverbindung mit einer basischen Gruppe ferner Hydrolyse- und/oder Kondensationprodukte der Alkyltrialkoxysilane.

In einer bevorzugten Ausführungsform des Herstellungsverfahrens wurde das in Schritt (α) eingesetzte Substratplättchen bereits mit mindestens einer Schicht aus einem Metalloxid und/oder Metalloxidhydrat beschichtet.

Ein beispielhaftes Herstellverfahren umfasst das Dispergieren der unbeschichteten Substratplättchen oder der bereits mit Schicht A oder mit den Schichten A und B beschichteten Substratplättchen und der siliciumorganischen Verbindung mit mindestens einer basischen Gruppe in einer Lösung eines Metallalkoxids wie Tetraethylorthosilikat oder Aluminiumtriisopropanolat (üblicherweise in einer Lösung von organischem Lösungsmittel oder einer Mischung von organischem Lösungsmittel und Wasser mit mindestens 50 Gew.-% organisches Lösungsmittel wie ein C1 bis C4-Alkohol), und Zugabe einer schwachen Base oder Säure zur Hydrolysierung des Metallalkoxids, wodurch ein Film, auf der Oberfläche der (beschichteten) Substratplättchen entsteht.

Die Schicht B kann beispielsweise durch hydrolytische Zersetzung einer oder mehrerer organischer Metallverbindungen und/oder durch Fällung eines oder mehrerer gelöster Metallsalze sowie eine ggf. anschließende Nachbehandlung (zum Beispiel Überführen einer gebildeten hydroxidhaltigen Schichten in die Oxidschichten durch Tempern) hergestellt werden.

Das nass-chemische Verfahren ist vorzugsweise ein Sol-Gel-Verfahrens in dem ein Metallalkoxid und eine siliciumorganische Verbindung mit einer basischen Gruppe eingesetzt werden.

Obwohl zur Herstellung der mindestens einen Schicht, vorzugsweise der Schichten A und/oder C, ein Gemisch von zwei oder mehreren Metallalkoxiden eingesetzt kann, werden zur Herstellung der mindestens einen Schicht vorzugsweise jeweils nur Metallalkoxide eines Metalls, beispielsweise nur Siliciumalkoxide oder nur Aluminiumalkoxide, verwendet.

Es ist bevorzugt, dass das in dem Sol-Gel-Verfahren zur Herstellung der mindestens einen Schicht eingesetzte Metallalkoxid ausgewählt ist aus der Gruppe bestehend aus Tetramethylorthosilikat, Tetraethylorthosilikat, Tetraisopropylorthosilikat und Mischungen daraus, wobei Tetraethylorthosilikat bevorzugt ist.

Es kann bevorzugt sein, dass die mindestens eine Schicht des Effektpigments ferner eine farbgebende Verbindung aus der Gruppe der Pigmente aufweist.

Die Schichten A und C dienen insbesondere auch als Korrosionsschutz als auch der chemischen und physikalischen Stabilisierung. Besonders bevorzugt enthalten die Schichten A und C Siliciumdioxid oder Aluminiumoxid, die nach dem Sol-Gel-Verfahren aufgebracht werden.

Die Effektpigmente auf Basis von beschichteten Substratplättchen weisen vorzugsweise eine Dicke von 70 bis 500 nm, besonders bevorzugt 100 bis 400 nm, insbesondere bevorzugt 150 bis 320 nm, beispielsweise 180 bis 290 nm, auf. Die geringe Dicke der beschichteten Substratplättchen wird insbesondere dadurch erzielt, dass die Dicke der unbeschichteten Substratplättchen gering ist, aber auch dadurch, dass die Dicken der Beschichtungen A und, falls vorhanden, C auf einen möglichst kleinen Wert eingestellt werden.

Die Haftung und Abriebbeständigkeit von Effektpigmenten, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, im keratinischen Material kann noch weiter erhöht werden, in dem die äußerste Schicht, je nach Aufbau Schicht A, B oder C, zusätzlich durch organische Verbindung wie Silane, Phosphorsäureester, Titanate, Borate oder Carbonsäuren modifiziert wird. Dabei sind die organischen Verbindungen an die Oberfläche der äußersten, vorzugsweise Metalloxid-haltigen, Schicht A, B oder C gebunden. Die äußerste Schicht bezeichnet die Schicht, die räumlich am weitesten von dem Substratplättchen entfernt ist. Bei den organischen Verbindungen handelt es sich vorzugsweise um funktionelle Silanverbindungen, die an die Metalloxid-haltige Schicht A, B oder C binden können. Hierbei kann es sich entweder um mono- als auch um bifunktionelle Verbindungen handeln. Beispiele für bifunktionelle organische Verbindungen sind Methacryloxypropenyltrimethoxysilan, 3-Methacryloxypropyltrimethoxysilan, 3-Acryloxypropyltrimethoxysilan, 2-Acryloxyethyltrimethoxysilan, 3-Methacryloxy- propyltriethoxysilan, 3-Acryloxypropyltrimethoxysilan, 2-Methacryloxyethyltriethoxysilan, 2-Acryloxyethyltriethoxysilan, 3-Methacryloxypropyltris(methoxyethoxy)silan, 3-Methacryloxypropyltris(butoxyethoxy)silan, 3-Methacryloxy- propyltris(propoxy)silan, 3-Methacryloxypropyltris(butoxy)silan, 3-Acryloxy-propyltris(methoxyethoxy)silan, 3-Acryloxypropyltris(butoxyethoxy)silan, 3-Acryl-oxypropyltris(butoxy)silan, Vinyltrimethoxysilan, Vinyltriethoxysilan, Vinylethyl- dichlorsilan, Vinylmethyldiacetoxysilan, Vinylmethyldichlorsilan, Vinylmethyldiethoxysilan, Vinyltriacetoxysilan, Vinyltrichlorsilan, Phenylvinyldiethoxysilan, oder Phenylallyldichlorsilan. Des Weiteren kann eine Modifizierung mit einem monofunktionellen Silan, insbesondere eines Alkylsilans oder Arylsilans, erfolgen. Dieses weist nur eine funktionelle Gruppe auf, welche kovalent an die Oberfläche des Effektpigments, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, (d.h. an die äußerste Metalloxid(hydrat)-haltige Schicht) oder, bei nicht ganz vollständiger Bedeckung, an die Metalloberfläche anbinden kann. Der Kohlenwasserstoffrest des Silans weist vom Pigment weg. Je nach der Art und Beschaffenheit des Kohlenwasserstoffrests des Silans wird ein unterschiedlicher Grad der Hydrophobierung des Pigments erreicht. Beispiele für solche Silane sind Hexadecyltrimethoxysilan, Propyltrimethoxysilan, etc. Besonders bevorzugt sind Effektpigmente auf Basis von Siliciumdioxid-beschichteten Aluminiumsubstratplättchen mit einem monofunktionellen Silan oberflächenmodifiziert. Besonders bevorzugt sind Octyltrimethoxysilan, Octyltriethoxysilan, Hecadecyltrimethoxysilan sowie Hecadecyltriethoxysilan. Durch die veränderten Oberflächeneigenschaften / Hydrophobierung kann eine Verbesserung bezüglich Haftung, Abriebfestigkeit und Ausrichtung in der Anwendung erzielt werden.

Es hat sich gezeigt, dass Effektpigmente mit einer solchen Oberflächenmodifikation auch eine bessere Kompatibilität mit den eingesetzten siliciumorganischen Verbindungen und/oder deren Kondensations- oder Polymerisationsprodukten zeigen.

Besonders gute Ergebnisse konnten erhalten werden, wenn das Mittel (a) - bezogen auf das Gesamtgewicht des Mittels (a) - ein oder mehrere Effektpigmente in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

Zusätzlich zu dem Effektpigment kann das Mittel (a) weitere farbgebende Verbindungen (a2) ausgewählt aus der Gruppe bestehend aus Pigmenten und/oder direktziehenden Farbstoffen umfassen.

Der Einsatz von Pigmenten hat sich in diesem Zusammenhang als ganz besonders bevorzugt herausgestellt.

In einer weiteren ganz besonders bevorzugten Ausführungsform ist ein Verfahren dadurch gekennzeichnet, dass das Mittel (a) mindestens eine weitere farbgebende Verbindung (a2) aus der Gruppe der Pigmente enthält.

Unter Pigmenten im Sinne der vorliegenden Erfindung werden farbgebende Verbindungen verstanden, welche bei 25 °C in Wasser eine Löslichkeit von weniger als 0,5 g/L, bevorzugt von weniger als 0,1 g/L, noch weiter bevorzugt von weniger als 0,05 g/L besitzen. Die Wasserlöslichkeit kann beispielsweise mittels der nachfolgend beschriebenen Methode erfolgen: 0,5 g des Pigments werden in einem Becherglas abgewogen. Ein Rührfisch wird hinzugefügt. Dann wird ein Liter destilliertes Wasser hinzugegeben. Dieses Gemisch wird unter Rühren auf einem Magnetrührer für eine Stunde auf 25 °C erhitzt. Sind in der Mischung nach diesem Zeitraum noch ungelöste Bestandteile des Pigments sichtbar, so liegt die Löslichkeit des Pigments unterhalb von 0,5 g/L. Sofern sich die Pigment-Wasser-Mischung aufgrund der hohen Intensität des gegebenenfalls feindispergiert vorliegenden Pigments nicht visuell beurteilten lässt, wird die Mischung filtriert. Bleibt auf dem Filterpapier ein Anteil an ungelösten Pigmenten zurück, so liegt die Löslichkeit des Pigments unterhalb von 0,5 g/L.

Geeignete Pigmente können anorganischen und/oder organischen Ursprungs sein.

In einer bevorzugten Ausführungsform ist ein Verfahren dadurch gekennzeichnet, dass das Mittel (a) mindestens eine weitere farbgebende Verbindung (a2) aus der Gruppe der anorganischen und/oder organischen Pigmente enthält.

Bevorzugte Pigmente sind ausgewählt aus synthetischen oder natürlichen anorganischen Pigmenten. Anorganische Pigmente natürlichen Ursprungs können beispielsweise aus Kreide, Ocker, Umbra, Grünerde, gebranntem Terra di Siena oder Graphit hergestellt werden. Weiterhin können als anorganische Pigmente Schwarzpigmente wie z. B. Eisenoxidschwarz, Buntpigmente wie z. B. Ultramarin oder Eisenoxidrot sowie Fluoreszenz- oder Phosphoreszenzpigmente eingesetzt werden.

Besonders geeignet sind farbige Metalloxide, -hydroxide und -oxidhydrate, Mischphasenpigmente, schwefelhaltige Silicate, Silicate, Metallsulfide, komplexe Metallcyanide, Metallsulfate, -chromate und/oder -molybdate. Insbesondere bevorzugte Pigmente sind schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510) und/oder Carmine (Cochineal).

Ebenfalls besonders bevorzugte Pigmente sind farbige Perlglanzpigmente. Diese basieren üblicherweise auf Mica- und/oder Glimmerbasis und können mit einem oder mehreren Metalloxiden beschichtet sein. Glimmer gehört zu den Schicht-Silicaten. Die wichtigsten Vertreter dieser Silicate sind Muscovit, Phlogopit, Paragonit, Biotit, Lepidolith und Margarit. Zur Herstellung der Perlglanzpigmente in Verbindung mit Metalloxiden wird der Glimmer, überwiegend Muscovit oder Phlogopit, mit einem Metalloxid beschichtet.

Alternativ zu natürlichem Glimmer kann auch ggfs. mit einem oder mehrere Metalloxide(en) beschichtetes, synthetisches Mica als Perlglanzpigment verwendet werden. Besonders bevorzugte Perlglanzpigmente basieren auf natürlichem oder synthetischem Mica (Glimmer) und sind mit einem oder mehreren der zuvor genannten Metalloxide beschichtet. Die Farbe der jeweiligen Pigmente kann durch Variation der Schichtdicke des oder der Metalloxids(e) variiert werden.

Ebenfalls bevorzugte Pigmente auf Glimmerbasis sind mit Metalloxid beschichtete synthetisch hergestellte Glimmerplättchen, insbesondere basierend auf synthetischem Fluorphlogopit (INCI: Synthetic Fluorphlogopite). Die synthetischen Fluorphlogopitplättchen sind beispielsweise mit Zinnoxid, Eisenoxid(en) und/oder Titandioxid beschichtet. Die Metalloxidschichten können ferner Pigmente wie Eisen(III)-hexacyanidoferrat(II/III) oder Karminrot aufweisen. Solche Glimmerpigmente sind beispielsweise unter der Bezeichnung SYNCRYSTAL von Eckart erhältlich.

Entsprechend ist ein bevorzugtes Verfahren dadurch gekennzeichnet, dass das Mittel (a) mindestens eine weitere farbgebende Verbindung (a2) aus der Gruppe der Pigmente enthält, die ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

In einer weiteren bevorzugten Ausführungsform ist das Verfahren dadurch gekennzeichnet, dass das Mittel (a) mindestens eine weitere farbgebende Verbindung (a2) aus der Gruppe der Pigmente enthält, die ausgewählt ist aus Pigmenten auf Mica- oder Glimmerbasis, die mit einem oder mehreren Metalloxiden aus der Gruppe aus Titandioxid (CI 77891), schwarzem Eisenoxid (CI 77499), gelbem Eisenoxid (CI 77492), rotem und/oder braunem Eisenoxid (CI 77491, CI 77499), Manganviolett (CI 77742), Ultramarine (Natrium-Aluminiumsulfosilikate, CI 77007, Pigment Blue 29), Chromoxidhydrat (CI 77289), Chromoxid (CI 77288) und/oder Eisenblau (Ferric Ferrocyanide, CI 77510) beschichtet sind.

Weitere geeignete Pigmente basieren auf Metalloxid-beschichteten plättchenförmigen Borosilikaten. Diese sind beispielsweise mit Zinnoxid, Eisenoxid(en), Siliciumdioxid und/oder Titandioxid beschichtet. Solche Borosilikat-basierten Pigmente sind beispielsweise unter der Bezeichnung MIRAGE von Eckart oder Reflecks von BASF SE erhältlich.

Beispiele für besonders geeignete Pigmente sind im Handel beispielsweise unter den Handelsbezeichnungen Rona^{®}, Colorona^{®}, Xirona^{®}, Dichrona^{®} und Timiron^{®} von der Firma Merck, Ariabel^{®} und Unipure^{®} von der Firma Sensient, Prestige^{®} von der Firma Eckart Cosmetic Colors, Flamenco^{®}, Cellini^{®}, Cloisonné^{®}, Duocrome^{®}, Gemtone^{®}, Timica^{®}, MultiReflections, Chione von der Firma BASF SE und Sunshine^{®} von der Firma Sunstar erhältlich.
Ganz besonders bevorzugte Pigmente mit der Handelsbezeichnung Colorona^{®} sind beispielsweise:
Colorona Copper, Merck, MICA, CI 77491 (IRON OXIDES)
Colorona Passion Orange, Merck, Mica, CI 77491 (Iron Oxides), Alumina
Colorona Patina Silver, Merck, MICA, CI 77499 (IRON OXIDES), CI 77891 (TITANIUM DIOXIDE)
Colorona RY, Merck, CI 77891 (TITANIUM DIOXIDE), MICA, CI 75470 (CARMINE)
Colorona Oriental Beige, Merck, MICA, CI 77891 (TITANIUM DIOXIDE), CI 77491 (IRON OXIDES)
Colorona Dark Blue, Merck, MICA, TITANIUM DIOXIDE, FERRIC FERROCYANIDE
Colorona Chameleon, Merck, CI 77491 (IRON OXIDES), MICA
Colorona Aborigine Amber, Merck, MICA, CI 77499 (IRON OXIDES), CI 77891 (TITANIUM DIOXIDE)
Colorona Blackstar Blue, Merck, CI 77499 (IRON OXIDES), MICA
Colorona Patagonian Purple, Merck, MICA, CI 77491 (IRON OXIDES), CI 77891 (TITANIUM DIOXIDE), CI 77510 (FERRIC FERROCYANIDE)
Colorona Red Brown, Merck, MICA, CI 77491 (IRON OXIDES), CI 77891 (TITANIUM DIOXIDE)
Colorona Russet, Merck, CI 77491 (TITANIUM DIOXIDE), MICA, CI 77891 (IRON OXIDES)
Colorona Imperial Red, Merck, MICA, TITANIUM DIOXIDE (CI 77891), D&C RED NO. 30 (CI 73360)
Colorona Majestic Green, Merck, CI 77891 (TITANIUM DIOXIDE), MICA, CI 77288 (CHROMIUM OXIDE GREENS)
Colorona Light Blue, Merck, MICA, TITANIUM DIOXIDE (CI 77891), FERRIC FERROCYANIDE (CI 77510)
Colorona Red Gold, Merck, MICA, CI 77891 (TITANIUM DIOXIDE), CI 77491 (IRON OXIDES)
Colorona Gold Plus MP 25, Merck, MICA, TITANIUM DIOXIDE (CI 77891), IRON OXIDES (CI 77491)
Colorona Carmine Red, Merck, MICA, TITANIUM DIOXIDE, CARMINE
Colorona Blackstar Green, Merck, MICA, CI 77499 (IRON OXIDES)
Colorona Bordeaux, Merck, MICA, CI 77491 (IRON OXIDES)
Colorona Bronze, Merck, MICA, CI 77491 (IRON OXIDES)
Colorona Bronze Fine, Merck, MICA, CI 77491 (IRON OXIDES)
Colorona Fine Gold MP 20, Merck, MICA, CI 77891 (TITANIUM DIOXIDE), CI 77491 (IRON OXIDES)
Colorona Sienna Fine, Merck, CI 77491 (IRON OXIDES), MICA
Colorona Sienna, Merck, MICA, CI 77491 (IRON OXIDES)
Colorona Precious Gold, Merck, Mica, CI 77891 (Titanium dioxide), Silica, CI 77491(Iron oxides), Tin oxide
Colorona Sun Gold Sparkle MP 29, Merck, MICA, TITANIUM DIOXIDE, IRON OXIDES, MICA, CI 77891, CI 77491 (EU)
Colorona Mica Black, Merck, CI 77499 (Iron oxides), Mica, CI 77891 (Titanium dioxide)
Colorona Bright Gold, Merck, Mica, CI 77891 (Titanium dioxide), CI 77491(Iron oxides)
Colorona Blackstar Gold, Merck, MICA, CI 77499 (IRON OXIDES)
Colorona SynCopper, Merck, Synthetic Fluorphlogopite (and) Iron Oxides
Colorona SynBronze, Merck, Synthetic Fluorphlogopite (and) Iron Oxides
Weiterhin besonders bevorzugte Pigmente mit der Handelsbezeichnung Xirona^{®} sind beispielsweise:
Xirona Golden Sky, Merck, Silica, CI 77891 (Titanium Dioxide), Tin Oxide
Xirona Caribbean Blue, Merck, Mica, CI 77891 (Titanium Dioxide), Silica, Tin Oxide
Xirona Kiwi Rose, Merck, Silica, CI 77891 (Titanium Dioxide), Tin Oxide
Xirona Magic Mauve, Merck, Silica, CI 77891 (Titanium Dioxide), Tin Oxide.
Xirona Le Rouge, Merck, Iron Oxides (and) Silica
Zudem sind besonders bevorzugte Pigmente mit der Handelsbezeichnung Unipure^{®} beispielsweise:
Unipure Red LC 381 EM, Sensient CI 77491 (Iron Oxides), Silica
Unipure Black LC 989 EM, Sensient, CI 77499 (Iron Oxides), Silica
Unipure Yellow LC 182 EM, Sensient, CI 77492 (Iron Oxides), Silica

Ebenfalls besonders bevorzugte Pigmente mit der Handelsbezeichnung Flamenco^{®} sind beispielsweise:
Flamenco^{®} Summit Turquoise T30D, BASF, Titanium Dioxide (and) Mica
Flamenco^{®} Super Violet 530Z, BASF, Mica (and) Titanium Dioxide

Im Rahmen einer weiteren Ausführungsform kann das in dem Verfahren eingesetzte Mittel (a) auch ein oder mehrere farbgebende Verbindungen (a2) aus der Gruppe der organischen Pigmente enthalten.

Bei den organischen Pigmenten handelt es sich um entsprechend unlösliche, organische Farbstoffe oder Farblacke, die beispielsweise aus der Gruppe der Nitroso-, Nitro- Azo-, Xanthen-, Anthrachinon-, Isoindolinon-, Isoindolin-, Chinacridon-, Perinon-, Perylen- , Diketopyrrolopyorrol-, Indigo-, Thioindido-, Dioxazin-, und/oder Triarylmethan-Verbindungen ausgewählt sein können.

Als besonders gut geeignete organische Pigmente können beispielsweise Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915 und/oder CI 75470 genannt werden.

In einer weiteren besonders bevorzugten Ausführungsform ist das Verfahren dadurch gekennzeichnet, dass das Mittel (a) mindestens eine weitere farbgebende Verbindungen (a2) aus der Gruppe der organischen Pigmente enthält, die ausgewählt ist aus der Gruppe bestehend aus Carmin, Chinacridon, Phthalocyanin, Sorgho, blaue Pigmente mit den Color Index Nummern CI 42090, CI 69800, CI 69825, CI 73000, CI 74100, CI 74160, gelbe Pigmente mit den Color Index Nummern CI 11680, CI 11710, CI 15985, CI 19140, CI 20040, CI 21100, CI 21108, CI 47000, CI 47005, grüne Pigmente mit den Color Index Nummern CI 61565, CI 61570, CI 74260, orange Pigmente mit den Color Index Nummern CI 11725, CI 15510, CI 45370, CI 71105, rote Pigmente mit den Color Index Nummern CI 12085, CI 12120, CI 12370, CI 12420, CI 12490, CI 14700, CI 15525, CI 15580, CI 15620, CI 15630, CI 15800, CI 15850, CI 15865, CI 15880, CI 17200, CI 26100, CI 45380, CI 45410, CI 58000, CI 73360, CI 73915, CI 75470 und Mischungen daraus.

Bei dem organischen Pigment kann es sich weiterhin auch um einen Farblack handeln. Unter der Bezeichnung Farblack wird im Sinn der Erfindung Partikel verstanden, welche eine Schicht aus absorbierten Farbstoffen umfassen, wobei die Einheit aus Partikel und Farbstoff unter den o.g. Bedingungen unlöslich ist. Bei den Partikeln kann es sich beispielsweise um anorganische Substrate handeln, die Aluminium, Silica, Calciumborosilkat, Calciumaluminiumborosilikat oder auch Aluminium sein können.

Als Farblack kann beispielsweise der Alizarin-Farblack eingesetzt werden.

Ebenfalls geeignete weitere farbgebende Verbindungen (a2) aus der Gruppe der Pigmente sind anorganische und/oder organische Pigmente, die mit einem Polymer modifiziert wurden. Durch die Polymermodifikation kann beispielsweise die Affinität der Pigmente zu dem jeweiligen Material der mindestens einen Schicht erhöht werden.

Es können auch andere Effektpigmente, wie beispielsweise Metallglanzpigmente, als weitere farbgebende Verbindung (a2) eingesetzt werden.

Die Effektpigmente können beispielsweise Pigmente auf Basis eines lamellaren Substratplättchens, Pigmente auf Basis von lentikularen Substratplättchen, Pigmente auf Basis von Substratplättchen, die "vacuum metallized pigments" (VMP) umfassen, enthalten.

Geeignete Effektpigmente umfassen beispielsweise die Pigmente Alegrace^{®} Marvelous, Alegrace^{©} Gorgeous oder Alegrace^{®} Aurous von Schlenk Metallic Pigments.

Ebenfalls geeignete Effektpigmente sind die Aluminium-basierten Pigmente der Reihe SILVERDREAM sowie die auf Aluminium oder auf kupfer-/ zinkhaltigen Metalllegierungen basierenden Pigmente der Reihe VISIONAIRE von Eckart.

Weitere geeignete Effektpigmente basieren auf Metalloxid-beschichteten plättchenförmigen Borosilikaten. Diese sind beispielsweise mit Zinnoxid, Eisenoxid(en), Siliciumdioxid und/oder Titandioxid beschichtet. Solche Borosilikat-basierten Pigmente sind beispielsweise unter der Bezeichnung MIRAGE von Eckart oder Reflecks von BASF SE erhältlich.

Im Rahmen einer weiteren Ausführungsform des Verfahrens kann das Mittel (a) auch ein oder mehrere farbgebende Verbindungen aus der Gruppe der organischen Pigmente enthalten.

Aufgrund ihrer ausgezeichneten Licht- und Temperaturbeständigkeit ist die Verwendung der zuvor genannten Pigmente in dem Mittel (a) besonders bevorzugt. Weiterhin ist es bevorzugt, wenn die eingesetzten Pigmente eine bestimmte Teilchengröße aufweisen. Diese Teilchengröße führt einerseits zu einer gleichmäßigen Verteilung der Pigmente in dem gebildeten Polymerfilm und vermeidet andererseits ein raues Haar- oder Hautgefühl nach dem Auftragen des kosmetischen Mittels. Es ist daher erfindungsgemäß vorteilhaft, wenn das mindestens eine Pigment eine mittlere Teilchengröße D₅₀ von 1 bis 50 µm, vorzugsweise von 5 bis 45 µm, bevorzugt von 10 bis 40 µm, insbesondere von 14 bis 30 µm, aufweist. Die mittlere Teilchengröße D₅₀ kann beispielsweise unter Verwendung von dynamischer Lichtstreuung (DLS) bestimmt werden.

In einer weiteren bevorzugten Ausführungsform ist das Verfahren dadurch gekennzeichnet, dass das Mittel (a) - bezogen auf das Gesamtgewicht des Mittels (a) - ein oder mehrere weitere farbgebende Verbindung(en) (a2) in Form von Pigmenten in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

Als weitere farbgebende Verbindung(en) (a2) können die in dem Verfahren angewendeten Mittel (a) auch einen oder mehrere direktziehende Farbstoffe enthalten. Bei direktziehenden Farbstoffen handelt es sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone, Triarylmethanfarbstoffe oder Indophenole.

Die direktziehenden Farbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die direktziehenden Farbstoffe im Sinne der vorliegenden Erfindung eine Löslichkeit in Wasser (760 mmHg) bei 25 °C von mehr als 1 g/L.

Direktziehende Farbstoffe können in anionische, kationische und nichtionische direktziehende Farbstoffe unterteilt werden.

In einer weiteren bevorzugten Ausführungsform ist das Verfahren dadurch gekennzeichnet, dass das Mittel (a) als weitere farbgebende Verbindung (a2) mindestens einen anionischen, kationischen und/oder nichtionischen direktziehenden Farbstoff enthält.

In einer weiteren bevorzugten Ausführungsform ist das Verfahren dadurch gekennzeichnet, dass das Mittel (a) mindestens eine weitere farbgebende Verbindung (a2) aus der Gruppe der anionischen, nichtionischen, und/oder kationischen direktziehenden Farbstoffe enthält.

Geeignete kationische direktziehende Farbstoffe sind beispielsweise Basic Blue 7, Basic Blue 26, Basic Violet 2 und Basic Violet 14, Basic Yellow 57, Basic Red 76, Basic Blue 16, Basic Blue 347 (Cationic Blue 347 / Dystar), HC Blue No. 16, Basic Blue 99, Basic Brown 16, Basic Brown 17, Basic Yellow 57, Basic Yellow 87, Basic Orange 31, Basic Red 51 Basic Red 76

Als nichtionische direktziehende Farbstoffe können beispielsweise nichtionische Nitro- und Chinonfarbstoffe und neutrale Azofarbstoffe eingesetzt werden. Geeignete nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)-aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)-amino-5-chlor-2-nitrobenzol, 4-Amino-3-nitrophenol, 1-(2'-Ureidoethyl)amino-4-nitrobenzol, 2-[(4-Amino-2-nitrophenyl)amino]-benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Im Zuge der zu dieser Erfindung führenden Arbeiten hat sich herausgestellt, dass insbesondere mit Mitteln (a), die mindestens einen anionischen direktziehenden Farbstoff enthalten, Färbungen mit besonders hoher Farbintensität erzeugt werden können.

In einer explizit ganz besonders bevorzugten Ausführungsform ist das Verfahren daher dadurch gekennzeichnet, dass das Mittel (a) ferner mindestens einen anionischen direktziehenden Farbstoff als weitere farbgebende Verbindung (a2) enthält.

Anionische direktziehende Farbstoffe werden auch als Säurefarbstoffe bezeichnet. Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO⁻, -SO₃⁻ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen neutralisiert. Die Säurefarbstoffe können auch in Form ihrer Natriumsalze und/oder ihrer Kaliumsalze eingesetzt werden.

Die Säurefarbstoffe im Sinne der vorliegenden Erfindung besitzen eine Löslichkeit in Wasser (760 mmHg) bei 25 °C von mehr als 0,5 g/L und sind daher nicht als Pigmente anzusehen. Bevorzugt besitzen die Säurefarbstoffe im Sinne der vorliegenden Erfindung besitzen eine Löslichkeit in Wasser (760 mmHg) bei 25 °C von mehr als 1 g/L.

Die Erdalkalisalze (wie beispielsweise Calciumsalze und Magnesiumsalze) bzw. Aluminiumsalze von Säurefarbstoffen besitzen oftmals eine schlechtere Löslichkeit als die entsprechenden Alkalisalze. Sofern die Löslichkeit dieser Salze unterhalb von 0,5 g/L (25 °C, 760 mmHg) liegt, fallen diese nicht unter die Definition eines direktziehenden Farbstoffes.

Ein wesentliches Merkmal der Säurefarbstoffe ist ihr Vermögen, anionische Ladungen auszubilden, wobei die hierfür verantwortlichen Carbonsäure- bzw. Sulfonsäuregruppen üblicherweise an verschiedene chromophore Systeme geknüpft sind. Geeignete chromophore Systeme finden sich beispielsweise in den Strukturen von Nitrophenylendiaminen, Nitroaminophenolen, Azofarbstoffen, Anthrachinonfarbstoffen, Triarylmethanfarbstoffen, Xanthen-Farbstoffen, Rhodamin-Farbstoffen, Oxazinfarbstoffen und/oder Indophenolfarbstoffen.

Im Rahmen einer Ausführungsform bevorzugt ist damit ein Verfahren zum Färben von keratinischem Material, welches dadurch gekennzeichnet ist, dass das Mittel (a) ferner mindestens einen anionischen direktziehenden Farbstoff als weitere farbgebende Verbindung (a2) enthält, der ausgewählt ist aus der Gruppe der Nitrophenylendiamine, der Nitroaminophenole, der Azofarbstoffe, der Anthrachinonfarbstoffe, der Triarylmethanfarbstoffe, der Xanthen-Farbstoffe, der Rhodamin-Farbstoffe, der Oxazinfarbstoffen und/oder der Indophenolfarbstoffe, wobei die Farbstoffe aus der vorgenannten Gruppe jeweils mindestens eine Carbonsäuregruppe (-COOH), eine Natriumcarboxylatgruppe (-COONa), eine Kaliumcarboxylatgruppe (-COOK), eine Sulfonsäuregruppe (-SO₃H) eine Natriumsulfonatgruppe (-SO₃Na) und/oder eine Kaliumsulfonatgruppe (-SO₃K) besitzen.

Als besonders gut geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403,CI 10316, COLIPA n° B001), Acid Yellow 3 (COLIPA n° : C 54, D&C Yellow N° 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 92 (COLIPA n° C53, CI 45410), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Die Wasserlöslichkeit der anionischen direktziehenden Farbstoffe kann beispielsweise auf dem folgenden Weg bestimmt werden. 0,1 g des anionischen direktziehenden Farbstoffes werden in ein Becherglas gegeben. Es wird ein Rührfisch hinzugegeben. Dann werden 100 ml Wasser hinzugefügt. Diese Mischung wird auf einem Magnetrührer unter Rühren auf 25 °C erwärmt. Es wird für 60 Minuten gerührt. Danach wird die wässrige Mischung visuell beurteilt. Sind noch ungelöste Reste vorhanden, wird die Wassermenge - beispielsweise in Schritten von 10 ml - erhöht. Es wird solange Wasser zugegeben, bis sich die eingesetzte Farbstoffmenge komplett gelöst hat. Sofern sich die Farbstoff-Wasser-Mischung aufgrund der hohen Intensität des Farbstoffes nicht visuell beurteilten lässt, wird die Mischung filtriert. Bleibt auf dem Filterpapier ein Anteil an ungelösten Farbstoffen zurück, wird der Löslichkeitsversuch mit einer höheren Wassermenge wiederholt. Lösen sich 0,1 g des anionischen direktziehenden Farbstoffes bei 25 °C in 100 ml Wasser, so liegt die Löslichkeit des Farbstoffes bei 1 g/L.

Acid Yellow 1 trägt den Namen 8-Hydroxy-5,7-dinitro-2-naphthalenesulfonsäure Dinatriumsalz und besitzt eine Löslichkeit in Wasser von mindestens 40 g/L (25°C).

Acid Yellow 3 ist ein Gemisch der Natriumsalze von Mono- und Disulfonsäuren von 2-(2-Chinolyl)-1H-indene-1,3(2H)-dion und besitzt eine Wasserlöslichkeit von 20 g/L (25 °C).

Acid Yellow 9 ist das Dinatriumsalz der 8-Hydroxy-5,7-dinitro-2-naphthalenesulfonsäure, seine Wasserlöslichkeit liegt oberhalb von 40 g/L (25 °C).

Acid Yellow 23 ist das Trinatriumsalz der4,5-Dihydro-5-oxo-1-(4-sulfophenyl)-4-((4-sulfophenyl)azo)-1H-pyrazole-3-carbonsäure und bei 25 °C gut in Wasser löslich.

Acid Orange 7 ist das Natriumsalz des 4-[(2-Hydroxy-1-naphthyl)azo]benzensulfonats. Seine Wasserlöslichkeit beträgt mehr als 7 g/L (25 °C).

Acid Red 18 ist das Trinatriumsalz von 7-Hydroxy-8-[(E)-(4-sulfonato-1-naphthyl)-diazenyl)]-1,3-naphthalenedisulfonat und besitzt eine sehr hohe Wasserlöslichkeit von mehr als 20 Gew.-%.

Acid Red 33 ist das Dinatriumsalz des 5-Amino-4-hydroxy-3-(phenylazo)-naphthalene-2,7-disulphonats, seine Wasserlöslichkeit liegt bei 2,5 g/L (25 °C).

Bei Acid Red 92 handelt es sich um das Dinatriumsalz der 3,4,5,6-Tetrachloro-2-(1,4,5,8-tetrabromo-6-hydroxy-3-oxoxanthen-9-yl)benzoesäure, dessen Wasserlöslichkeit mit größer 10 g/L angegeben wird (25 °C).

Acid Blue 9 ist das Dinatriumsalz von 2-({4-[N-ethyl(3-sulfonatobenzyl]amino]phenyl}{4-[(N-ethyl(3-sulfonatobenzyl)imino]-2,5-cyclohexadien-1-ylidene}methyl)-benzenesulfonat und besitzt eine Wasserlöslichkeit von mehr als 20 Gew.-% (25 °C).

Ein ganz besonders bevorzugtes Verfahren ist daher dadurch gekennzeichnet, dass das Mittel (a) mindestens eine weitere farbgebende Verbindung (a2) aus der Gruppe der anionischen direktziehenden Farbstoffe enthält, die ausgewählt ist aus der Gruppe aus Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red, Acid Red 27, Acid Red 33, Acid Red 35, Acid Red 51, Acid Red 52, Acid Red 73, Acid Red 87, Acid Red 92, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 8, D&C Green 5, D&C Orange 10, D&C Orange 11, D&C Red 21, D&C Red 27, D&C Red 33, D&C Violet 2 und/oder D&C Brown 1.

Der oder die direktziehenden Farbstoffe, insbesondere die anionischen direktziehenden Farbstoffe, können je nach erwünschter Farbintensität in verschiedenen Mengen im Mittel (a) eingesetzt werden. Besonders gute Ergebnisse konnten erhalten werden, wenn das Mittel (a) - bezogen auf sein Gesamtgewicht - ein oder mehrere direktziehende Farbstoffe als weitere farbgebende Verbindung (a2) in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

In einer weiteren bevorzugten Ausführungsform ist das Verfahren dadurch gekennzeichnet, dass das Mittel (a) - bezogen auf das Gesamtgewicht des Mittels (a) - ferner ein oder mehrere direktziehende Farbstoffe als weitere farbgebende Verbindung (a2) in einer Gesamtmenge von 0,01 bis 10 Gew.-%, bevorzugt von 0,1 bis 8 Gew.-%, weiter bevorzugt von 0,2 bis 6 Gew.-% und ganz besonders bevorzugt von 0,5 bis 4,5 Gew.-% enthält.

### Silikonpolymere (a3)

In einer weiteren ganz besonders bevorzugten Ausführungsform enthält das in dem Verfahren eingesetzte Mittel (a) zusätzlich mindestens ein Silikonpolymer (a3).

Unter Silikonpolymeren, die alternativ verkürzt auch Silikone genannt werden können, werden Poly(organo)siloxane verstanden. Unter Silikonpolymeren wird eine Gruppe synthetischer Polymere verstanden, bei denen Siliciumatome über Sauerstoffatome verknüpft sind.

Silikonpolymere sind im allgemeinen Makromoleküle mit einem Molekulargewicht von mindestens 500 g/mol, bevorzugt mindestens 1000 g/mol, weiter bevorzugt von mindestens 2500 g/mol, besonders bevorzugt von mindestens 5000 g/mol, welche sich wiederholende organische Einheiten umfassen.

Das maximale Molekulargewicht des Silikonpolymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des Silikonpolymers nicht mehr als 10⁷ g/mol, bevorzugt nicht mehr als 10⁶ g/mol und besonders bevorzugt nicht mehr als 10⁵ g/mol beträgt.

Die Silikonpolymere umfassen viele Si-O-Wiederholungseinheiten, wobei die Si-Atome organische Reste wie beispielsweise Alkylgruppen oder substituierte Alkylgruppen tragen können.

In Entsprechung des hohen Molekulargewichts der Silikonpolymere basieren diese auf mehr als 10 Si-O Wiederholungseinheiten, bevorzugt mehr als 50 Si-O-Wiederholungseinheiten und besonders bevorzugt mehr als 100 Si-O-Wiederholungseinheiten, ganz besonders bevorzugt mehr als 500 Si-O-Wiederholungseinheiten.

Die im Mittel (a) enthaltenen Silikonpolymere (a3) sind daher von den ebenfalls im Mittel (a) enthaltenen Silanen (a1) verschieden.

Im Rahmen einer Ausführungsform bevorzugt ist damit ein Verfahren zum Färben von keratinischem Material, welches dadurch gekennzeichnet ist, dass das Mittel (a) enthält:
(a3) mindestens ein Silikonpolymer.

In den zu dieser Erfindung führenden Arbeiten hat sich herausgestellt, dass die Einarbeitung des Silikonpolymers (a3) in das Mittel (a) zu einer Verbesserung des Haargefühls führte.

Der durch die Oligomerisierung bzw. Polymerisierung der siliciumorganischen Verbindungen (Silane) (a1) erzeugte Film kann - insbesondere bei höheren Einsatzmengen an Silanen (a1) - eine gewisse Klebrigkeit oder auch Weichheit aufweisen, die sich zum einen nachteilig auf die Haptik der keratinischen Materialien, zum anderen aber auch negativ auf die Beständigkeit des Films auswirken kann. Ohne auf diese Theorie festgelegt zu sein, wird vermutet, dass die gemeinsame Anwendung des Silans (a1) und des Silikonpolymers (a3) im Mittel (a) zu einer Reaktion oder Interaktion der beiden Komponenten miteinander führt. Bei gemeinsamer Anwendung von Silan und Silikonpolymer scheinen die Silane wie zuvor beschrieben einen Film auszubilden, in den die Silikonpolymere entweder eingelagert werden, oder an den die Silikonpolymere agglomerieren. Es hat sich herausgestellt, dass der auf diese Weise ausgebildete Film wesentlich geschmeidiger, flexibler, beständiger und weniger brüchig ist.

Demzufolge konnte beobachtet werden, dass die rheologischen Eigenschaften des mit dem Mittel (a) erzeugten Films sich durch den Zusatz mindestens eines Silikonpolymers (a3) stark verbessern ließen. In Anwesenheit der Silikonpolymere (a3) wurde der Film fester bzw. rigider, so dass die gefärbten keratinischen Materialien einen weniger klebrigen, glatteren und angenehmeren Eindruck hinterließen. Des Weiteren hatte die höhere Festigkeit des Films auch positive Auswirkungen auf die Echtheitseigenschaften der keratinischen Materialien, insbesondere auf deren Reibechtheiten. Da die gefärbten Filme bei Kontakt mit Kamm, Bürste und Textilien widerstandsfähiger waren, zeigte sich bei Kontakt mit diesen Gegenständen ein weniger starker Abrieb.

Bei Einsatz von bestimmten Silikonpolymeren (a3) waren die zuvor beschriebenen Vorteile ganz besonders ausgeprägt. Es hat sich daher als ganz besonders bevorzugt herausgestellt, wenn die im Verfahren eingesetzten Mittel (a) mindestens ein alkoxy-modifiziertes Silikonpolymer und/oder mindestens ein amino-modifiziertes Silikonpolymer (a3) enthalten.

Im Rahmen einer Ausführungsform bevorzugt ist damit ein Verfahren zum Färben von keratinischem Material, welches dadurch gekennzeichnet ist, dass das Mittel (a) enthält:
(a3) mindestens ein alkoxy-modifiziertes und/oder amino-modifiziertes Silikonpolymer.

In einer weiteren bevorzugten Ausführungsform ist ein Verfahren dadurch gekennzeichnet, dass das Mittel (a) mindestens ein alkoxy-modifiziertes Silikonpolymer enthält.

Unter alkoxy-modifizierten Silikonen werden Silikone verstanden, deren Struktur mindestens eine strukturelle Alkoxy-Einheit umfasst. Diese strukturelle Alkoxy-Einheit kann beispielsweise eine Alkoxy-Gruppe sein. Unter Alkoxygruppen werden C₂-C₁₀-Alkoxygruppen verstanden. Die Alkoxygruppe kann sich endständig am Silikon befinden (d.h. zum Beispiel als Gruppe -O-CH₃ oder als Gruppe -O-CH₂-CH₃ vorliegen). Ebenso ist es aber erfindungsgemäß, wenn die Alkoxygruppe selbst noch einen Substituenten trägt; in diesem Fall wird unter einer Alkoxy-Modifizierung mindestens eine am Silikon befindliche Gruppierung wie beispielsweise (-CH2-CH2-O-), (-CH2-CH2-CH2-O-), (-CH(CH3)-CH2-O-), (-CH2-CH(CH3)-CH2-O-) oder (-CH2-CH2-CH2-CH2-O-) verstanden. Bevorzugt tragen die alkoxy-modifizierten Silikone (A) mindestens eine Gruppierung (-CH2-CH2-O-) und/oder (-CH2-CH2-CH2-O-).

Die Alkoxygruppen können entweder über ein Kohlenstoffatom oder über ein Sauerstoffatom mit dem Silikon verknüpft sein, beispielsweise können die Silikone die Struktureinheiten der Formel (S-a), (S-b), (S-c) und/oder (S-d) tragen:

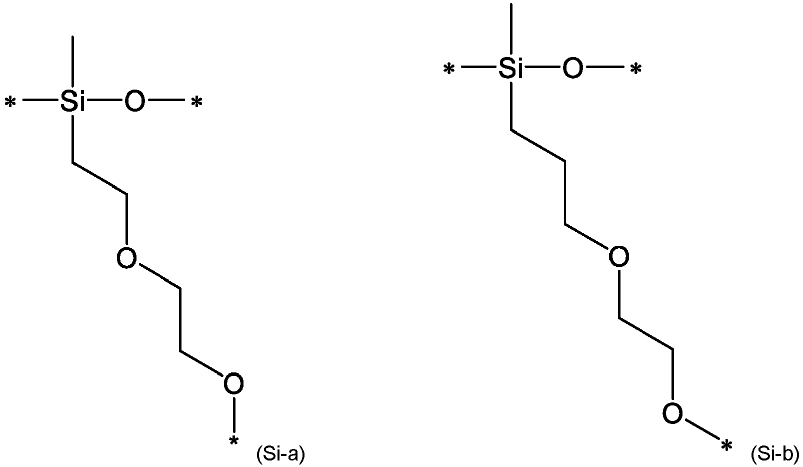

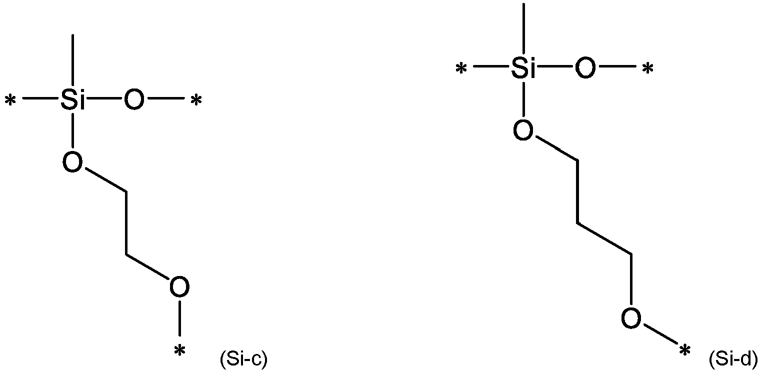

Besonders bevorzugt ist es, wenn das bzw. die alkoxy-modifizierten Silikonpolymere (a3) mehr als eine Alkoxy-Gruppe tragen, d.h. wenn die Silikonpolymere (a3) polyalkoxyliert sind. Polyalkoxylierte Silikone tragen als Struktureinheiten Polyoxyalkylengruppen, insbesondere Polyoxyethylengruppen (d.h. Gruppen des Typs [-CH2-CH2-O-]ₘ) und/oder Polyoxypropylengruppen (d.h. Gruppen des Typs [-CH(CH3)-CH2-O-]ₘ und/oder [-CH2-CH2-CH2-O-]ₘ). Bevorzugt liegt die Zahl der Polyoxyalkyleineinheiten im Silikon-Polymer liegt bei mindestens 2. m ist daher eine ganze Zahl größer oder gleich 2.

Besonders bevorzugt handelt es sich bei dem alkoxy-modifizierten Silikon (a3) um ein nichtionisches Silikon. Nichtionische Silikone tragen weder positive noch negative Ladungen.

Ganz besonders gut geeignete polyalkoxylierte Silikone (a3) umfassen mindestens eine Struktureinheit der Formel (S-I) 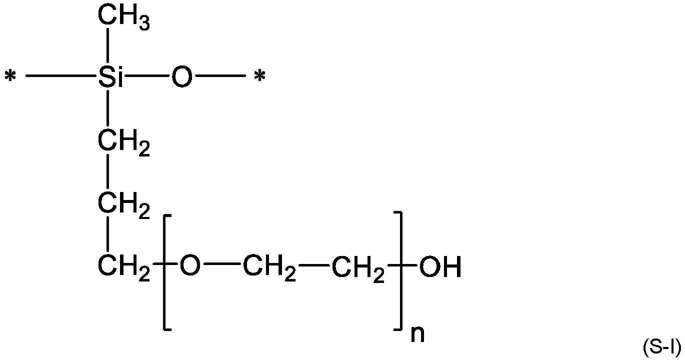 worin
n für eine ganze Zahl von 2 bis 20, bevorzugt für eine ganze Zahl von 4 bis 18, weiter bevorzugt für eine ganze Zahl von 6 bis 16, noch weiter bevorzugt für eine ganze Zahl von 8 bis 14 und ganz besonders bevorzugt für die Zahl 12 steht.

Die mit einem Stern * gekennzeichneten Stellen in den o.g. Formeln stehen hierbei für die freien Valenzen der entsprechenden Bindungen, wobei die Bindung zu einem weiteren Si-Atom, einem weiteren O-Atom und/oder einem weiteren C-Atom erfolgen kann.

Im Rahmen einer Ausführungsform bevorzugt ist damit ein Verfahren zum Färben von keratinischem Material, welches dadurch gekennzeichnet ist, dass das Mittel (a) enthält:
(a3) mindestens ein Silikonpolymer, welches mindestens eine Struktureinheit der Formel (S-I) umfasst 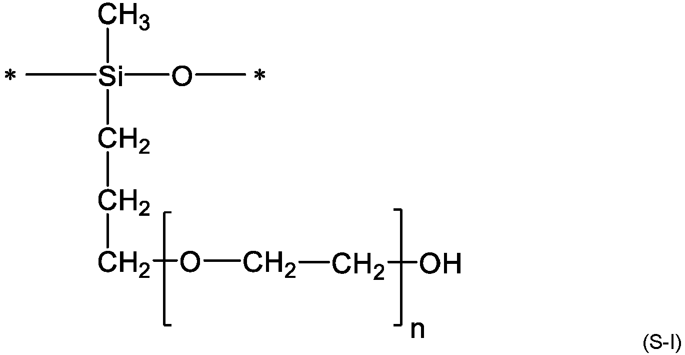 worin
n für eine ganze Zahl von 2 bis 20, bevorzugt für eine ganze Zahl von 4 bis 18, weiter bevorzugt für eine ganze Zahl von 6 bis 16, noch weiter bevorzugt für eine ganze Zahl von 8 bis 14 und ganz besonders bevorzugt für die Zahl 12 steht.

Ein bevorzugtes alkoxy-modifiziertes Silikonpolymer (a3) kann neben einer oder mehrerer Struktureinheiten der allgemeinen Formel (S-I) auch weitere Struktureinheiten enthalten, die sich strukturell von den Einheiten der Formel (S-I) unterscheiden. Besonders bevorzugt umfasst das alkoxy-modifizierte Silikonpolymer zusätzlich ein oder mehrere Dimethylsiloxaneinheiten. Je nachdem, ob das Silikon linear oder verzweigt ist, besitzt es zwei (im Falle eines kettenförmigen, linearen Silikons) oder mehrere (im Falle eines verzweigten Silikons) Endgruppen. Als besonders vorteilhaft hat es sich herausgestellt, wenn ein Silikonpolymer (a3) als Endgruppen jeweils eine Trimethylsilyloxy-Gruppe (d.h. eine Gruppe -O-Si(CH₃)₃) besitzt.

In einer weiteren ganz besonders bevorzugten Ausführungsform ist das Verfahren daher dadurch gekennzeichnet, dass das Mittel (a) mindestens ein Silikonpolymer (a3) enthält, welches sich aus Struktureinheiten der Formel (S-I), der Formel (S-II), der Formel (S-III) und der Formel (S-IV) zusammensetzt, 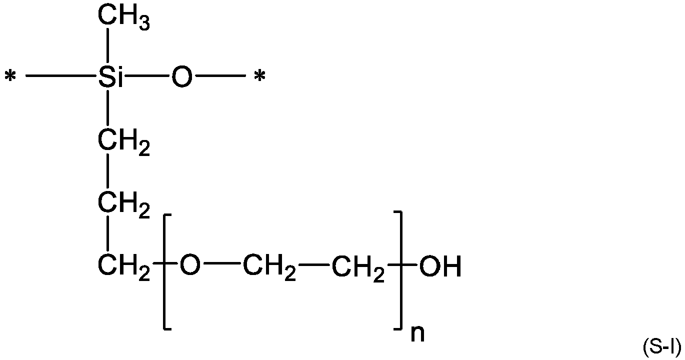 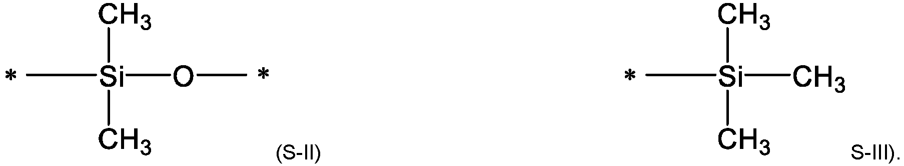 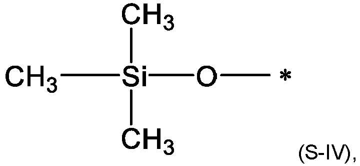 worin n - unabhängig in jeder Struktureinheit (S-I) - jeweils für eine ganze Zahl von 2 bis 20, bevorzugt für eine ganze Zahl von 4 bis 18, weiter bevorzugt für eine ganze Zahl von 6 bis 16, noch weiter bevorzugt für eine ganze Zahl von 8 bis 14 und ganz besonders bevorzugt für die Zahl 12 steht.

Unter einem Silikonpolymer (a3), das sich aus Struktureinheiten der Formel (S-I), der Formel (S-II), der Formel (S-III) und der Formel (S-IV) zusammensetzt, wird in diesem Zusammenhang ein Silikon verstanden, welches ausschließlich (jeweils eine oder mehrere) Struktureinheiten der Formeln (S-I), (S-II), (S-III) und (S-IV) besitzt. Hierbei kann das Silikon auch verschiedene Struktureinheiten der Formel (S-I) enthalten, die sich jeweils durch ihre Zahl n unterscheiden.

Die in den vorgenannten Struktureinheiten mit einem Stern gekennzeichneten Positionen stellen jeweils die Verknüpfungsstellen zu den anderen Struktureinheiten dar. Beispielsweise kann ein ganz besonders bevorzugtes Silikonpolymer (a3), welches sich aus Struktureinheiten der Formel (S-I), der Formel (S-II), der Formel (S-III) und der Formel (S-IV) zusammensetzt, die folgende Struktur besitzen: 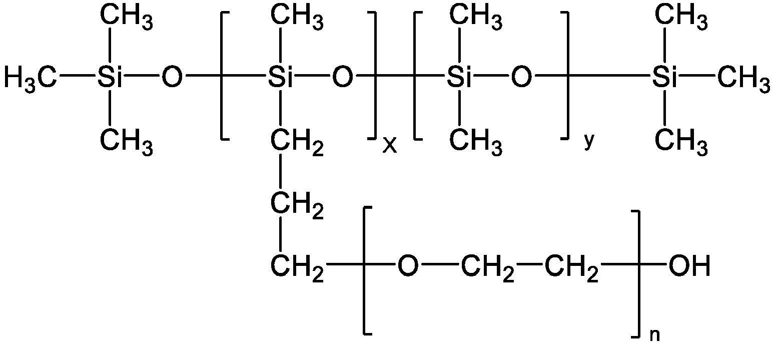 x und y werden hierbei abhängig vom gewünschten Molekulargewicht des Silikons gewählt, und n stellt eine der oben beschriebenen erfindungsgemäßen, bevorzugten oder besonders bevorzugten ganzen Zahlen dar.

Als Silikonpolymere (a3) können sowohl niedermolekulare als auch höhermolekulare alkoxy-modifizierte Silikone eingesetzt werden. Besonders vorteilhafte Effekte wurden bei Silikonpolymeren (a3) mit einer Molmasse von 800 bis 10.000 g/mol, bevorzugt von 1.000 bis 9.000 g/mol, weiter bevorzugt von 2.000 bis 8.000 g/mol und besonders bevorzugt von 2.500 bis 5.000 g/mol beobachtet.

Besonders gut geeignete Silikonpolymere sind beispielsweise:
Abil B 8843 der Firma Evonik, PEG-14 DIMETHICONE
Xiameter OFX 0193 Fluid der Firma Dow Corning, PEG-12 Dimethicone

Weiterhin konnten auch ganz besonders gute Ergebnisse konnten erhalten werden, wenn in dem Verfahren ein Mittel (a) eingesetzt wurde, welches ein amino-modifiziertes Silikonpolymer (a3) enthält. Das amino-modifizierte Silikonpolymer kann alternativ auch als aminofunktionalisiertes Silikonpolymer oder auch als Aminosilikon bezeichnet werden.

In einer weiteren bevorzugten Ausführungsform ist ein Verfahren dadurch gekennzeichnet, dass das Mittel (a) mindestens ein amino-modifiziertes Silikonpolymer enthält.

Das Mittel (a) kann ein oder mehrere verschiedene amino-modifizierte Silikonpolymere (a3) enthalten. Solche Silicone können z.B. durch die Formel (S-V)

M(RₐQ_{b}SiO_{(4-a-b)/2})ₓ(R_{c}SiO_{(4-c)/2)y}M (S-V)

beschrieben werden, wobei in der obigen Formel R ein Kohlenwasserstoff oder ein Kohlenwasserstoffrest mit 1 bis etwa 6 Kohlenstoffatomen ist, Q ein polarer Rest der allgemeinen Formel -R¹HZ ist, worin R¹ eine zweiwertige, verbindende Gruppe ist, die an Wasserstoff und den Rest Z gebunden ist, zusammengesetzt aus Kohlenstoff- und Wasserstoffatomen, Kohlenstoff-, Wasserstoff- und Sauerstoffatomen oder Kohlenstoff-, Wasserstoff- und Stickstoffatomen, und Z ein organischer, aminofunktioneller Rest ist, der mindestens eine aminofunktionelle Gruppe enthält; "a" Werte im Bereich von etwa 0 bis etwa 2 annimmt, "b" Werte im Bereich von etwa 1 bis etwa 3 annimmt, "a" + "b" kleiner als oder gleich 3 ist, und "c" eine Zahl im Bereich von etwa 1 bis etwa 3 ist, und x eine Zahl im Bereich von 1 bis etwa 2.000, vorzugsweise von etwa 3 bis etwa 50 und am bevorzugtesten von etwa 3 bis etwa 25 ist, und y eine Zahl im Bereich von etwa 20 bis etwa 10.000, vorzugsweise von etwa 125 bis etwa 10.000 und am bevorzugtesten von etwa 150 bis etwa 1.000 ist, und M eine geeignete Silicon-Endgruppe ist, wie sie im Stand der Technik bekannt ist, vorzugsweise Trimethylsiloxy. Nicht einschränkende Beispiele der durch R repräsentierten Reste schließen Alkylreste, wie Methyl, Ethyl, Propyl, Isopropyl, Isopropyl, Butyl, Isobutyl, Amyl, Isoamyl, Hexyl, Isohexyl und ähnliche; Alkenylreste, wie Vinyl, Halogenvinyl, Alkylvinyl, Allyl, Halogenallyl, Alkylallyl; Cycloalkylreste, wie Cyclobutyl, Cyclopentyl, Cyclohexyl und ähnliche; Phenylreste, Benzylreste, Halogenkohlenwasserstoffreste, wie 3-Chlorpropyl, 4-Brombutyl, 3,3,3-Trifluorpropyl, Chlorcyclohexyl, Bromphenyl, Chlorphenyl und ähnliche sowie schwefelhaltige Reste, wie Mercaptoethyl, Mercaptopropyl, Mercaptohexyl, Mercaptophenyl und ähnliche ein; vorzugsweise ist R ein Alkylrest, der 1 bis etwa 6 Kohlenstoffatomen enthält, und am bevorzugtesten ist R Methyl. Beispiele von R¹ schließen Methylen, Ethylen, Propylen, Hexamethylen, Decamethylen, -CH₂CH(CH₃)CH₂-, Phenylen, Naphthylen, -CH₂CH₂SCH₂CH ₂-, -CH₂CH₂OCH₂-, -OCH₂CH₂-, -OCH₂ CH₂CH₂-, -CH₂CH(CH₃)C(O)OCH₂-, -(CH₂)₃ CC(O)OCH₂CH₂-, -C₆H ₄C₆H₄-, -C₆H ₄CH₂C₆H₄-; und -(CH ₂)₃C(O)SCH₂CH₂- ein.

Z ist ein organischer, aminofunktioneller Rest, enthaltend mindestens eine funktionelle Aminogruppe. Eine mögliche Formel für Z ist NH(CH₂ )_{z}NH₂, worin z 1 oder mehr ist. Eine andere mögliche Formel für Z ist -NH(CH₂)_{z}(CH ₂)_{zz}NH, worin sowohl z als auch zz unabhängig 1 oder mehr sind, wobei diese Struktur Diamino-Ringstrukturen umfasst, wie Piperazinyl. Z ist am bevorzugtesten ein -NHCH₂CH ₂NH₂-Rest. Eine andere mögliche Formel für Z ist - N(CH₂)_{z}(CH₂)_{zz}NX₂ oder -NX₂, worin jedes X von X₂ unabhängig ausgewählt ist aus der Gruppe bestehend aus Wasserstoff und Alkylgruppen mit 1 bis 12 Kohlenstoffatomen, und zz 0 ist.

Q ist am bevorzugtesten ein polarer, aminfunktioneller Rest der Formel -CH₂CH₂CH₂NHCH₂CH₂NH ₂. In den Formeln nimmt "a" Werte im Bereich von etwa 0 bis etwa 2 an, "b" nimmt Werte im Bereich von etwa 2 bis etwa 3 an, "a" + "b" ist kleiner als oder gleich 3, und "c" ist eine Zahl im Bereich von etwa 1 bis etwa 3. Das molare Verhältnis der RₐQ_{b} SiO_{(4-a-b)/2}-Einheiten zu den R_{c}SiO _{(4-c)/2}-Einheiten liegt im Bereich von etwa 1 : 2 bis 1 : 65, vorzugsweise von etwa 1 : 5 bis etwa 1 : 65 und am bevorzugtesten von etwa 1 : 15 bis etwa 1 : 20. Werden ein oder mehrere Silicone der obigen Formel eingesetzt, dann können die verschiedenen variablen Substituenten in der obigen Formel bei den verschiedenen Silikonkomponenten, die in der Silikonmischung vorhanden sind, verschieden sein.

Im Rahmen einer besonders bevorzugten Ausführungsform ist ein Verfahren gekennzeichnet durch die Anwendung eines Mittels (a) auf dem keratinischem Material, wobei das Mittel (a) ein amino-modifiziertes Silikonpolymer (a3) der Formel (S-VI)

R'ₐG₃₋ₐ-Si(OSiG ₂)ₙ-(OSiG _{b}R'_{2-b})ₘ-O-SiG₃₋ₐ-R'ₐ (S-VI),

enthält, worin bedeutet:
- G ist-H, eine Phenylgruppe, -OH, -O-CH₃, -CH₃, -O-CH₂CH₃, -CH₂CH₃, -O-CH₂CH₂CH₃,-CH₂CH₂CH₃, -O-CH(CH₃)₂, -CH(CH₃)₂, -O-CH₂CH₂CH₂CH₃, -CH₂CH₂CH₂CH₃, -O-CH₂CH(CH₃)₂, -CH₂CH(CH₃)₂, -O-CH(CH₃)CH₂CH₃, -CH(CH₃)CH₂CH₃, -O-C(CH₃)₃, -C(CH₃)₃
- a steht für eine Zahl zwischen 0 und 3, insbesondere 0;
- b steht für eine Zahl zwischen 0 und 1, insbesondere 1,
- m und n sind Zahlen, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt,
- R' ist ein monovalenter Rest ausgewählt aus
   ∘ -Q-N(R")-CH₂-CH₂-N(R")₂
   ∘ -Q-N(R")₂
   ∘ -Q-N⁺(R")₃A⁻
   ∘ -Q-N⁺H(R")₂ A⁻
   ∘ -Q-N⁺H₂(R")A⁻
   ∘ -Q-N(R")-CH₂-CH₂-N⁺R"H₂A⁻ ,

   wobei jedes Q für eine chemische Bindung, -CH₂-, -CH₂-CH₂-, -CH₂CH₂CH₂-, -C(CH₃)₂-, - CH₂CH₂CH₂CH₂-, -CH₂C(CH₃)₂-, -CH(CH₃)CH₂CH₂- steht,
   R" für gleiche oder verschiedene Reste aus der Gruppe -H, -Phenyl, -Benzyl, -CH₂-CH(CH₃)Ph, der C₁₋₂₀-Alkylreste, vorzugsweise -CH₃, -CH₂CH₃, -CH₂CH₂CH₃, - CH(CH₃)₂, -CH₂CH₂CH₂H₃, -CH₂CH(CH₃)₂, -CH(CH₃)CH₂CH₃, -C(CH₃)₃, steht und A ein Anion repräsentiert, welches vorzugsweise ausgewählt ist aus Chlorid, Bromid, Iodid oder Methosulfat.

Im Rahmen einer weiteren bevorzugten Ausführungsform ist ein Verfahren gekennzeichnet durch die Anwendung eines Mittels (a) auf dem keratinischem Material, wobei das Mittel (a) mindestens ein amino-modifiziertes Silikonpolymer (a3) der Formel (S-VII) enthält, 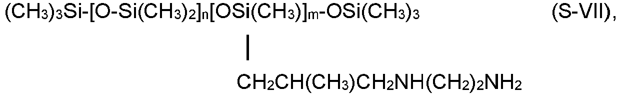 worin m und n Zahlen sind, deren Summe (m + n) zwischen 1 und 2000, vorzugsweise zwischen 50 und 150 beträgt, wobei n vorzugsweise Werte von 0 bis 1999 und insbesondere von 49 bis 149 und m vorzugsweise Werte von 1 bis 2000, insbesondere von 1 bis 10 annimmt.

Diese Silicone werden nach der INCI-Deklaration als Trimethylsilylamodimethicone bezeichnet.

Im Rahmen einer weiteren bevorzugten Ausführungsform ist ein Verfahren gekennzeichnet durch die Anwendung eines Mittels (a) auf dem keratinischem Material, wobei das Mittel (a) mindestens ein amino-modifiziertes Silikonpolymer (a3) der Formel (S-VIII) enthält 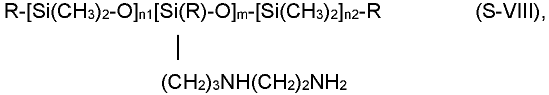 enthalten, worin R für -OH, -O-CH₃ oder eine -CH₃-Gruppe steht und m, n1 und n2 Zahlen sind, deren Summe (m + n1 + n2) zwischen 1 und 2000, vorzugsweise zwischen 50 und 150 beträgt, wobei die Summe (n1 + n2) vorzugsweise Werte von 0 bis 1999 und insbesondere von 49 bis 149 und m vorzugsweise Werte von 1 bis 2000, insbesondere von 1 bis 10 annimmt.

Diese amino-modifiziertes oder auch aminofunktionalisierten Silikonpolymere werden nach der INCI-Deklaration als Amodimethicone bezeichnet.

Unabhängig davon, welche amino-modifizierten Silicone eingesetzt werden, sind Mittel (a) bevorzugt, die ein amino-modifiziertes Silikonpolymer enthalten, dessen Aminzahl oberhalb von 0,25 meq/g, vorzugsweise oberhalb von 0,3 meq/g und insbesondere oberhalb von 0,4 meq/g liegt. Die Aminzahl steht dabei für die Milli-Äquivalente Amin pro Gramm des aminofunktionellen Silicons. Sie kann durch Titration ermittelt und auch in der Einheit mg KOH/g angegeben werden.

Im Rahmen einer weiteren bevorzugten Ausführungsform ist ein Verfahren gekennzeichnet durch die Anwendung eines Mittels (a) auf dem keratinischem Material, wobei das Mittel (a) mindestens ein amino-modifiziertes Silikonpolymer (a3) der Formel der Formel (S-IX) enthält, 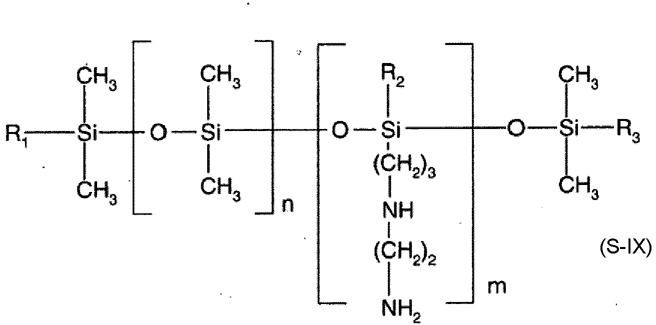 wobei
- m und n bedeuten Zahlen, die so gewählt sind, dass die Summe (n + m) im Bereich von 1 bis 1000 liegt,
- n ist eine Zahl im Bereich von 0 bis 999 und m ist eine Zahl im Bereich von 1 bis 1000,
- R1, R2 und R3, die gleich oder verschieden sind, bedeuten eine Hydroxygruppe oder eine C1-4-Alkoxygruppe,
- wobei mindestens eine der Gruppen R1 bis R3 eine Hydroxygruppe bedeutet;

Weitere bevorzugte Verfahren sind gekennzeichnet durch die Anwendung eines Mittels (a) auf dem keratinischem Material, wobei das Mittel (a) mindestens aminofunktionelles Silikonpolymer der Formel der Formel (S-X) 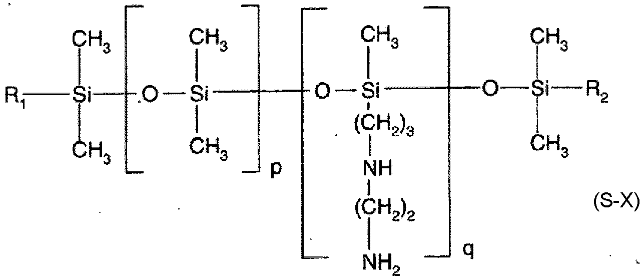 enthält, in der
- p und q bedeuten Zahlen, die so gewählt sind, dass die Summe (p + q) im Bereich von 1 bis 1000 liegt,
- p ist eine Zahl im Bereich von 0 bis 999 und q ist eine Zahl im Bereich von 1 bis 1000,
- R1 und R2, die verschieden sind, bedeuten eine Hydroxygruppe oder eine C1-4-Alkoxygruppe, wobei mindestens eine der Gruppen R1 bis R2 eine Hydroxygruppe bedeutet.

Die Silikone der Formeln (S-IX) und (S-X) unterscheiden sich durch die Gruppierung am Si-Atom, das die stickstoffhaltige Gruppe trägt: In Formel (S-IX) bedeutet R2 eine Hydroxygruppe oder eine C1-4-Alkoxygruppe, während der Rest in Formel (S-X) eine Methylgruppe ist. Die einzelnen Si-Gruppierungen, die mit den Indices m und n bzw. p und q gekennzeichnet sind, müssen nicht als Blöcke vorliegen, vielmehr können die einzelnen Einheiten auch statistisch verteilt vorliegen, d.h. in den Formeln (S-IX) und (S-X) ist nicht zwingend jedes R1-Si(CH₃)₂-Gruppe an eine -[O-Si(CH₃)₂]-Gruppierung gebunden.

Als besonders wirkungsvoll im Hinblick auf die gewünschten Effekte haben sich auch Verfahren erwiesen, in welchen ein Mittel (a) auf den Keratinfasern appliziert wird, welches mindestens ein amino-modifiziertes Silikonpolymer (a3) der Formel der Formel (S-XI) enthält 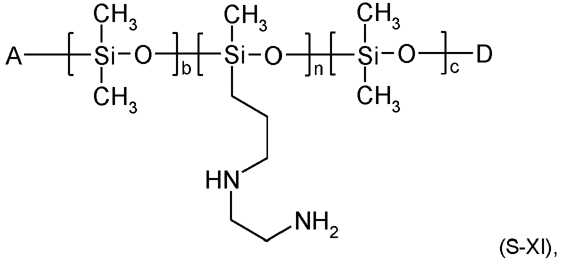 in der
- A: für eine Gruppe -OH, -O-Si(CH₃)₃,-O-Si(CH₃)₂OH ,-O-Si(CH₃)₂OCH₃ steht,
- D: für eine Gruppe -H, -Si(CH₃)₃,-Si(CH₃)₂OH, -Si(CH₃)₂OCH₃ steht,
- b, n und c: für ganze Zahlen zwischen 0 und 1000 stehen, mit den Maßgaben
- n > 0 und b + c > 0
- mindestens eine der Bedingungen A = -OH bzw. D = -H ist erfüllt.

In der vorstehend genannten Formel (S-XI) sind die einzelnen Siloxaneinheiten mit den Indices b, c und n statistisch verteilt, d.h. es muss sich nicht zwingend um Blockcopolymere handeln.

Sehr gute Effekte im Hinblick auf die Verbesserung der Reibechtheit konnten dann beobachtet werden, wenn in den Verfahren ein Mittel (a) auf dem keratinischen Material appliziert wurde, welches ein spezielles 4-Morpholinomethyl-substituiertes Silikonpolymer (a3) enthielt. Dieses ganz besonders bevorzugte aminofunktionalisierte Silikonpolymer umfasst mindestens eine Struktureinheit der Formel (S-XIII)

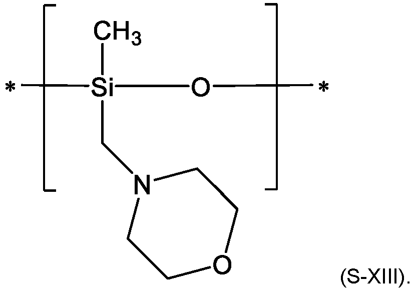

Im Rahmen einer Ausführungsform bevorzugt ist damit ein Verfahren zum Färben von keratinischem Material, welches dadurch gekennzeichnet ist, dass das Mittel (a) enthält:
(a3) mindestens ein Silikonpolymer, das mindestens eine Struktureinheit der Formel (S-XIII) umfasst

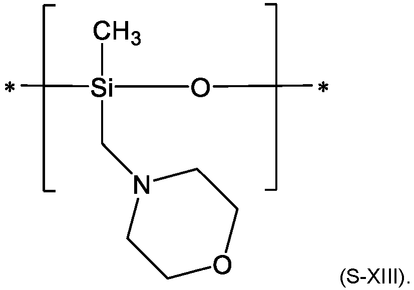

Besonders gute Effekte im Hinblick auf die Verbesserung der Reibechtheit konnten auch dann beobachtet werden, wenn in den Verfahren ein Mittel (a) auf dem keratinischen Material appliziert wurde, welches ein spezielles 4-Morpholinomethyl-substituiertes Silikonpolymer (a3) enthielt. Dieses ganz besonders bevorzugte aminofunktionalisierte Silikonpolymer umfasst Struktureinheiten der Formeln (S-XII) und der Formel (S-XIII)

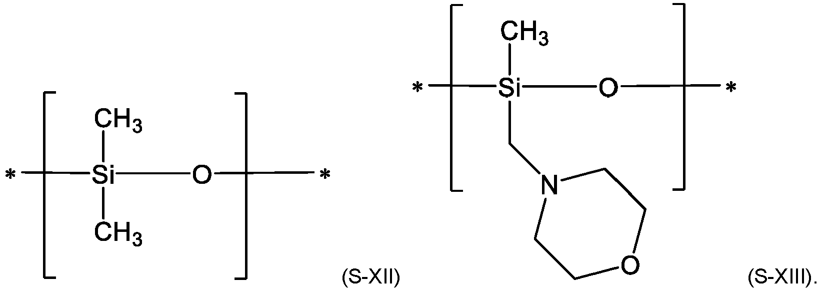

Im Rahmen einer explizit ganz besonders bevorzugten Ausführungsform ist ein Verfahren dadurch gekennzeichnet, dass das Mittel (a) mindestens ein amino-modifiziertes Silikonpolymer (a3) enthält, das Struktureinheiten der Formel (S-XII) und der Formel (S-XIII) umfasst

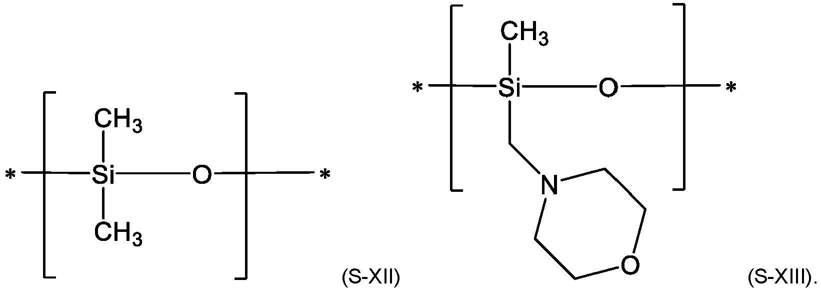

Entsprechende 4-Morpholinomethyl-substituiertes Silikonpolymere werden im Folgenden beschrieben.

Ein ganz besonders bevorzugtes, aminofunktionalisiertes Silikonpolymer ist unter dem Namen Amodimethicone/Morpholinomethyl Silsesquioxane Copolymer bekannt und in Form des Rohstoffes Belsil ADM 8301 E von Wacker kommerziell erhältlich.

Als 4-morpholinomethyl-substituiertes Silikon kann beispielsweise ein Silikon eingesetzt werden, welches Struktureinheiten der Formeln (S-XII), (S-XIII') und (S-XIV') aufweist 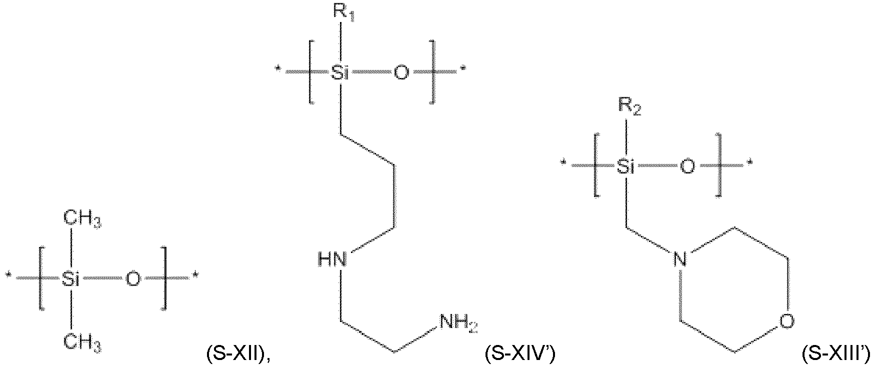 in denen
- R1: für -CH₃, -OH, -OCH₃, -O-CH₂CH₃, -O-CH₂CH₂CH₃, oder -O-CH(CH₃)₂ steht;
- R2: für -CH₃, -OH, oder -OCH₃ steht.

Besonders bevorzugte Mittel (a) enthalten mindestens ein 4-morpholinomethyl-substituierten Silikons der Formel (S-XV) 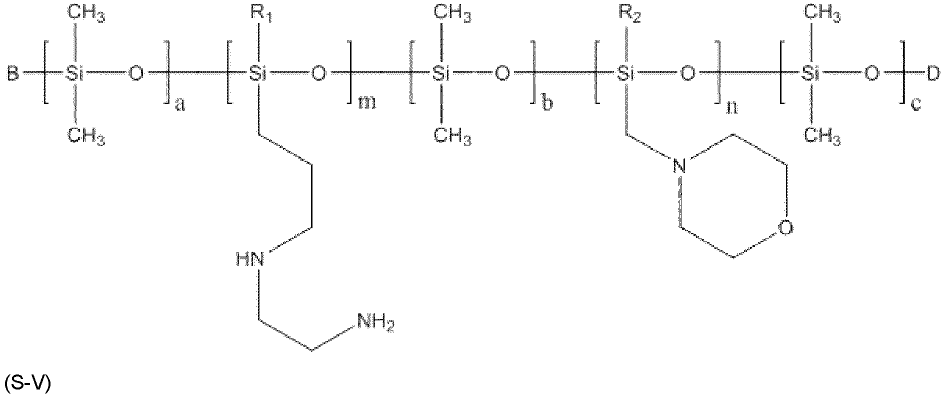 in der
R1 für -CH₃, -OH, -OCH₃, -O-CH₂CH₃, -O-CH₂CH₂CH₃, oder -O-CH(CH₃)₂ steht;
R2 für -CH₃, -OH, oder -OCH₃ steht.
B für eine Gruppe -OH, -O-Si(CH₃)₃,-O-Si(CH₃)₂OH ,-O-Si(CH₃)₂OCH₃ steht,
D für eine Gruppe -H, -Si(CH₃)₃,-Si(CH₃)₂OH, -Si(CH₃)₂OCH₃ steht,
a, b und c unabhängig voneinander für ganze Zahlen zwischen 0 und 1000 stehen, mit der Maßgabe a + b + c > 0
m und n unabhängig voneinander für ganze, Zahlen zwischen 1 und 1000 stehen
mit den Maßgabe, dass
   - mindestens eine der Bedingungen B = -OH bzw. D = -H erfüllt ist,
   - die Einheiten a, b, c, m und n statistisch oder blockweise im Molekül verteilt vorliegen.

Strukturformel (Si-VI) soll verdeutlichen, dass die Siloxangruppen n und m nicht zwingend direkt an eine Endgruppierung B bzw. D gebunden sein müssen. Vielmehr gilt in bevorzugten Formeln (Si-VI) a > 0 oder b > 0 und in besonders bevorzugten Formeln (Si-VI) a > 0 und c > 0, d.h. die terminale Gruppierung B bzw. D ist vorzugsweise an eine Dimethylsiloxy-Gruppierung gebunden. Auch in Formel (Si-VI) sind die Siloxaneinheiten a, b, c, m und n vorzugsweise statistisch verteilt.

Die durch Formel (Si-VI) dargestellten erfindungsgemäß eingesetzten Silikone können trimethylsilylterminiert sein (D oder B = -Si(CH₃)₃), sie können aber auch zweiseitig dimethylsilylhydroxy- oder einseitig dimethylsilylhydroxy- und dimethylsilylmethoxy-terminiert sein. Im Rahmen der vorliegenden Erfindung besonders bevorzugt eingesetzte Silikone sind ausgewählt aus Silikonen, in denen

| | |
|---|---|
| B = -O-Si(CH₃)₂OH und | D = -Si(CH₃)₃ |
| B = -O-Si(CH₃)₂OH und | D = -Si(CH₃)₂OH |
| B = -O-Si(CH₃)₂OH und | D = -Si(CH₃)₂OCH₃ |
| B = -O-Si(CH₃)₃ | und D = -Si(CH₃)₂OH |
| B = -O-Si(CH₃)₂OCH₃ | und D = -Si(CH₃)₂OH |

bedeutet.

Zur Erzeugung besonders widerstandsfähiger Filme enthält das Mittel (a) das oder die Silikonpolymere, insbesondere die alkoxy-modifizierten und/oder die amino-modifizierten Silikonpolymere, bevorzugt in bestimmten Mengenbereichen.

Besonders flexible Filme geringer Klebrigkeit wurden erhalten, wenn in dem Verfahren ein Mittel (a) eingesetzt wurde, welches - bezogen auf das Gesamtgewicht des Mittels (a) - ein oder mehrere Silikonpolymere (a3) in einer Gesamtmenge von 0,1 bis 8 Gew.-%, bevorzugt 0,1 bis 5 Gew.-%, weiter bevorzugt von 0,1 bis 3 Gew.-% und ganz besonders bevorzugt von 0,1 bis 0,5 Gew.-% enthält.

Im Rahmen einer weiteren bevorzugten Ausführungsform ist ein Verfahren dadurch gekennzeichnet, dass das Mittel (a) - bezogen auf das Gesamtgewicht des Mittels (a) - ein oder mehrere Silikonpolymere (a3) in einer Gesamtmenge von 0,1 bis 15 Gew.-%, bevorzugt 0,5 bis 12 Gew.-%, weiter bevorzugt von 1 bis 10 Gew.-% und ganz besonders bevorzugt von 2 bis 8 Gew.-% enthält.

Im Rahmen einer explizit ganz besonders bevorzugten Ausführungsform ist ein Verfahren dadurch gekennzeichnet, dass das Mittel (a) - bezogen auf das Gesamtgewicht des Mittels (a) - ein oder mehrere alkoxy-modifizierte Silikonpolymere (a3) in einer Gesamtmenge von 0,1 bis 15 Gew.-%, bevorzugt 0,5 bis 12 Gew.-%, weiter bevorzugt von 1 bis 10 Gew.-% und ganz besonders bevorzugt von 2 bis 8 Gew.-% enthält.

Im Rahmen einer explizit ganz besonders bevorzugten Ausführungsform ist ein Verfahren dadurch gekennzeichnet, dass das Mittel (a) - bezogen auf das Gesamtgewicht des Mittels (a) - ein oder mehrere amino-modifizierte Silikonpolymere in einer Gesamtmenge von 0,1 bis 15 Gew.-%, bevorzugt 0,5 bis 12 Gew.-%, weiter bevorzugt von 1 bis 10 Gew.-% und ganz besonders bevorzugt von 2 bis 8 Gew.-% enthält.

### pH-Wert des Mittels (a)

Es hat sich als bevorzugt herausgestellt, wenn das Mittel (a) in Form eines wasserhaltigen Mittels konfektioniert wird, das auf einen alkalischen pH-Wert eingestellt wird.

Zur Einstellung des pH-Wertes kann das Mittel (a) mindestens ein Alkalisierungsmittel enthalten.

Zur Einstellung des gewünschten pH-Wertes können die Mittel (a) daher auch mindestens ein Alkalisierungsmittel enthalten. Bei den pH-Werten im Sinne der vorliegenden Erfindung handelt es sich um pH-Werte, die bei einer Temperatur von 22°C gemessen wurden.

Als Alkalisierungsmittel kann das Mittel (a) beispielsweise Ammoniak, Alkanolamine und/oder basische Aminosäuren enthalten.

Die in dem Mittel einsetzbaren Alkanolamine werden bevorzugt ausgewählt aus primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

Besonders bevorzugte Alkanolamine werden ausgewählt aus 2-Aminoethan-1-ol und/oder 2-Amino-2-methylpropan-1-ol. Eine besonders bevorzugte Ausführungsform ist daher dadurch gekennzeichnet, dass das Mittel als Alkalisierungsmittel ein Alkanolamin ausgewählt aus 2-Aminoethan-1-ol und/oder 2-Amino-2-methylpropan-1-ol enthält.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α-Aminocarbonsäuren besonders bevorzugt sind.

Unter basischen Aminosäuren sind solche Aminosäuren zu verstehen, welche einen isoelektrischen Punkt pl von größer 7 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist ein Mittel daher dadurch gekennzeichnet, dass es sich bei dem Alkalisierungsmittel um eine basische Aminosäure aus der Gruppe Arginin, Lysin, Ornithin und/oder Histidin handelt.

Darüber hinaus kann das Mittel weitere Alkalisierungsmittel, insbesondere anorganische Alkalisierungsmittel enthalten. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Ganz besonders bevorzugte Alkalisierungsmittel sind Ammoniak, 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid, Kaliumhydroxid, Calciumhydroxid, Bariumhydroxid, Natriumphosphat, Kaliumphosphat, Natriumsilicat, Natriummetasilicat, Kaliumsilicat, Natriumcarbonat und Kaliumcarbonat.

Obwohl die Mittel (a) bevorzugt auf pH-Werte im alkalischen Bereich eingestellt werden, kann es dennoch prinzipiell notwendig sein, zur Feinjustierung des gewünschten pH-Wertes in geringen Mengen auch Acidifizierungsmittel einzusetzen. Erfindungsgemäß geeignete Acidifizierungsmittel sind beispielsweise Zitronensäure, Milchsäure, Essigsäure oder auch verdünnte Mineralsäuren (wie beispielsweise Salzsäure, Schwefelsäure, Phosphorsäure).

Im Rahmen der zu dieser Erfindung führenden Arbeiten hat es sich jedoch herausgestellt, dass die Gegenwart des Alkalisierungsmittels bzw. die Einstellung des alkalischen pH-Wertes für die Ausbildung von resistenten Filmen auf dem keratinischen Material essentiell ist. Die Gegenwart zu großer Mengen an Säuren kann sich hierbei negativ auf die Festigkeit der Filme auswirken. Aus diesem Grund hat es sich als bevorzugt herausgestellt, die Einsatzmengen an Säuren im Mittel (a) möglichst gering zu halten. Aus diesem Grund ist es von Vorteil, wenn die Gesamtmenge der im Mittel (a) enthaltenen organischen und/oder anorganischen Säuren einen bestimmten Wert nicht überschreitet.

In einer weiteren bevorzugten Ausführungsform ist ein Verfahren dadurch gekennzeichnet, dass die Gesamtmenge der im Mittel (a) enthaltenen organischen Säuren aus der Gruppe aus Citronensäure, Weinsäure, Äpfelsäure und Milchsäure bei einem Gehalt unterhalb von 1 Gew.-%, bevorzugt unterhalb von 0,7 Gew.-%, weiter bevorzugt unterhalb von 0,5 Gew.-%, noch weiter bevorzugt unterhalb von 0,1 Gew.-% und ganz besonders bevorzugt unterhalb von 0,01 Gew.-% liegt.

In einer weiteren bevorzugten Ausführungsform ist ein Verfahren dadurch gekennzeichnet, dass die Gesamtmenge der im Mittel (a) enthaltenen anorganischen Säuren aus der Gruppe aus Salzsäure, Schwefelsäure und Phosphorsäure bei einem Gehalt unterhalb von 1 Gew.-%, bevorzugt unterhalb von 0,7 Gew.-%, weiter bevorzugt unterhalb von 0,5 Gew.-%, noch weiter bevorzugt unterhalb von 0,1 Gew.-% und ganz besonders bevorzugt unterhalb von 0,01 Gew.-% liegt.

Die zuvor angegebenen maximalen Gesamtmengen der im Mittel (a) enthaltenen Säuren ist hierbei immer auf das Gesamtgewicht des Mittels (a) bezogen.

### Mittel (b)

Das Verfahren zur Behandlung von keratinischen Material umfasst neben der Anwendung des Mittels (a) auch die Anwendung des Mittels (b). Das in dem Verfahren eingesetzte Mittel (b) ist dadurch gekennzeichnet, dass es mindestens ein Versieglungsreagenz (b1), umfassend eine Verbindung ausgewählt aus der Gruppe bestehend aus filmbildenden Polymeren, Alkalisierungsmitteln, Acidifizierungsmittel und Mischungen daraus, enthält.

Das Mittel (b) ist ein Nachbehandlungsmittel und die Anwendung des Mittels (b) auf das mit Mittel (a) behandelte keratinische Material führt dazu, dass die in dem Verfahren erzielten Färbungen haltbarer gemacht werden. Insbesondere kann durch Anwendung des Mittels (b) die Waschechtheit und die Reibechtheit der im Verfahren erhaltenen Färbungen verbessert werden.

Es kann bevorzugt sein, dass das Versiegelungsreagenz (b1) ein filmbildendes Polymer umfasst.

Unter Polymeren werden Makromoleküle mit einem Molekulargewicht von mindestens 1000 g/mol, bevorzugt von mindestens 2500 g/mol, besonders bevorzugt von mindestens 5000 g/mol, verstanden, welche aus gleichen, sich wiederholenden organischen Einheiten bestehen. Bei den Polymeren der vorliegenden Erfindung kann es sich um synthetisch hergestellte Polymere handeln, die durch Polymerisation eines Monomertyps oder durch Polymerisation verschiedener, strukturell voneinander unterschiedlicher Monomertypen hergestellt werden. Wird das Polymer durch Polymerisation eines Monomertyps hergestellt, spricht man von Homo-Polymeren. Werden strukturell unterschiedliche Monomertypen in der Polymerisation eingesetzt, wird das resultierende Polymer als Copolymer bezeichnet.

Das maximale Molekulargewicht des Polymers hängt von dem Polymerisationsgrad (Anzahl der polymerisierten Monomere) und der Ansatzgröße ab und wird durch die Polymerisationsmethode mitbestimmt. Im Sinne der vorliegenden Erfindung ist es bevorzugt, wenn das maximale Molekulargewicht des filmbildenden, hydrophoben Polymers nicht mehr als 10⁷ g/mol, bevorzugt nicht mehr als 10⁶ g/mol und besonders bevorzugt nicht mehr als 10⁵ g/mol beträgt.

Unter einem filmbildenden Polymer wird im Sinne der Erfindung ein Polymer verstanden, welches in der Lage ist, auf einem Substrat, beispielsweise auf einem keratinischen Material oder einer keratinischen Faser, einen Film auszubilden. Die Ausbildung eines Films kann beispielsweise durch Betrachtung des mit dem Polymer behandelten keratinischen Materials unter einem Mikroskop nachgewiesen werden.

Die filmbildenden Polymere im Mittel (b) können hydrophil oder hydrophob sein.

Im Rahmen einer ersten Ausführungsform kann es bevorzugt sein, im Mittel (b) mindestens ein hydrophobes, filmbildendes Polymer als Versiegelungsreagenz (b1) einzusetzen.

Unter einem hydrophoben Polymer wird ein Polymer verstanden, dass eine Löslichkeit in Wasser bei 25 °C (760 mmHg) von weniger als 1 Gew.-% besitzt.

Die Wasserlöslichkeit des filmbildenden, hydrophoben Polymers kann beispielsweise auf dem folgenden Weg bestimmt werden. 1 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Sofern sich die Polymer-Wasser-Mischung aufgrund einer hohen Trübung des Gemisches nicht visuell beurteilten lässt, wird die Mischung filtriert. Bleibt auf dem Filterpapier ein Anteil an ungelöstem Polymer zurück, dann liegt die Löslichkeit des Polymers bei weniger als 1 Gew.-%.

Hier können insbesondere die Polymere des Acrylsäure-Typs, die Polyurethane, die Polyester, die Polyamide, die Polyharnstoffe, die Cellulose-Polymere, die Nitro-Cellulose-Polymere, die SilikonPolymere, die Polymere des Acrylamid-Typs und die Polyisoprene genannt werden.

Besonders gut geeignete filmbildende, hydrophobe Polymere sind beispielsweise Polymere aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

In einer weiteren bevorzugten Ausführungsform ist ein Verfahren dadurch gekennzeichnet, dass das Mittel (b) mindestens ein filmbildendes, hydrophobes Polymer als Versiegelungsreagenz (b1) enthält, das ausgewählt ist aus der Gruppe der Copolymere von Acrylsäure, der Copolymere der Methacrylsäure, der Homopolymere oder Copolymere von Acrylsäure-Estern, der Homopolymere oder Copolymere von Methacrylsäure-Estern, der Homopolymere oder Copolymere von Acrylsäureamiden, der Homopolymere oder Copolymere von Methacrylsäure-Amiden, der Copolymere des Vinylpyrrolidons, der Copolymere des Vinylalkohols, der Copolymere des Vinylacetats, der Homopolymere oder Copolymere des Ethylens, der Homopolymere oder Copolymere des Propylens, der Homopolymere oder Copolymere des Styrens, der Polyurethane, der Polyester und/oder der Polyamide.

Zur Lösung der erfindungsgemäßen Aufgabenstellung haben sich insbesondere die filmbildenden hydrophoben Polymere als gut geeignet erwiesen, die aus der Gruppe der synthetischen Polymere, der durch radikalische Polymerisation erhältlichen Polymere oder der natürlichen Polymere ausgewählt werden.

Weitere besonders gut geeignete filmbildende hydrophobe Polymere können ausgewählt werden aus den Homopolymere oder Copolymeren von Olefinen, wie beispielsweise Cycloolefinen, Butadien, Isopren oder Styren, Vinylethern, Vinylamiden, den Estern oder Amiden von (Meth)Acrylsäure mit mindestens einer C₁-C₂₀-Alkylgruppe, einer Arylgruppe oder einer C₂-C₁₀-Hydroxyalkylgruppe.

Weitere filmbildende hydrophobe Polymere können ausgewählt sein aus den Homo- oder Copolymeren von Isooctyl(meth)acrylat, Isononyl(meth)acrylat, 2-Ethylhexyl(meth)acrylat , Lauryl(meth)acrylat), Isopentyl(meth)acrylat, n-Butyl(meth)acrylat), Isobutyl(meth)acrylat , Ethyl(meth)acrylat, Methyl(meth)acrylat, tert-Butyl(meth)acrylat, Stearyl(meth)acrylat, Hydroxyethyl-(meth)acrylat, 2-Hydroxypropyl(meth)acrylat, 3-Hydroxypropyl(meth)acrylat und/oder Gemischen hiervon.

Weitere filmbildende hydrophobe Polymere können ausgewählt sein aus den Homo- oder Copolymeren von (Meth)acrylamid, N-Alkyl(meth)acrylamiden, insbesondere solchen mit C2-C18 Alkylgruppen, wie beispielsweise N-Ethylacrylamid, N-tert-Butylacrylamid, le N-Octylacrylamid, N-Di(C1-C4)alkyl(meth)acrylamid.

Weitere bevorzugte anionische Copolymere sind beispielsweise Copolymere aus Acrylsäure, Methacrylsäure oder deren C₁-C₆-Alkylestern, wie sie unter der INCI-Deklaration Acrylates Copolymere vertrieben werden. Ein geeignetes Handelsprodukt ist beispielsweise Aculyn^{®} 33 der Firma Rohm & Haas. Weiterhin bevorzugt sind aber auch Copolymere aus Acrylsäure, Methacrylsäure oder deren C₁-C₆-Alkylestern und den Estern einer ethylenisch ungesättigten Säure und einem alkoxylierten Fettalkohol. Geeignete ethylenisch ungesättigte Säuren sind insbesondere Acrylsäure, Methacrylsäure und Itaconsäure; geeignete alkoxylierte Fettalkohole sind insbesondere Steareth-20 oder Ceteth-20.

Auf dem Markt befindliche ganz besonders bevorzugte Polymere sind beispielsweise Aculyn^{®} 22 (Acrylates/Steareth-20 Methacrylate Copolymer), Aculyn^{®} 28 (Acrylates/Beheneth-25 Methacrylate Copolymer), Structure 2001^{®} (Acryla-tes/Steareth-20 Itaconate Copolymer), Structure 3001^{®} (Acrylates/Ceteth-20 Itaconate Copolymer), Structure Plus^{®} (Acrylates/Aminoacrylates C10-30 Alkyl PEG-20 Itaconate Copolymer), Carbopol^{®} 1342, 1382, Ultrez 20, Ultrez 21 (Acrylates/C10-30 Alkyl Acrylate Crosspolymer), Synthalen W 2000^{®} (Acrylates/Palmeth-25 Acrylate Copolymer) oder das Rohme und Haas vertriebene Soltex OPT (Acrylates/C12-22 Alkyl methacrylate Copolymer).

Als geeignete Polymere auf der Basis von Vinyl-Monomeren können beispielsweise genannt werden die Homo- und Copolymere des N-Vinylpyrrolidons, des Vinylcaprolactams, des Vinyl-(C1-C6-)alkyl-Pyrrols, des Vinyloxazols, des Vinylthiazols, des Vinylpyrimidins oder des Vinylimidazols.

Weiterhin ganz besonders gut geeignet sind die Copolymere Octylacrylamid/acrylates/ butylaminoethyl-methacrylate copolymer, wie es beispielsweise unter den Handelsnamen AMPHOMER^{®} oder LOVOCRYL^{®} 47 von NATIONAL STARCH kommerziell vertrieben wird, oder auch die Copolymere von Acrylates/Octylacrylamide die unter den Handelsnamen DERMACRYL^{®} LT und DERMACRYL^{®} 79 von NATIONAL STARCH vertrieben werden.

Als geeignete Polymere auf der Basis von Olefinen können beispielsweise genannt werden die Homo- und Copolymere des Ethylens, des Propylens, des Butens, des Isoprens und des Butadiens.

Im Rahmen einer weiteren Ausführungsform können als filmbildenden hydrophoben Polymere die Block-Copolymere eingesetzt werden, die mindestens einen Block aus Styren oder den Derivaten des Styrens umfassen. Bei diesen Block-Copolymeren kann es sich um Copolymere handeln, die neben einem Styren-Block einen oder mehrere weitere Blöcke enthalten, wie beispielsweise Styren/Ethylen, Styren/Ethylen/Butylen, Styren/Butylen, Styren/Isopren, Styren/Butadien. Entsprechende Polymere werden von der BASF unter dem Handelsnamen "Luvitol HSB" kommerziell vertrieben.

Überraschenderweise hat sich herausgestellt, dass dann ganz besonders intensive und waschechte Färbungen erhalten werden konnten, wenn das Mittel (b) mindestens ein filmbildendes Polymer als Versiegelungsreagenz (b1) enthielt, das ausgewählt wurde aus der Gruppe der der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

In einer weiteren bevorzugten Ausführungsform ist ein Verfahren dadurch gekennzeichnet, dass das Mittel (b) mindestens ein filmbildendes Polymer als Versiegelungsreagenz (b1) enthält, das ausgewählt ist aus der Gruppe der Homopolymere und Copolymere von Acrylsäure, der Homopolymere und Copolymere von Methacrylsäure, der Homopolymere und Copolymere von Acrylsäure-Estern, der Homopolymere und Copolymere von Methacrylsäure-Estern, der Homopolymere und Copolymere von Acrylsäureamiden, der Homopolymere und Copolymere von Methacrylsäure-Amiden, der Homopolymere und Copolymere des Vinylpyrrolidons, der Homopolymere und Copolymere des Vinylalkohols, der Homopolymere und Copolymere des Vinylacetats, der Homopolymere und Copolymere des Ethylens, der Homopolymere und Copolymere des Propylens, der Homopolymere und Copolymere des Styrens, der Polyurethane, der Polyester und der Polyamide.

Im Rahmen einer weiteren Ausführungsform kann es bevorzugt sein, im Mittel (b) mindestens ein hydrophiles, filmbildendes Polymer als Versiegelungsreagenz (b1) einzusetzen.

Unter einem hydrophilen Polymer wird ein Polymer verstanden, dass eine Löslichkeit in Wasser bei 25 °C (760 mmHg) von mehr als 1 Gew.-%, bevorzugt von mehr als 2 Gew.-%, besitzt.

Die Wasserlöslichkeit des filmbildenden, hydrophilen Polymers kann beispielsweise auf dem folgenden Weg bestimmt werden. 1 g des Polymers werden in ein Becherglas gegeben. Mit Wasser wird auf 100 g aufgefüllt. Es wird ein Rührfisch hinzugegeben, und die Mischung wird auf einem Magnetrührer unter Rühren auf 25 °C erwärmt. Es wird für 60 Minuten gerührt. Danach wird die wässrige Mischung visuell beurteilt. Ein vollständig gelöstes Polymer erscheint makroskopisch homogen. Sofern sich die Polymer-Wasser-Mischung aufgrund einer hohen Trübung des Gemisches nicht visuell beurteilten lässt, wird die Mischung filtriert. Bleibt auf dem Filterpapier kein ungelöstes Polymer zurück, dann liegt die Löslichkeit des Polymers bei mehr als 1 Gew.-%.

Als filmbildende, hydrophile Polymere können nichtionische, anionische und kationische Polymere eingesetzt werden.

Geeignete filmbildende, hydrophile Polymere können beispielsweise aus der Gruppe der Polyvinylpyrrolidon-(Co)Polymere, der Polyvinylalkohol-(Co)Polymere, der Vinylacetat-(Co)Polymere, der Carboxyvinyl-(Co)Polymere, der Acrylsäure-(Co)Polymere, der Methacrylsäure-(Co)Polymere, der natürlichen Gummis, der Polysaccharide und/oder der Acrylamid-(Co)Polymere ausgewählt werden.

Weiterhin ist es ganz besonders bevorzugt, als filmbildendes hydrophiles Polymer Polyvinylpyrrolidon (PVP) und/oder ein Vinylpyrrolidon-haltiges Copolymer einzusetzen.

In einer weiteren ganz besonders bevorzugten Ausführungsform ist ein Verfahren dadurch gekennzeichnet, dass das Mittel (b) mindestens ein filmbildendes, hydrophiles Polymer als Versiegelungsreagenz (b1) enthält, das ausgewählt ist aus der Gruppe aus Polyvinylpyrrolidon (PVP) und den Copolymeren des Polyvinylpyrrolidons.

Es ist weiterhin bevorzugt, wenn das Mittel als filmbildendes, hydrophiles Polymer Polyvinylpyrrolidon (PVP) enthält. Überraschenderweise war auch die Waschechtheit der Färbungen, die mit PVPhaltigen Mitteln (b) erhalten werden konnten, sehr gut.

Besonders gut geeignete Polyvinylpyrrolidone sind beispielsweise unter der Bezeichnung Luviskol^{®} K von der BASF SE erhältlich, insbesondere Luviskol^{®} K 90 oder Luviskol^{®} K 85 der Firma BASF SE.

Als weiteres explizit ganz besonders gut geeignetes Polyvinylpyrrolidon (PVP) kann auch das Polymer PVP K30 eingesetzt werden, das von der Firma Ashland (ISP, POI Chemical) vertrieben wird. PVP K 30 ist ein in kaltem Wasser sehr gut lösliches Polyvinylpyrrolidon, welches die CAS-Nummer 9003-39-8 besitzt. Das Molgewicht von PVP K 30 liegt bei ca. 40000 g/mol.

Weitere ganz besonders gut geeignete Polyvinylpyrrolidone sind die unter den Handelsnamen LUVITEC K 17, LUVITEC K 30, LUVITEC K 60, LUVITEC K 80, LUVITEC K 85, LUVITEC K 90 und LUVITEC K 115 bekannten und von der BASF erhältlichen Substanzen.

Der Einsatz von filmbildenden hydrophilen Polymeren als Versiegelungsreagenz (b1) aus der Gruppe der Copolymere des Polyvinylpyrrolidons hat ebenfalls zu besonders guten und waschechten Farbergebnissen geführt.

Als besonders gut geeignete filmbildende, hydrophile Polymere können in diesem Zusammenhang Vinylpyrrolidon-Vinylester-Copolymere genannt werden, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind besonders bevorzugte nichtionische Polymere.

Von den Vinylpyrrolidon-haltigen Copolymeren werden ganz besonders bevorzugt ein Styrene/VP Copolymer und/oder ein Vinylpyrrolidon-Vinylacetat-Copolymer und/oder ein VP/DMAPA Acrylates Copolymer und/oder ein VP/Vinyl Caprolactam/DMAPA Acrylates Copolymer in den kosmetischen Zusammensetzungen eingesetzt.

Vinylpyrrolidon-Vinylacetat-Copolymere werden unter der Bezeichnung Luviskol^{®} VA von der BASF SE vertrieben. Ein VP/Vinyl Caprolactam/DMAPA Acrylates Copolymer wird beispielsweise unter dem Handelsnamen Aquaflex^{®} SF-40 von Ashland Inc. vertrieben. Ein VP/DMAPA Acrylates Copolymer wird beispielsweise unter der Bezeichnung Styleze CC-10 von Ashland vertrieben und ist ein höchst bevorzugtes Vinylpyrrolidon-haltiges Copolymer.

Als weitere geeignete Copolymere des Polyvinylpyrrolidons können auch die Copolymere genannt werden, die durch Umsetzung von N-Vinylpyrrolidon mit mindestens einem weiteren Monomer aus der Gruppe aus V-Vinylformamid, Vinylacetat, Ethylen, Propylen, Acrylamid, Vinylcaprolactam, Vinylcaprolacton und/oder Vinylalkohol erhalten werden.

In einer weiteren ganz besonders bevorzugten Ausführungsform ist ein Verfahren dadurch gekennzeichnet, dass das Mittel (b) mindestens ein filmbildendes, hydrophiles Polymer als Versiegelungsreagenz (b1) enthält, das ausgewählt ist aus der Gruppe aus Polyvinylpyrrolidon (PVP), Vinylpyrrolidon/Vinylacetat-Copolymeren, Vinylpyrrolidon/Styren-Copolymeren, Vinylpyrrolidon/Ethylen-Copolymeren, Vinylpyrrolidon/Propylen-Copolymeren, Vinylpyrrolidon/Vinylcaprolactam-Copolymeren, Vinylpyrrolidon/Vinylformamid-Copolymeren und/oder Vinylpyrrolidon/Vinylalkohol-Copolymeren.

Ein weiteres geeigetes Copolymer des Vinylpyrrolidons ist das unter der INCI Bezeichnung Maltodextrin/VP Copolymer bekannte Polymer.

Weiterhin konnte intensiv gefärbtes keratinischen Material, insbesondere Haare, mit sehr guten Waschechtheiten erhalten werden, wenn als filmbildendes, hydrophiles Polymer ein nichtionisches, filmbildendes, hydrophiles Polymer eingesetzt wurde.

Im Rahmen einer weiteren Ausführungsform kann das Mittel (b) mindestens ein nichtionisches, filmbildendes, hydrophiles Polymer als Versiegelungsreagenz (b1) enthalten.

Unter einem nichtionischen Polymer wird erfindungsgemäß ein Polymer verstanden, das in einem protischen Lösemittel - wie beispielsweise Wasser - bei Standardbedingungen keine Struktureinheiten mit permanent kationischen oder anionischen Gruppen trägt, welche durch Gegenionen unter Erhaltung der Elektroneutralität kompensiert werden müssen. Unter kationische Gruppen fallen beispielsweise quaternisierte Ammoniumgruppen jedoch keine protonierten Amine. Unter anionische Gruppen fallen beispielsweise Carboxyl- und Sulfonsäuregruppen.

Es sind die Mittel ganz besonders bevorzugt, die als nichtionisches, filmbildendes, hydrophiles Polymer mindestens ein Polymer enthalten, das ausgewählt ist aus der Gruppe aus
- Polyvinylpyrrolidon,
- Copolymeren aus N-Vinylpyrrolidon und Vinylestern von Carbonsäuren mit 2 bis 18 Kohlenstoffatomen, insbesondere aus N-Vinylpyrrolidon und Vinylacetat,
- Copolymeren aus N-Vinylpyrrolidon und N-Vinylimidazol und Methacrylamid,
- Copolymeren aus N-Vinylpyrrolidon und N-Vinylimidazol und Acrylamid,
- Copolymeren aus N-Vinylpyrrolidon mit N,N-Di(C1 bis C4)-Alkylamino-(C2 bis C4)-alkylacrylamid.

Kommen Copolymere aus N-Vinylpyrrolidon und Vinylacetat zum Einsatz, ist es wiederum bevorzugt, wenn das Molverhältnis der aus dem Monomer N-Vinylpyrrolidon enthaltenen Struktureinheiten zu den aus dem Monomer Vinylacetat enthaltenen Struktureinheiten des Polymers im Bereich von 20 zu 80 bis 80 zu 20, insbesondere von 30 zu 70 bis 60 zu 40, liegt. Geeignete Copolymerisate aus Vinylpyrrolidon und Vinylacetat sind beispielsweise unter dem Warenzeichen Luviskol^{®} VA 37, Luviskol^{®} VA 55, Luviskol^{®} VA 64 und Luviskol^{®} VA 73 von der Firme BASF SE erhältlich.

Ein weiteres besonders bevorzugtes Polymer wird dabei ausgewählt aus den Polymeren der INCI-Bezeichnung VP/Methacrylamide/Vinyl Imidazole Copolymer, die beispielsweise unter dem Handelsnamen Luviset Clear von der Fa. BASF SE erhältlich sind.

Ein weiteres ganz besonders bevorzugtes nichtionisches, filmbildendes, hydrophiles Polymer st ein Copolymer aus N-Vinylpyrrolidon und N,N-Dimethylaminiopropylmethacrylamid,welches beispielsweise mit der INCI-Bezeichnung VP/DMAPA Acrylates Copolymer z. B. unter der Handelsbezeichnung Styleze^{®} CC 10 von der Firma ISP verkauft wird.

Ein kationisches Polymer ist das Copolymer aus N-Vinylpyrrolidon, N-Vinylcaprolactam, N-(3-Dimethylaminopropyl)methacrylamid und 3-(Methacryloylamino)propyl-lauryl-dimethylammoniumchlorid (INCI-Bezeichnung: Polyquaternium-69), welches beispielsweise unter dem Handelsnamen AquaStyle^{®} 300 (28-32 Gew.-% Aktivsubstanz in Ethanol-Wasser-Gemisch, Molekulargewicht 350000) von der Firma ISP vertrieben wird.

Weitere geeignete filmbildende, hydrophile Polymere sind beispielsweise
- Vinylpyrrolidon-Vinylimidazoliummethochlorid-Copolymere, wie sie unter den Bezeichnungen Luviquat^{®} FC 370, FC 550 und der INCI-Bezeichnung Polyquaternium-16 sowie FC 905 und HM 552 angeboten werden,
- Vinylpyrrolidon-Vinylcaprolactam-Acrylat-Terpolymere, wie sie mit Acrylsäureestern und Acrylsäureamiden als dritter Monomerbaustein im Handel beispielsweise unter der Bezeichnung Aquaflex^{®} SF 40 angeboten werden.

Polyquaternium-11 ist das Reaktionsprodukt von Diethylsulfat mit einem Copolymer von Vinylpyrrolidon und Dimethylaminoethylmethacrylat. Geeignete Handelsprodukte sind beispielsweise unter den Bezeichnungen Dehyquart^{®} CC 11 und Luviquat^{®} PQ 11 PN von der BASF SE oder Gafquat 440, Gafquat 734, Gafquat 755 oder Gafquat 755N von Ashland Inc. erhältlich.

Polyquaternium-46 ist das Reaktionsprodukt von Vinylcaprolactam und Vinylpyrrolidon mit Methylvinylimidazoliummethosulfat und ist beispielsweise unter der Bezeichnung Luviquat^{®} Hold von der BASF SE erhältlich. Polyquaternium-46 wird bevorzugt in einer Menge von 1 bis 5 Gew.-% - bezogen auf das Gesamtgewicht der kosmetischen Zusammensetzung - eingesetzt. Es ganz besonders bevorzugt, dass Polyquaternium-46 in Kombination mit einer kationischen Guar-Verbindung eingesetzt wird. Es ist sogar höchst bevorzugt, dass Polyquaternium-46 in Kombination mit einer kationischen Guar-Verbindung und Polyquaternium-11 eingesetzt wird.

Als geeignete anionische filmbildende, hydrophile Polymere können zum Beispiel Acrylsäure-Polymere eingesetzt werden, die in unvernetzter oder vernetzter Form vorliegen können. Entsprechende Produkte werden beispielsweise unter den Handelsnamen Carbopol 980, 981, 954, 2984 and 5984 von der Firma Lubrizol oder auch unter den Namen Synthalen M and Synthalen K von der Firma 3V Sigma (The Sun Chemicals, Inter Harz) kommerziell vertrieben.

Beispiele für geeignete filmbildende, hydrophile Polymere aus der Gruppe der natürlichen Gums sind Xanthan Gum, Gellan Gum, Carob Gum.

Beispiele für geeignete filmbildende, hydrophile Polymere aus der Gruppe der Polysaccharide sind Hydroxyethylcellulose, Hydroxypropylcellulose, Ethylcellulose und Carboxymethyl-Cellulose.

Geeignete filmbildende, hydrophile Polymere aus der Gruppe der Acrylamde sind beispielsweise Polymere, welche ausgehend von Monomeren der (Meth)Acrylamido-C1-C4-alkyl-sulfonsäure bzw. der Salze hiervon hergestellt werden. Entsprechende Polymere können ausgewählt sein aus den Polymeren von Polyacrylamidomethansulfonsäure, Polyacrylamidoethansulfonsäure, Polyacrylamidopropansulfonsäure, Poly2-acrylamido-2-methylpropansulfonsäure, Poly-2-Methylacrylamido-2-methylpropansulfonsäure und/oder Poly-2-methylacrylamido-n-butansulfonsäure.

Bevorzugte Polymere der Poly(meth)arylamido-C1-C4-alkyl-sulfonsäuren sind vernetzt und zu mindestens 90 % neutralisiert. Diese Polymere können vernetzt oder auch unvernetzt sein.

Vernetzte und ganz oder teilweise neutralisierte Polymere des Typs der Poly-2-acrylamido-2-methylpropansulfonsäuren sind unter den INCI-Namen "Ammonium Polyacrylamido-2-methyl-propanesulphonate" oder "Ammonium Polyacryldimethyltauramide" bekannt.

Ein weiteres bevorzugtes Polymer dieses Typs ist das der Firma Clariant unter dem Handelsnamen Hostacerin AMPS vertriebene vernetzte Poly-2-acrylamido-2methyl-propanesulphonsäure-Polymer, das teilweise mit Ammoniak neutralisiert ist.

In einer weiteren explizit ganz besonders bevorzugten Ausführungsform ist ein Verfahren dadurch gekennzeichnet, dass das Mittel (b) mindestens ein anionisches, filmbildendes, Polymer als Versiegelungsreagenz (b1) enthält.

In diesem Zusammenhang konnten die besten Ergebnisse erhalten werden, wenn das Mittel (b) mindestens ein filmbildendes Polymer als Versiegelungsreagenz (b1) enthält, das mindestens eine Struktureinheit der Formel (P-I) und mindestens eine Struktureinheit der Formel (P-II) umfasst 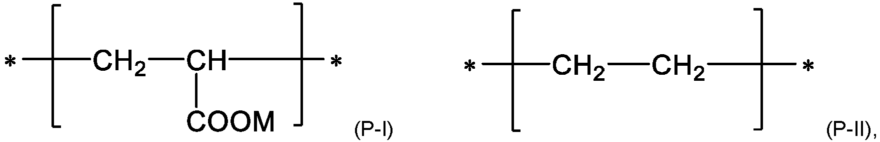 wobei
M für ein Wasserstoffatom oder für Ammonium (NH₄), Natrium, Kalium, ½ Magnesium oder ½ Calcium steht.

In einer weiteren bevorzugten Ausführungsform ist ein erfindungsgemäßes Verfahren dadurch gekennzeichnet, dass das Mittel (b) mindestens ein filmbildendes Polymer als Versiegelungsreagenz (b1) enthält, das mindestens eine Struktureinheit der Formel (P-I) und mindestens eine Struktureinheit der Formel (P-II) umfasst 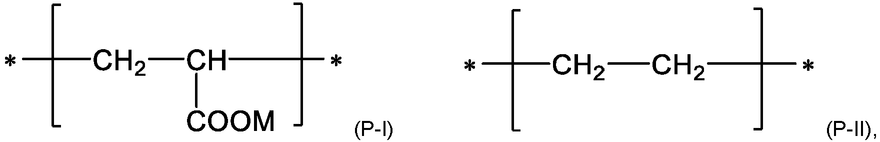 wobei
M für ein Wasserstoffatom oder für Ammonium (NH₄), Natrium, Kalium, ½ Magnesium oder ½ Calcium steht.

Wenn M für ein Wasserstoffatom steht, basiert die Struktureinheit der Formel (P-I) auf einer Acrylsäure-Einheit.

Wenn M für ein Ammonium-Gegenion steht, basiert die Struktureinheit der Formel (P-I) auf dem Ammoniumsalz der Acrylsäure.

Wenn M für ein Natrium-Gegenion steht, basiert die Struktureinheit der Formel (P-I) auf dem Natriumsalz der Acrylsäure.

Wenn M für ein Kalium-Gegenion steht, basiert die Struktureinheit der Formel (P-I) auf dem Kaliumsalz der Acrylsäure.

Wenn M für ein halbes Äquivalent eines Magnesium-Gegenions steht, basiert die Struktureinheit der Formel (P-I) auf dem Magnesiumsalz der Acrylsäure.

Wenn M für ein halbes Äquivalent eines Calcium-Gegenions steht, basiert die Struktureinheit der Formel (P-I) auf dem Calciumsalz der Acrylsäure.

Das oder die filmbildenden Polymere werden bevorzugt in bestimmten Mengenbereichen in dem Mittel (b) eingesetzt. In diesem Zusammenhang hat es sich zur Lösung der erfindungsgemäßen Aufgabenstellung als besonders bevorzugt erwiesen, wenn das Mittel (b) - bezogen auf das Gesamtgewicht des Mittels (b) - ein oder mehrere filmbildende Polymere als Versiegelungsreagenz (b1) in einer Gesamtmenge von 0,1 bis 18 Gew.-%, bevorzugt von 1 bis 16 Gew.-%, weiter bevorzugt von 5 bis 14,5 Gew.-% und ganz besonders bevorzugt von 8 bis 12 Gew.-% enthält.

In einer weiteren bevorzugten Ausführungsform ist ein Verfahren dadurch gekennzeichnet, dass das Mittel (b) - bezogen auf das Gesamtgewicht des Mittels (b) - ein oder mehrere filmbildende Polymere als Versiegelungsreagenz (b1) in einer Gesamtmenge von 0,1 bis 18 Gew.-%, bevorzugt von 1 bis 16 Gew.-%, weiter bevorzugt von 5 bis 14,5 Gew.-% und ganz besonders bevorzugt von 8 bis 12 Gew.-% enthält.

Mit der Anwendung des Mittels (b), umfassend ein filmbildendes Polymer als Versiegelungsreagenz (b1), soll der zunächst durch die Anwendung des Mittels (a) erzeugte gefärbte Film versiegelt und/oder fixiert werden. Mit Applikation des zweiten Mittels (b) mit einem filmbildenden Polymer als Versiegelungsreagenz (b1) lagert sich auf dem im ersten Schicht erzeugten gefärbten Film das filmbildende Polymer (b1) in Form eines weiteren Films ab. Das auf diese Weise erzeugte mehrschichtige Filmsystem eine verbesserte Widerstandsfähigkeit gegenüber äußeren Einflüssen auf.

Hierbei ist der durch das Mittel (b), umfassend ein filmbildendes Polymer als Versiegelungsreagenz (b1) erzeugte Film bevorzugt selbst nicht gefärbt. Auf diese Weise kann auch gewährleistet werden, dass ein in gewissem Ausmaß stattfindender Abrieb des durch das Mittel (b) gebildeten, zweiten Films zu keinen farblichen Veränderungen im gesamten Filmsystem führt. Daher ist es ganz besonders bevorzugt, wenn das Mittel (b) keine bzw. nur sehr geringe Mengen an farbgebenden Verbindungen enthält.

In einer alternativen Ausführungsform enthält das Versiegelungsreagenz (b1) ein Alkalisierungsmittel.

Besonders bevorzugt wird das Alkalisierungsmittel ausgewählt aus der Gruppe aus Ammoniak, C₂-C₆-Alkanolaminen, basischen Aminosäuren, Alkalimetallhydroxiden und Erdalkalimetallhydroxiden.

Im Rahmen einer weiteren besonders bevorzugten Ausführungsform ist ein Verfahren dadurch gekennzeichnet, dass das Mittel (b) mindestens ein Alkalisierungsmittel als Versiegelungsreagenz (b1) enthält, das ausgewählt ist aus der Gruppe aus Ammoniak, C₂-C₆-Alkanolaminen, basischen Aminosäuren, Alkalimetallhydroxiden, Erdalkalimetallhydroxiden, Alkalimetallsilikaten, Alkalimetallmetasilikaten, Erdalkalimetallsilikaten, Erdalkalimetallmetasilikaten, Alkalimetallcarbonaten und Erdalkalimetallcarbonaten.

Es hat sich herausgestellt, dass die Nachbehandlung mit einem Mittel (b), das Ammoniak enthält, einen besonders guten Einfluss auf die Verbesserung der Waschechtheit und der Reibechtheit der im Verfahren erhaltenen Färbungen ausübt.

Im Rahmen einer weiteren ganz besonders bevorzugten Ausführungsform ist ein Verfahren dadurch gekennzeichnet, dass die Zusammensetzung (b) als Versiegelungsreagenz (b1) Ammoniak enthält.

Ebenfalls gute Ergebnisse konnten erhalten werden, wenn die Zusammensetzung (b) als Versiegelungsreagenz (b1) mindestens ein C₂-C₆-Alkanolamin enthielt.

Die in der Zusammensetzung (b) einsetzbaren Alkanolamine können beispielsweise ausgewählt werden aus der Gruppe der primären Aminen mit einem C₂-C₆-Alkylgrundkörper, der mindestens eine Hydroxylgruppe trägt. Bevorzugte Alkanolamine werden aus der Gruppe ausgewählt, die gebildet wird aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol.

In einer weiteren bevorzugten Ausführungsform ist ein erfindungsgemäßes Verfahren dadurch gekennzeichnet, dass die Zusammensetzung (b) als Versiegelungsreagenz (b1) mindestens ein Alkalisierungsmittel aus der Gruppe der Alkanolamine enthält, das bevorzugt ausgewählt ist aus der Gruppe aus 2-Aminoethan-1-ol (Monoethanolamin), 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol und 2-Amino-2-methylpropan-1,3-diol.

Ebenfalls gute Ergebnisse konnten erhalten werden, wenn die Zusammensetzung (b) als Versiegelungsreagenz (b1) mindestens eine basische Aminosäure enthielt.

Als Aminosäure im Sinne der Erfindung gilt eine organische Verbindung, die in ihrer Struktur mindestens eine protonierbare Aminogruppe und mindestens eine -COOH- oder eine -SO₃H-Gruppe enthält. Bevorzugte Aminosäuren sind Aminocarbonsäuren, insbesondere α-(alpha)-Aminocarbonsäuren und ω-Aminocarbonsäuren, wobei α,-Aminocarbonsäuren besonders bevorzugt sind.

Unter basischen Aminosäuren sind erfindungsgemäß solche Aminosäuren zu verstehen, welche einen isoelektrischen Punkt pl von größer 7,0 besitzen.

Basische α-Aminocarbonsäuren enthalten mindestens ein asymmetrisches Kohlenstoffatom. Im Rahmen der vorliegenden Erfindung können beide möglichen Enantiomere als spezifische Verbindung oder auch deren Gemische, insbesondere als Racemate, gleichermaßen eingesetzt werden. Es ist jedoch besonders vorteilhaft, die natürlich bevorzugt vorkommende Isomerenform, üblicherweise in L-Konfiguration, einzusetzen.

Die basischen Aminosäuren werden bevorzugt ausgewählt aus der Gruppe, die gebildet wird aus Arginin, Lysin, Ornithin und Histidin, besonders bevorzugt aus Arginin und Lysin. In einer weiteren besonders bevorzugten Ausführungsform ist das Verfahren daher dadurch gekennzeichnet, dass es sich bei dem Versiegelungsreagenz (b1) um Alkalisierungsmittel, umfassend eine basische Aminosäure aus der Gruppe Arginin, Lysin, Ornithin und/oder Histidin handelt.

In einer weiteren bevorzugten Ausführungsform ist das Verfahren dadurch gekennzeichnet, dass das Mittel (b) als Versiegelungsreagenz (b1) mindestens ein Alkalisierungsmittel aus der Gruppe der basischen Aminosäuren enthält, das bevorzugt ausgewählt ist aus der Gruppe aus Arginin, Lysin, Ornithin und Histidin.

Ebenfalls gute Ergebnisse konnten erhalten werden, wenn das Mittel (b) als Versiegelungsreagenz (b1) mindestens ein Alkalimetallhydroxid enthält. Als gut geeignete Alkalimetallhydroxide können beispielsweise Natriumhydroxid und Kaliumhydroxid genannt werden.

Ebenfalls gute Ergebnisse konnten erhalten werden, wenn die Zusammensetzung (b) als Versiegelungsreagenz (b1) ein Alkalisierungsmittel, umfassend mindestens ein Erdalkalimetallhydroxid enthielt. Als gut geeignete Erdalkalimetallhydroxide können beispielsweise Magnesiumhydroxid, Calciumhydroxid und Bariumhydroxid genannt werden.

Ebenfalls gute Ergebnisse konnten erhalten werden, wenn das Mittel (b) als Versiegelungsreagenz (b1) mindestens ein Alkalimetallsilikat und/oder Alkalimetallmetasilikat enthielt. Geeignete Alkalimetallsilikate sind beispielsweise Natriumsilikat und Kaliumsilikat. Geeignete Alkalimetallmetasilikate sind beispielsweise Natriummetasilikat und Kaliummetasilikat.

Ebenfalls gute Ergebnisse konnten erhalten werden, wenn das Mittel (b) als Versiegelungsreagenz (b1) mindestens ein Alkalimetallcarbonat und/oder Erdalkalimetallcarbonat enthielt. Geeignete Alkalimetallcarbonate sind beispielsweise Natriumcarbonat und Kaliumcarbonat. Geeignete Erdalkalimetallcarbonate sind beispielsweise Magnesiumcarbonat und Calciumcarbonat.

Innerhalb der Gruppe der vorgenannten Versiegelungsreagenz (b1) in Form eines Alkalisierungsmittels haben sich Ammoniak, C₂-C₆-Alkanolaminene, basischen Aminosäuren und Alkalimetallhydroxide als ganz besonders gut geeignet herausgestellt.

Im Rahmen einer weiteren besonders bevorzugten Ausführungsform ist das Verfahren dadurch gekennzeichnet, dass das Mittel (b) als Versiegelungsreagenz (b1) mindestens ein Alkalisierungsmittel ausgewählt aus der Gruppe aus Ammoniak, C₂-C₆-Alkanolaminenen, basischen Aminosäuren und Alkalimetallhydroxiden umfasst.

Im Rahmen einer weiteren besonders bevorzugten Ausführungsform ist das Verfahren dadurch gekennzeichnet, dass das Mittel (b) als Versiegelungsreagenz (b1) mindestens ein Alkalisierungsmittel enthält, das ausgewählt ist aus der Gruppe aus Ammoniak, 2-Aminoethan-1-ol, 3-Aminopropan-1-ol, 4-Aminobutan-1-ol, 5-Aminopentan-1-ol, 1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol, Arginin, Lysin, Ornithin, Histidin, Natriumhydroxid und Kaliumhydroxid.

Das Mittel (b) enthält das Alkalisierungsmittel als Versiegelungsreagenz (b1) in einem kosmetischen Träger, bevorzugt in einem wässrigen kosmetischen Träger.

In diesem Zusammenhang hat es sich als bevorzugt herausgestellt, wenn das Mittel (b) - bezogen auf das Gesamtgewicht des Mittels (b) - 5,0 bis 99,0 Gew.-%, bevorzugt 15,0 bis 97,0 Gew.-%, weiter bevorzugt 25,0 bis 97,0 Gew.-% , nocht weiter bevorzugt 35,0 bis 97,0 Gew.-% und ganz besonders bevorzugt 45,0 bis 97,0 Gew.-% Wasser enthält.

Im Rahmen einer weiteren Ausführungsform ist das Verfahren dadurch gekennzeichnet dass das Mittel (b) - bezogen auf das Gesamtgewicht des Mittels (b) - 5,0 bis 99,0 Gew.-%, bevorzugt 15,0 bis 97,0 Gew.-%, weiter bevorzugt 25,0 bis 97,0 Gew.-% , nocht weiter bevorzugt 35,0 bis 97,0 Gew.-% und ganz besonders bevorzugt 45,0 bis 97,0 Gew.-% Wasser enthält.

Die in dem Mittel (b) enthaltenen Alkalisierungsmittel üben einen Einfluss auf den pH-Wert des Mittels (b) auf. Hierbei wurde gefunden, dass sich insbesondere bestimmte alkalische pH-Werte vorteilhaft auf die im Verfahren erzielbare Färbeleistung und die Echtheitseigenschaften der Färbungen auswirken.

Aus diesem Grund ist es bevorzugt, dass das Mittel (b), umfassend ein Alkalisierungsmittel als Versiegelungsreagenz (b1), einen pH-Wert von 7,0 bis 12,0, bevorzugt von 7,5 bis 11,5, weiter bevorzugt von 8,0 bis 11,0 und ganz besonders bevorzugt von 8,5 bis 9,5 besitzt.

Die Messung des pH-Wertes kann mit den üblichen aus dem Stand der Technik bekannten Methoden wie beispielsweise der pH-Wert Messung mittels Glaselektroden über Einstabmessketten oder aber über pH-Indikator-Papier erfolgen.

In einer weiteren ganz besonders bevorzugten Ausführungsform ist das Verfahren, dadurch gekennzeichnet, dass das Mittel (b) ein Alkalisierungsmittel als Versiegelungsreagenz (b1) enthält und einen pH-Wert von 7,0 bis 12,0, bevorzugt von 7,5 bis 11,5, weiter bevorzugt von 8,0 bis 11,0 und ganz besonders bevorzugt von 8,5 bis 9,5 besitzt.

Bei den pH-Werten im Sinne der vorliegenden Erfindung handelt es sich um pH-Werte, die bei einer Temperatur von 22°C gemessen wurden.

In einer noch weiteren alternativen Ausführungsform enthält das Versiegelungsreagenz (b1) ein Acidifizierungsmittel.

Besonders bevorzugt wird das Acidifizierungsmittel ausgewählt aus der Gruppe bestehend aus anorganischen Säuren, organischen Säuren und Mischungen daraus.

Gute Ergebnisse konnten erhalten werden, wenn das Mittel (b) als Versiegelungsreagenz (b1) mindestens eine anorganische Säure enthält. Geeignete anorganische Säuren sind zum Beispiel Phosphorsäure, Schwefelsäure und/oder Salzsäure, wobei Schwefelsäure besonders bevorzugt ist.

In einer weiteren bevorzugten Ausführungsform ist das Verfahren dadurch gekennzeichnet, dass das Mittel (b) als Versiegelungsreagenz (b1) mindestens ein Acidifizierungsmittel aus der Gruppe der anorganischen Säuren enthält, die bevorzugt ausgewählt ist aus der Gruppe bestehend aus Phosphorsäure, Schwefelsäure, Salzsäure und Mischungen daraus.

In einer weiteren, noch mehr bevorzugten Ausführungsform ist das Verfahren dadurch gekennzeichnet, dass das Mittel (b) als Versiegelungsreagenz (b1) Schwefelsäure enthält.

Ebenfalls gute Ergebnisse konnten erhalten werden, wenn das Mittel (b) als Versiegelungsreagenz (b1) mindestens eine organische Säure enthält. Die organische Säure ist vorzugsweise ausgewählt aus der Gruppe bestehend Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, Glycolsäure, Gluconsäure, Milchsäure, Maleinsäure, Ascorbinsäure, Äpfelsäure, Weinsäure, Citronensäure und Mischungen daraus.

In einer weiteren bevorzugten Ausführungsform ist das Verfahren dadurch gekennzeichnet, dass das Mittel (b) als Versiegelungsreagenz (b1) mindestens ein Acidifizierungsmittel aus der Gruppe der organischen Säuren enthält, wobei die organische Säure vorzugsweise ausgewählt ist aus der Gruppe bestehend aus Ameisensäure, Essigsäure, Propionsäure, Buttersäure, Isobuttersäure, Valeriansäure, Isovaleriansäure, Pivalinsäure, Oxalsäure, Malonsäure, Bernsteinsäure, Glutarsäure, Glycerinsäure, Glyoxylsäure, Adipinsäure, Pimelinsäure, Korksäure, Azelainsäure, Sebacinsäure, Propiolsäure, Crotonsäure, Isocrotonsäure, Elaidinsäure, Maleinsäure, Fumarsäure, Muconsäure, Citraconsäure, Mesaconsäure, Camphersäure, Benzoesäure, o,m,p-Phthalsäure, Naphthoesäure, Toluoylsäure, Hydratropasäure, Atropasäure, Zimtsäure, Isonicotinsäure, Nicotinsäure, Bicarbaminsäure, 4,4'-Dicyano-6,6'-binicotinsäure, 8-Carbamoyloctansäure, 1,2,4-Pentantricarbonsäure, 2-Pyrrolcarbonsäure, 1,2,4,6,7-Napthalinpentaessigsäure, Malonaldehydsäure, 4-Hydroxy-phthalamidsäure, 1-Pyrazolcarbonsäure, Gallussäure oder Propantricarbonsäure, Glycolsäure, Gluconsäure, Milchsäure, Maleinsäure, Ascorbinsäure, Äpfelsäure, Weinsäure, Citronensäure und Mischungen daraus.

In einer weiteren, noch mehr bevorzugten Ausführungsform ist das Verfahren dadurch gekennzeichnet, dass das Mittel (b) als Versiegelungsreagenz (b1) Essigsäure enthält.

Ebenfalls geeignete Acidifizierungsmittel umfassen Methansulfonsäure und/oder 1-Hydroxyethan-1,1-diphosphonsäure.

Innerhalb der Gruppe der vorgenannten Versiegelungsreagenzien (b1) in Form eines Acidifizierungsmittels haben sich Schwefelsäure und/oder Essigsäure als ganz besonders gut geeignet herausgestellt.

Im Rahmen einer weiteren besonders bevorzugten Ausführungsform ist das Verfahren dadurch gekennzeichnet, dass das Mittel (b) als Versiegelungsreagenz (b1) mindestens ein Acidifizierungsmittel ausgewählt aus der Gruppe aus Schwefelsäure, Essigsäure und Mischungen daraus umfasst.

Das Mittel (b) enthält das Acidifizierungsmittel als Versiegelungsreagenz (b1) in einem kosmetischen Träger, bevorzugt in einem wässrigen kosmetischen Träger.

Die in dem Mittel (b) enthaltenen Acidifizierungsmittel üben einen Einfluss auf den pH-Wert des Mittels (b) auf. Hierbei wurde gefunden, dass sich auch saure pH-Werte vorteilhaft auf die im Verfahren erzielbare Färbeleistung und die Echtheitseigenschaften der Färbungen auswirken.

Aus diesem Grund ist es bevorzugt, dass das Mittel (b), umfassend ein Acidifizierungsmittel als Versiegelungsreagenz (b1), einen pH-Wert von 2,0 bis 6,5, bevorzugt von 3,0 bis 6,0, weiter bevorzugt von 4,0 bis 6,0 und ganz besonders bevorzugt von 4,5 bis 5,5 besitzt.

Die Messung des pH-Wertes kann mit den üblichen aus dem Stand der Technik bekannten Methoden wie beispielsweise der pH-Wert Messung mittels Glaselektroden über Einstabmessketten oder aber über pH-Indikator-Papier erfolgen.

In einer weiteren ganz besonders bevorzugten Ausführungsform ist das Verfahren, dadurch gekennzeichnet, dass das Mittel (b) ein Acidifizierungsmittel als Versiegelungsreagenz (b1) enthält und einen pH-Wert von 2,0 bis 6,5, bevorzugt von 3,0 bis 6,0, weiter bevorzugt von 4,0 bis 6,0 und ganz besonders bevorzugt von 4,5 bis 5,5 besitzt.

Bei den pH-Werten im Sinne der vorliegenden Erfindung handelt es sich um pH-Werte, die bei einer Temperatur von 22°C gemessen wurden.

Mit der Anwendung des Mittels (b) soll der zunächst durch die Anwendung des Mittels (a) erzeugte Film versiegelt und fixiert werden.

### Weitere Inhaltsstoffe in den Mitteln (a) und (b)

Die zuvor beschriebenen Mittel (a) und (b) können ferner auch noch ein oder mehrere optionale Inhaltsstoffe enthalten.

Die Mittel können zusätzlich ein oder mehrere Tenside enthalten. Unter dem Begriff Tenside werden grenzflächenaktive Substanzen verstanden. Man unterscheidet anionische Tenside bestehend aus einem hydrophoben Rest und einer negativ geladenen hydrophilen Kopfgruppe, amphotere Tenside, welche sowohl eine negative als auch eine kompensierende positive Ladung tragen, kationische Tenside, welche neben einem hydrophoben Rest eine positiv geladene hydrophile Gruppe aufweisen, und nichtionische Tenside, welche keine Ladungen sondern starke Dipolmomente aufweisen und in wässriger Lösung stark hydratisiert sind.

Als zwitterionische Tenside werden solche oberflächenaktiven Verbindungen bezeichnet, die im Molekül mindestens eine quartäre Ammoniumgruppe und mindestens eine -COO⁽⁻⁾ - oder -SO₃⁽⁻⁾ - Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyldimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der INCI-Bezeichnung Cocamidopropyl Betaine bekannte Fettsäureamid-Derivat.

Unter ampholytischen Tensiden werden solche oberflächenaktiven Verbindungen verstanden, die außer einer C₈ - C₂₄ -Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 24 C-Atomen in der Alkylgruppe. Typische Beispiele für amphotere bzw. zwitterionische Tenside sind Alkylbetaine, Alkylamidobetaine, Amino-propionate, Aminoglycinate, Imidazoliniumbetaine und Sulfobetaine.

Besonders bevorzugte ampholytische Tenside sind das N-Kokosalkylaminopropionat, das Kokosacylaminoethylaminopropionat und das C₁₂ - C₁₈ -Acylsarcosin.

Die Mittel können auch zusätzlich mindestens ein nichtionisches Tensid enthalten. Geeignete nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit guten Eigenschaften werden ebenfalls erhalten, wenn sie als nichtionische Tenside Fettsäureester von ethoxyliertem Glycerin enthalten, die mit mindestens 2 Mol Ethylenoxid umgesetzt wurden.

Weiterhin können die Mittel zusätzlich auch mindestens ein kationisches Tensid enthalten. Unter kationischen Tensiden werden Tenside, also grenzflächenaktive Verbindungen, mit jeweils einer oder mehreren positiven Ladungen verstanden. Kationische Tenside enthalten ausschließlich positive Ladungen. Üblicherweise sind diese Tenside aus einem hydrophoben Teil und einer hydrophilen Kopfgruppe aufgebaut, wobei der hydrophobe Teil in der Regel aus einem Kohlenwasserstoff-Gerüst (z.B. bestehend aus einer oder zwei linearen oder verzweigten Alkylketten) besteht, und die positive(n) Ladung(en) in der hydrophilen Kopfgruppe lokalisiert sind. Beispiele für kationische Tenside sind
- quartäre Ammoniumverbindungen, die als hydrophobe Reste ein oder zwei Alkylketten mit einer Kettenlänge von 8 bis 28 C-Atomen tragen können,
- quartäre Phosphoniumsalze, substituiert mit einer oder mehreren Alkylketten mit einer Kettenlänge von 8 bis 28 C-Atomen oder
- tertiäre Sulfonium-Salze.

Weiterhin kann die kationische Ladung auch in Form einer Onium-Struktur Bestandteil eines heterozyklischen Ringes (z.B. eines Imidazoliumringe oder einer Pyridiniumringes) sein. Neben der funktionellen Einheit, welche die kationische Ladung trägt, kann das kationische Tensid auch weitere ungeladene funktionelle Gruppen beinhalten, wie dies beispielsweise bei Esterquats der Fall ist. Die kationischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Weiterhin können die Mittel auch mindestens ein anionisches Tensid enthalten. Als anionische Tenside werden oberflächenaktive Mittel mit ausschließlich anionischen Ladungen (neutralisiert durch ein entsprechendes Gegenkation) bezeichnet. Beispiele für anionische Tenside sind Fettsäuren, Alkylsulfate, Alkylethersulfate und Ethercarbonsäuren mit 12 bis 20 C-Atomen in der Alkylgruppe und bis zu 16 Glykolethergruppen im Molekül.

Die anionischen Tenside werden in einer Gesamtmenge von 0,1 bis 45 Gew.-%, bevorzugt 1 bis 30 Gew.-% und ganz besonders bevorzugt von 1 bis 15 Gew.-% - bezogen auf das Gesamtgewicht des jeweiligen Mittels - eingesetzt.

Das Mittel (a) und/oder das Mittel (b) können ferner ein Mattierungsmittel enthalten. Geeignete Mattierungsmittel umfassen beispielsweise (modifizierte) Stärken, Wachse, Talkum und/oder (modifizierte) Kieselsäuren. Die Menge an Mattierungsmittel liegt bevorzugt zwischen 0,1 und 10 Gew.-% bezogen auf die Gesamtmenge an Mittel (a) oder Mittel (b). Bevorzugt enthält Mittel (a) ein Mattierungsmittel.

Die Mittel können ferner auch noch weitere Wirk-, Hilfs- und Zusatzstoffe enthalten, wie beispielsweise Lösungsmittel, Fettbestandteile wie beispielsweise der C₈-C₃₀-Fettsäuretriglyceride, der C₈-C₃₀-Fettsäuremonoglyceride, der C₈-C₃₀-Fettsäurediglyceride und/oder der Kohlenwasserstoffe; Strukturanten wie Glucose, Maleinsäure und Milchsäure, haarkonditionierende Verbindungen wie Phospholipide, beispielsweise Lecithin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; sowie Treibmittel wie Propan-Butan-Gemische, N₂O, Dimethylether, CO₂ und Luft.

Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Zubereitungen bevorzugt in Mengen von jeweils 0,0001 bis 25 Gew.-%, insbesondere von 0,0005 bis 15 Gew.-%, bezogen auf das Gesamtgewicht des jeweiligen Mittels, eingesetzt.

### Verfahren zum Färben von keratinischen Materialien

Im Rahmen des erfindungsgemäßen Verfahrens werden die Mittel (a) und (b) auf die keratinischen Materialien, insbesondere auf die menschlichen Haare, appliziert. Damit sind die Mittel (a) und (b) die anwendungsbereiten Mittel. Die Mittel (a) und (b) sind voneinander verschieden.

Die Mittel (a) und (b) können prinzipiell gleichzeitig oder sukzessive angewendet werden, wobei die sukzessive Anwendung bevorzugt ist.

Die besten Ergebnisse konnten erhalten werden, wenn zunächst in einem ersten Schritt das Mittel (a) auf die keratinischen Materialien gegeben wurde und in einem zweiten Schritt das Mittel (b) angewendet wurde.

Ganz besonders bevorzugt ist daher ein Verfahren zum Behandeln von keratinischem Material, insbesondere zum Färben von keratinischem Material, insbesondere menschlichen Haaren, umfassend die folgenden Schritte in der angegebenen Reihenfolge:
∘ in einem ersten Schritt Anwendung eines Mittels (a) auf dem keratinischem Material, wobei das Mittel (a) enthält:
   (a1) mindestens zwei ausgewählte organische Siliciumverbindungen, und
   (a2) mindestens eine farbgebende Verbindung, umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, und
   in einem zweiten Schritt Anwendung eines Mittels (b) auf dem keratinischen Material, wobei das Mittel (b) enthält:
   (b1) mindestens ein Versiegelungsreagenz, umfassend eine Verbindung ausgewählt aus der Gruppe bestehend aus filmbildenden Polymeren, Alkalisierungsmitteln, Acidifizierungsmittel und Mischungen daraus.

Um dem gefärbten keratinischen Material über einen längeren Zeitraum eine hohe Auswaschresistenz zu verleihen, werden die Mittel (a) und (b) zudem besonders bevorzugt innerhalb ein und desselben Färbeverfahrens angewendet, was bedeutet, dass zwischen der Anwendung der Mittel (a) und (c) ein Zeitraum von maximal einigen Stunden liegt.

Im Rahmen einer weiteren bevorzugten Ausführungsform ist das Verfahren dadurch gekennzeichnet, das zunächst das Mittel (a) angewendet wird und danach das Mittel (b) angewendet wird, wobei der Zeitraum zwischen der Anwendung der Mittel (a) und (b) bei maximal 24 Stunden, bevorzugt bei maximal 12 Stunden und besonders bevorzugt bei maximal 6 Stunden liegt.

Ein kennzeichnendes Merkmal des Mittels (a) ist sein Gehalt an mindestens zwei reaktiven organischen Siliciumverbindungen (a1). Die reaktiven organischen Siliciumverbindungen (a1) gehen eine Oligomerisierungs- oder Polymerisierungsreaktion ein und funktionalisieren auf diesem Wege die Haaroberfläche, sobald sie mit dieser in Kontakt kommen. Auf diesem Wege wird ein erster, Film ausgebildet. Die farbgebenden Verbindungen (a2) werden in den Film inkorporiert, so dass dieser gefärbt ist. Im zweiten Schritt des Verfahrens wird nun ein zweites Mittel (b) auf die Haare aufgetragen. Während der Anwendung des Mittels (b), umfassend mindestens ein filmbildendes Polymer als Versiegelungsreagenz (b1), geht dieses eine Wechselwirkung mit dem Silan-Film ein und werden auf diese Weise an die keratinischen Materialien gebunden. Während der Anwendung des Mittels (b), umfassend mindestens ein Alkalisierungsmittel oder Acidifizierungsmittel als Versiegelungsreagenz (b1), wird die Ausbildung des Silan-Films positiv beeinflusst.

Im Rahmen einer weiteren Ausführungsform ganz besonders bevorzugt ist ein Verfahren, umfassend die folgenden Schritte in der angegebenen Reihenfolge
(1) Anwendung des Mittels (a) auf dem keratinischen Material,
(2) Einwirken lassen des Mittels (a) für einen Zeitraum von 10 Sekunden bis 10 Minuten, bevorzugt von 10 Sekunden bis 5 Minuten,
(3) gegebenenfalls Ausspülen des keratinischen Materials mit Wasser,
(4) Anwendung des Mittels (b) auf dem keratinischen Material,
(5) Einwirken lassen des Mittels (b) für einen Zeitraum von 30 Sekunden bis 30 Minuten, bevorzugt von 30 Sekunden bis 10 Minuten,
(6) Ausspülen des keratinischen Materials mit Wasser.

Unter dem Ausspülen des keratinischen Materials mit Wasser in den Schritten (3) und (6) des Verfahrens wird erfindungsgemäß verstanden, dass für den Ausspülvorgang nur Wasser verwendet wird, ohne dass noch weitere, von den Mitteln (a) und (b) verschiedene Mittel zur Anwendung kämen.

In einem Schritt (1) wird zunächst das Mittel (a) auf die keratinischen Materialien, insbesondere die menschlichen Haare, appliziert.

Nach dem Auftragen wird das Mittel (a) auf die keratinischen Materialien einwirken gelassen. In diesem Zusammenhang haben sich Einwirkzeiten von 10 Sekunden bis 10 Minuten, bevorzugt von 20 Sekunden bis 5 Minuten und ganz besonders bevorzugt von 30 Sekunden bis 2 Minuten auf den Haaren haben sich als besonders vorteilhaft erwiesen.

Im Rahmen einer bevorzugten Ausführungsform des Verfahrens kann das Mittel (a) nun von den keratinischen Materialien ausgespült werden, bevor das Mittel (b) im nachfolgenden Schritt auf die Haare appliziert wird.

Färbungen mit ebenfalls guten Waschechtheiten wurden erhalten, wenn das Mittel (b) auf die keratinischen Materialien appliziert wurde, die noch mit dem Mittel (a) beaufschlagt waren.

In Schritt (4) wird nun das Mittel (b) auf die keratinischen Materialien appliziert. Nach dem Auftragen wird nun das Mittel (b) auf die Haare einwirken gelassen.

Das Verfahren erlaubt selbst bei kurzer Einwirkzeit des Mittels (b) die Erzeugung von Färbungen mit besonders guter Intensität und Waschechtheit. Einwirkzeiten von 10 Sekunden bis 10 Minuten, bevorzugt von 20 Sekunden bis 5 Minuten und ganz besonders bevorzugt von 30 Sekunden bis 3 Minuten auf den Haaren haben sich als besonders vorteilhaft erwiesen.

In Schritt (6) wird nun das Mittel (b) (sowie ggf. noch vorhandenes Mittel (a)) mit Wasser aus dem keratinischen Material ausgespült.

In Rahmen dieser Ausführungsform erfolgt die Abfolge der Schritte (1) bis (6) bevorzugt innerhalb von 24 Stunden.

Das Mittel (a) enthält mit den organischen Siliciumverbindungen eine Klasse von hochreaktiven Verbindungen, die bei ihrer Anwendung eine Hydrolyse oder Oligomerisierung und/oder Polymerisierung eingehen können. Infolge ihrer hohen Reaktivität bilden diese organischen Siliciumverbindungen auf dem keratinischen Material einen Film aus.

Zur Vermeidung der vorzeitigen Oligomerisierung bzw. Polymerisierung ist es für den Anwender von wesentlichem Vorteil, das anwendungsbereite Mittel (a) erst kurz vor der Anwendung herstellen.

Im Rahmen einer weiteren Ausführungsform bevorzugt ist ein Verfahren, umfassend die folgenden Schritte in der angegebenen Reihenfolge
(1) Herstellung eines Mittels (a) durch Vermischen eines ersten Mittels (a') und eines zweiten Mittels (a"), wobei
   ∘ das erste Mittel (a') mindestens zwei ausgewählte organische Siliciumverbindungen (a1) und das zweite Mittel (a") mindestens eine farbgebende Verbindung (a2), umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, enthält,
(2) Anwendung des Mittels (a) auf dem keratinischen Material,
(3) Einwirken lassen des Mittels (a) für einen Zeitraum von 10 Sekunden bis 10 Minuten, bevorzugt von 10 Sekunden bis 5 Minuten,
(4) gegebenenfalls Ausspülen des keratinischen Materials mit Wasser,
(5) Anwendung des Mittels (b) auf dem keratinischen Material,
(6) Einwirken lassen des Mittels (b) für einen Zeitraum von 30 Sekunden bis 30 Minuten, bevorzugt von 30 Sekunden bis 10 Minuten,
(7) Ausspülen des keratinischen Materials mit Wasser.

Um eine möglichst lagerstabile Formulierung bereitstellen zu können, wird das Mittel (a') selbst bevorzugt wasserarm oder wasserfrei konfektioniert.

In einer bevorzugten Ausführungsform ist eine Mehrkomponenten-Verpackungseinheit (Kit-of-parts) dadurch gekennzeichnet, dass das Mittel (a') - bezogen auf das Gesamtgewicht des Mittels (a') - einen Wassergehalt von 0,001 bis 10 Gew.-%, bevorzugt von 0,5 bis 9 Gew.-%, weiter bevorzugt von 1 bis 8 Gew.-% und ganz besonders bevorzugt von 1,5 bis 7 Gew.-% enthält.

Das Mittel (a") enthält Wasser. In einer bevorzugten Ausführungsform ist eine Mehrkomponenten-Verpackungseinheit (Kit-of-parts) dadurch gekennzeichnet, dass das Mittel (a") - bezogen auf das Gesamtgewicht des Mittels (a2) - einen Wassergehalt von 15 bis 100 Gew.-%, bevorzugt von 35 bis 100 Gew.-%, weiter bevorzugt von 55 bis 100 Gew.-%, noch weiter bevorzugt von 65 bis 100 Gew.-% und ganz besonders bevorzugt von 75 bis 100 Gew.-% besitzt.

Innerhalb dieser Ausführungsform wird das anwendungsbereite Mittel (a) nun durch Vermischen der Mittel (a') und (a") hergestellt.

Beispielsweise kann der Anwender das Mittel (a'), welches die organische Siliciumverbindungen (a1) enthält, zunächst mit dem wasserhaltigen, Effektpigment-haltigen Mittel (a") verrühren oder verschütteln. Diese Mischung aus (a') und (a") kann der Anwender nun - entweder direkt nach ihrer Herstellung oder nach einer kurzen Reaktionszeit von 10 Sekunden bis 20 Minuten - auf die keratinischen Materialien applizieren. Im Anschluss daran kann der Anwender wie zuvor beschrieben das Mittel (b) anwenden.

Das optional enthaltene Silikonpolymer (a3) kann in dem Mittel (a') oder in dem Mittel (a") enthalten sein. Bevorzugt ist das Silikonpolymer (a3) in dem Mittel (a") enthalten.

Im Rahmen einer weiteren Ausführungsform bevorzugt ist ein Verfahren, umfassend die folgenden Schritte in der angegebenen Reihenfolge
(1) Herstellung eines Mittels (a) durch Vermischen eines ersten Mittels (a') und eines zweiten Mittels (a"), wobei
   ∘ das erste Mittel (a') mindestens zwei ausgewählte organische Siliciumverbindungen (a1) und weiterhin mindestens ein Silikonpolymer (a3) enthält, und
   ∘ das zweite Mittel (a") mindestens eine farbgebende Verbindung (a2), umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, enthält,
(2) Anwendung des Mittels (a) auf dem keratinischen Material,
(3) Einwirken lassen des Mittels (a) für einen Zeitraum von 10 Sekunden bis 10 Minuten, bevorzugt von 10 Sekunden bis 5 Minuten,
(4) gegebenenfalls Ausspülen des keratinischen Materials mit Wasser,
(5) Anwendung des Mittels (b) auf dem keratinischen Material,
(6) Einwirken lassen des Mittels (b) für einen Zeitraum von 30 Sekunden bis 30 Minuten, bevorzugt von 30 Sekunden bis 10 Minuten,
(7) Ausspülen des keratinischen Materials mit Wasser.

Im Rahmen einer weiteren Ausführungsform besonders bevorzugt ist ein Verfahren, umfassend die folgenden Schritte in der angegebenen Reihenfolge
(1) Herstellung eines Mittels (a) durch Vermischen eines ersten Mittels (a') und eines zweiten Mittels (a"), wobei
   ∘ das erste Mittel (a') mindestens zwei ausgewählte organische Siliciumverbindungen (a1) enthält, und
   ∘ das zweite Mittel (a") mindestens eine farbgebende Verbindung (a2), umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, und weiterhin mindestens ein Silikonpolymer (a3) enthält,
(2) Anwendung des Mittels (a) auf dem keratinischen Material,
(3) Einwirken lassen des Mittels (a) für einen Zeitraum von 10 Sekunden bis 10 Minuten, bevorzugt von 10 Sekunden bis 5 Minuten,
(4) gegebenenfalls Ausspülen des keratinischen Materials mit Wasser,
(5) Anwendung des Mittels (b) auf dem keratinischen Material,
(6) Einwirken lassen des Mittels (b) für einen Zeitraum von 30 Sekunden bis 30 Minuten, bevorzugt von 30 Sekunden bis 10 Minuten,
(7) Ausspülen des keratinischen Materials mit Wasser.

Im Rahmen einer weiteren bevorzugten Ausführungsform kann ein Verfahren auch dadurch gekennzeichnet sein, dass das bzw. die Silikonpolymere (a3) in einem dritten getrennt konfektionierten Mittel (a‴) zur Verfügung gestellt werden.

Im Rahmen dieser weiteren Ausführungsform bevorzugt ist ein Verfahren, umfassend die folgenden Schritte in der angegebenen Reihenfolge
(1) Herstellung eines Mittels (a) durch Vermischen eines ersten Mittels (a') und eines zweiten Mittels (a") und eines dritten Mittels (a‴), wobei
   das erste Mittel (a') mindestens zwei ausgewählte organische Siliciumverbindungen (a1) aus der Gruppe der Silane mit einem, zwei oder drei Siliciumatomen enthält, und
   das zweite Mittel (a") mindestens eine farbgebende Verbindung (a2), umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, enthält, und
   das dritte Mittel (a‴) mindestens ein Silikonpolymer (a3) enthält,
(2) Anwendung des Mittels (a) auf dem keratinischen Material,
(3) Einwirken lassen des Mittels (a) für einen Zeitraum von 10 Sekunden bis 10 Minuten, bevorzugt von 10 Sekunden bis 5 Minuten,
(4) gegebenenfalls Ausspülen des keratinischen Materials mit Wasser,
(5) Anwendung des Mittels (b) auf dem keratinischen Material,
(6) Einwirken lassen des Mittels (b) für einen Zeitraum von 30 Sekunden bis 30 Minuten, bevorzugt von 30 Sekunden bis 10 Minuten,
(7) Ausspülen des keratinischen Materials mit Wasser.

### Mehrkomponenten-Verpackungseinheit (Kit-of-parts)

Zur Erhöhung des Anwenderkomforts werden dem Anwender bevorzugt alle benötigten Mittel in Form einer Mehrkomponenten-Verpackungseinheit (Kit-of-parts) zur Verfügung gestellt.

Ein zweiter Gegenstand der vorliegenden Erfindung ist daher eine Mehrkomponenten-Verpackungseinheit (Kit-of-parts) zum Färben von keratinischem Material, umfassend getrennt voneinander konfektioniert
- einen ersten Container mit einem Mittel (a'), wobei das Mittel (a') enthält:
   (a1) mindestens eine organische Siliciumverbindung der Formel (I) und mindestens eine organische Siliciumverbindung der Formel (IV),
   wobei in der organischen Siliciumverbindung der Formel (I)

      R₁R₂N-L-Si(OR₃)ₐ(R₄)_{b} (I),

      - R₁, R₂ unabhängig voneinander für ein Wasserstoffatom oder eine C₁-C₆-Alkylgruppe stehen,
      - L für eine lineare oder verzweigte, zweiwertige C₁-C₂₀-Alkylengruppe steht,
      - R₃ für ein Wasserstoffatom oder für eine C₁-C₆-Alkylgruppe steht,
      - R₄ für eine C₁-C₆-Alkylgruppe steht,
      - a, für eine ganze Zahl von 1 bis 3 steht, und
      - b für die ganze Zahl 3 - a steht, und
   wobei in der organischen Siliciumverbindung der Formel (IV)

      R₉Si(OR₁₀)ₖ(R₁₁)ₘ (IV),

      - R₉ für eine C₁-C₁₈-Alkylgruppe steht,
      - R₁₀ für ein Wasserstoffatom oder eine C₁-C₆-Alkylgruppe steht,
      - R₁₁ für eine C₁-C₆-Alkylgruppe steht
      - k für eine ganze Zahl von 1 bis 3 steht, und
      - m für die ganze Zahl 3 - k steht, und
- einen zweiten Container mit einem Mittel (a"), wobei das Mittel (a") enthält:
   (a2) mindestens eine farbgebende Verbindung, umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, und
- einen dritten Container mit einem Mittel (b), wobei das Mittel (b) enthält:
   (b1) mindestens ein Versiegelungsreagenz, umfassend eine Verbindung ausgewählt aus der Gruppe bestehend aus filmbildenden Polymeren, Alkalisierungsmitteln, Acidifizierungsmittel und Mischungen daraus,
wobei die Bestandteile (a1), (a2) und (b1) im Detail oben offenbart wurden.

Die im Mittel (a') des Kits enthaltenen organischen Siliciumverbindungen (a1) aus der Gruppe der Silane mit einem, zwei oder drei Siliciumatomen entsprechen den organischen Siliciumverbindungen (a1), die auch im Mittel (a) des zuvor beschriebenen Verfahrens eingesetzt wurden.

Die im Mittel (a") des Kits enthaltenen farbgebenden Verbindungen (a2), umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, entsprechen den farbgebenden Verbindungen (a2), umfassend ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, die auch im Mittel (a) des zuvor beschriebenen Verfahrens eingesetzt wurden.

Das im Mittel (b) des Kits enthaltene Versiegelungsreagenz (b1) entspricht dem Versiegelungsreagenz, das auch im Mittel (b) des zuvor beschriebenen Verfahrens eingesetzt wurde.

Auch in diesem Zusammenhang ist es wieder möglich, das optional enthaltene Silikonpoylmer (a3) Im Mittel (a'), im Mittel (a") oder in einem weiteren Mittel (a‴) zu konfektionieren.

Im Rahmen einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (a'), wobei das Mittel (a') enthält:
   mindestens zwei ausgewählte organische Siliciumverbindungen (a1) und weiterhin mindestens ein Silikonpolymer (a3), und
- einen zweiten Container mit einem Mittel (a"), wobei das Mittel (a") enthält:
   (a2) mindestens eine farbgebende Verbindung, umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, und
- einen dritten Container mit einem Mittel (b), wobei das Mittel (b) enthält:
   (b1) mindestens ein Versiegelungsreagenz,
wobei die Bestandteile (a1), (a2), (a3) und (b1) im Detail oben offenbart wurden.

Im Rahmen einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (a'), wobei das Mittel (a') enthält:
   mindestens zwei ausgewählte organische Siliciumverbindungen (a1),
- einen zweiten Container mit einem Mittel (a"), wobei das Mittel (a") enthält:
   (a2) mindestens eine farbgebende Verbindung, umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, und weiterhin mindestens ein Silikonpolymer (a3), und
- einen dritten Container mit einem Mittel (a‴), wobei das Mittel (a‴) ein wasserhaltiger kosmetischer Träger ist
- einen vierten Container mit einem Mittel (b), wobei das Mittel (b) enthält:
   (b1) mindestens ein Versiegelungsreagenz,
wobei die Bestandteile (a1), (a2), (a3) und (b1) im Detail oben offenbart wurden.

Im Rahmen dieser Ausführungsform besitzen die Mittel (a') und (a") einen geringen Wassergehalt. Zur Herstellung des anwendungsbereiten Mittels (a) werden die Mittel (a'), (a") und (a‴) vermischt. Das Mittel (a‴) stellt hierbei einen wasserhaltigen kosmetischen Träger dar.

Im Rahmen einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (a'), wobei das Mittel (a') enthält:
   mindestens zwei ausgewählte organische Siliciumverbindungen (a1),
- einen zweiten Container mit einem Mittel (a"), wobei das Mittel (a") enthält:
   (a2) mindestens eine farbgebende Verbindung, umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, und weiterhin mindestens ein Silikonpolymer (a3), und
- einen dritten Container mit einem Mittel (b), wobei das Mittel (b) enthält:
   (b1) mindestens ein Versiegelungsreagenz,
wobei die Bestandteile (a1), (a2), (a3) und (b1) im Detail oben offenbart wurden.

Im Rahmen einer weiteren Ausführungsform bevorzugt ist eine Mehrkomponenten-Verpackungseinheit (Kit-of-parts) zum Färben von keratinischem Material, umfassend getrennt voneinander konfektioniert
- einen ersten Container mit einem Mittel (a'), wobei das Mittel (a') enthält:
   mindestens zwei ausgewählte organische Siliciumverbindungen (a1),
- einen zweiten Container mit einem Mittel (a"), wobei das Mittel (a") enthält:
   (a2) mindestens eine farbgebende Verbindung, umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist,
- einen dritten Container mit einem Mittel (a‴), wobei das Mittel (a‴) enthält:
   mindestens ein Silikonpolymer (a3), und
- einen vierten Container mit einem Mittel (b), wobei das Mittel (b) enthält:
   (b1) mindestens ein Versiegelungsreagenz,
wobei die Bestandteile (a1), (a2), (a3) und (b1) im Detail oben offenbart wurden.

In dieser Ausführungsform der Mehrkomponenten-Verpackungseinheit ist es bevorzugt, dass das Mittel (a‴) ferner mindestens eine weitere farbgebende Verbindung (a2) enthält.

Betreffend die weiteren bevorzugten Ausführungsformen der Mehrkomponenten-Verpackungseinheit gilt *mutatis mutantis* das zum Verfahren gesagte.

Beispiele

### Beispiel 1

Es wurden die folgenden Formulierungen hergestellt (sofern nichts anderes angegeben ist, sind alle Angaben in Gew.-%)

### Mittel (a')

| Mittel (a') | Gew.-% |
|---|---|
| (3-Aminopropyl)triethoxysilan (a1) | 20 |
| Methyltrimethoxysilan (a1) | 70 |
| Wasser | ad 100 |

### Mittel (a")

| Mittel (a") | Gew.-% |
|---|---|
| Effektpigment gemäß Anspruch 1 (a2) | 5 |
| PEG-12 Dimethicone (a3) | 5 |
| Hydroxyethylcellulose | 1 |
| Wasser | ad 100 |

Das anwendungsbereite Mittel (a) wurde durch Vermischen von 5 g des Mittels (a') und 20 g des Mittels (a") hergestellt. Der pH-Wert des Mittels (a) wurde durch Zugabe von Ammoniak bzw. Milchsäure auf einen Wert von 10,5 eingestellt. Dann wurde das Mittel (a) für ca. 5 Minuten stehen gelassen.

### Mittel (b)

| Mittel (b) | Gew.-% |
|---|---|
| Ethylene/Sodium Acrylate Copolymer (b1) (25%ige Lösung) | 40 |
| Wasser | ad 100 |

Das Mittel (a) wurde jeweils in eine Haarsträhne (Kerling, Euronaturhaar weiß) einmassiert, und für 1 Minute einwirken gelassen. Danach wurde das Mittel (a) mit Wasser ausgespült.

Im Anschluss daran wurde das Mittel (b) auf die Haarsträhne appliziert, für 1 Minute einwirken gelassen und danach ebenfalls mit Wasser ausgespült.

## Patentansprüche

1. Verfahren zum Färben von keratinischem Material, insbesondere menschlichen Haaren, umfassend die folgenden Schritte:
- Anwendung eines Mittels (a) auf dem keratinischem Material, wobei das Mittel (a) enthält:
(a1) mindestens eine organische Siliciumverbindung der Formel (I) und mindestens eine organische Siliciumverbindung der Formel (IV),
wobei in der organischen Siliciumverbindung der Formel (I)
R₁R₂N-L-Si(OR₃)ₐ(R₄)_{b} (I),
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder für eine C₁-C₆-Alkylgruppe steht,
- R₄ für eine C₁-C₆-Alkylgruppe steht,
- a, für eine ganze Zahl von 1 bis 3 steht, und
- b für die ganze Zahl 3 - a steht, und
wobei in der organischen Siliciumverbindung der Formel (IV)
R₉Si(OR₁₀)ₖ(R₁₁)ₘ (IV),
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht,, und
(a2) mindestens eine farbgebende Verbindung, enthaltend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist, und
- Anwendung eines Mittels (b) auf dem keratinischen Material, wobei das Mittel (b) enthält:
(b1) mindestens ein Versiegelungsreagenz, umfassend eine Verbindung ausgewählt aus der Gruppe bestehend aus filmbildenden Polymeren, Alkalisierungsmitteln, Acidifizierungsmittel und Mischungen daraus.

2. Verfahren nach Anspruch 1, **dadurch gekennzeichnet, dass** das Mittel (a) ferner mindestens eine organische Siliciumverbindung (a1) der Formel (II) enthält
wobei in der organischen Siliciumverbindung der Formel (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R5, R5', R5", R6, R6' und R6" unabhängig voneinander für eine C₁-C₆-Alkylgruppe stehen,
- A, A', A", A‴ und Aʺʺ unabhängig voneinander für eine lineare oder verzweigte, zweiwertige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III) stehen
- (Aʺʺ)-Si(R₆")_{d}"(OR₅")_{c}" (III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

3. Verfahren nach einem der Ansprüche 1 bis 2, **dadurch gekennzeichnet, dass** das Mittel (a) mindestens eine organische Siliciumverbindung (a1) der Formel (I) enthält,
R₁R₂N-L-Si(OR₃)ₐ(R₄)_{b} (I),
wobei
- R₁, R₂ beide für ein Wasserstoffatom stehen, und
- L für eine lineare, zweiwertige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht.

4. Verfahren nach einem der Ansprüche 1 bis 3, **dadurch gekennzeichnet, dass** das Mittel (a) mindestens eine organische Siliciumverbindung (a1) der Formel (I) enthält, die ausgewählt ist aus der Gruppe aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan.
- (2-Dimethylaminoethyl)trimethoxysilan und/oder
- 1-(2-Dimethylaminoethyl)silantriol.

5. Verfahren nach einem der Ansprüche 2 bis 4, **dadurch gekennzeichnet, dass** das Mittel (a) mindestens eine organische Siliciumverbindung (a1) der Formel (II) enthält,
(R₅O)_{c}(R₆)_{d}Si-(A)ₑ-[NR₇-(A')]_{f}-[O-(A")]_{g}-[NR₈-(A‴)]ₕ-Si(R₆')_{d'} (OR₅')_{c'} (II),
wobei
- e und f beide für die Zahl 1 stehen,
- g und h beide für die Zahl 0 stehen,
- A und A' unabhängig voneinander für eine lineare, zweiwertige C₁-C₆-Alkylengruppe stehen
und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

6. Verfahren nach einem der Ansprüche 2 bis 5, **dadurch gekennzeichnet, dass** das Mittel (a) mindestens eine organische Siliciumverbindung (a1) der Formel (II) enthält, die ausgewählt ist aus der Gruppe aus
- 3-(Trimethoxysilyl)-N-[3-(trimethoxysilyl)propyl]-1-propanamin
- 3-(Triethoxysilyl)-N-[3-(triethoxysilyl)propyl]-1-propanamin
- N-Methyl-3-(trimethoxysilyl)-N-[3-(trimethoxysilyl)propyl]-1 -propanamin
- N-Methyl-3-(triethoxysilyl)-N-[3-(triethoxysilyl)propyl]-1-propanamin
- 2-[Bis[3-(trimethoxysilyl)propyl]amino]-ethanol
- 2-[Bis[3-(triethoxysilyl)propyl]amino]-ethanol
- 3-(Trimethoxysilyl)-N,N-bis[3-(trimethoxysilyl)propyl]-1-Propanamin
- 3-(Triethoxysilyl)-N,N-bis[3-(triethoxysilyl)propyl]-1-Propanamin
- N1,N1-Bis[3-(trimethoxysilyl)propyl]-1,2-Ethanediamin,
- N1,N1-Bis[3-(triethoxysilyl)propyl]-1,2-Ethanediamin,
- N,N-Bis[3-(trimethoxysilyl)propyl]-2-Propen-1-amin und/oder
- N,N-Bis[3-(triethoxysilyl)propyl]-2-Propen-1-amin.

7. Verfahren nach einem der Ansprüche 1 bis 6, **dadurch gekennzeichnet, dass** das Mittel (a) mindestens eine organische Siliciumverbindung (a1) der Formel (IV) enthält, die ausgewählt ist aus der Gruppe aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan,
- Dodecyltriethoxysilan,
- Octadecyltrimethoxysilan,
- Octadecyltriethoxysilan und
- Mischungen daraus.

8. Verfahren nach einem der Ansprüche 1 bis 7, **dadurch gekennzeichnet, dass** das Substratplättchen Aluminium umfasst.

9. Verfahren nach einem der Ansprüche 1 bis 8, **dadurch gekennzeichnet, dass** das Metallalkoxid ein Siliciumalkoxid umfasst, welches vorzugsweise ausgewählt aus der Gruppe bestehend aus Tetramethylorthosilikat, Tetraethylorthosilikat, Tetraisopropylorthosilikat und Mischungen daraus.

10. Verfahren nach einem der Ansprüche 1 bis 9, **dadurch gekennzeichnet, dass** die siliciumorganischen Verbindung mit einer basischen Gruppe ein Silan mit einem, zwei oder drei Siliciumatomen umfasst, welches außerdem eine oder mehrere basische chemische Gruppen und eine oder mehrere Hydroxylgruppen oder hydrolysierbare Gruppen pro Molekül umfasst.

11. Verfahren nach einem der Ansprüche 1 bis 10, **dadurch gekennzeichnet, dass** die siliciumorganischen Verbindung mit einer basischen Gruppe ausgewählt ist aus der Gruppe bestehend aus
- (3-Aminopropyl)triethoxysilan
- (3-Aminopropyl)trimethoxysilan
- 1-(3-Aminopropyl)silantriol
- (2-Aminoethyl)triethoxysilan
- (2-Aminoethyl)trimethoxysilan
- 1-(2-Aminoethyl)silantriol
- (3-Dimethylaminopropyl)triethoxysilan
- (3-Dimethylaminopropyl)trimethoxysilan
- 1-(3-Dimethylaminopropyl)silantriol
- (2-Dimethylaminoethyl)triethoxysilan
- (2-Dimethylaminoethyl)trimethoxysilan
- 1-(2-Dimethylaminoethyl)silantriol und
- Mischungen daraus.

12. Verfahren nach einem der Ansprüche 1 bis 11, **dadurch gekennzeichnet, dass** das Mittel (a) mindestens eine weitere farbgebende Verbindung (a2) enthält, welche vorzugsweise ausgewählt ist aus der Gruppe der farbigen Metalloxide, Metallhydroxide, Metalloxidhydrate, Silicate, Metallsulfide, komplexen Metallcyanide, Metallsulfate, Bronzepigmente und/oder aus farbigen Pigmenten auf Mica- oder Glimmerbasis, die mit mindestens einem Metalloxid und/oder einem Metalloxychlorid beschichtet sind.

13. Mehrkomponenten-Verpackungseinheit (Kit-of-parts) zum Färben von keratinischem Material, umfassend getrennt voneinander konfektioniert
- einen ersten Container mit einem Mittel (a'), wobei das Mittel (a') enthält:
(a1) mindestens eine organische Siliciumverbindung der Formel (I) und mindestens eine organische Siliciumverbindung der Formel (IV),
wobei in der organischen Siliciumverbindung der Formel (I)
R₁R₂N-L-Si(OR₃)ₐ(R₄)_{b} (I),
- R₁, R₂ unabhängig voneinander für ein Wasserstoffatom oder eine C₁-C₆-Alkylgruppe stehen,
- L für eine lineare oder verzweigte, zweiwertige C₁-C₂₀-Alkylengruppe steht,
- R₃ für ein Wasserstoffatom oder für eine C₁-C₆-Alkylgruppe steht,
- R₄ für eine C₁-C₆-Alkylgruppe steht,
- a, für eine ganze Zahl von 1 bis 3 steht, und
- b für die ganze Zahl 3 - a steht, und
wobei in der organischen Siliciumverbindung der Formel (IV)
R₉Si(OR₁₀)ₖ(R₁₁)ₘ (IV),
- R₉ für eine C₁-C₁₈-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht,, und
- einen zweiten Container mit einem Mittel (a"), wobei das Mittel (a") enthält:
(a2) mindestens eine farbgebende Verbindung, umfassend mindestens ein Effektpigment, umfassend α) ein Substratplättchen und β) eine Beschichtung, wobei die Beschichtung mindestens eine Schicht aufweist, die nass-chemisch unter Verwendung eines Metallalkoxids und einer siliciumorganischen Verbindung mit einer basischen Gruppe hergestellt worden ist,
- einen dritten Container mit einem Mittel (b), wobei das Mittel (b) enthält:
(b1) mindestens ein Versiegelungsreagenz, umfassend eine Verbindung ausgewählt aus der Gruppe bestehend aus filmbildenden Polymeren, Alkalisierungsmitteln, Acidifizierungsmittel und Mischungen daraus.

## Claims

1. Method for dyeing keratinous material, in particular human hair, comprising the following steps:
- applying an agent (a) to the keratinous material, wherein the agent (a) contains:
(a1) at least one organic silicon compound of formula (I) and at least one organic silicon compound of formula (IV),
wherein, in the organic silicon compound of formula (I)
R₁ R₂ N-L-Si(OR₃)ₐ (R₄)_{b} (I),
- R₁, R₂ independently of one another represent a hydrogen atom or a C₁ -C₆ alkyl group,
- L represents a linear or branched, divalent C₁-C₂₀alkylene group,
- R₃ represents a hydrogen atom or a C₁ -C₆ alkyl group,
- R₄ represents a C₁ -C₆ -alkyl group,
- a is an integer from 1 to 3, and
- b is the integer 3 - a, and
wherein, in the organic silicon compound of formula (IV)
R₉ Si(OR₁₀)ₖ (R₁₁)ₘ (IV),
- R₉ represents a C₁ -C₁₈ alkyl group,
- R₁₀ represents a hydrogen atom or a C₁ -C₆ alkyl group,
- R₁₁ represents a C₁ -C₆ alkyl group
- k is an integer from 1 to 3, and
- m is the integer 3 - k, and
(a2) at least one coloring compound containing at least one effect pigment ( ) comprising α) a substrate plate and β) a coating, wherein the coating has at least one layer which has been produced by wet chemical means using a metal alkoxide and an organosilicon compound with a basic group, and
- applying an agent (b) to the keratinous material, wherein the agent (b) comprises:
(b1) at least one sealing reagent comprising a compound selected from the group consisting of film-forming polymers, alkalizing agents, acidifying agents, and mixtures thereof.

2. Method according to claim 1, **characterized in that** agent (a) further contains at least one organic silicon compound (a1) of formula (II)
wherein, in the organic silicon compound of formula (II)
(R₅O)_{c}(R₆)_{d}Si-(A)ₑ-[NR₇-(A')]_{f} -[O-(A")]_{g} -[NR₈ -(A‴)]ₕ -Si(R₆')_{d'} (OR₅')_{c'} (II),
- R5, R5', R5", R6, R6' and R6" independently represent a C₁ -C₆ alkyl group,
- A, A', A", A‴ and Aʺʺ independently represent a linear or branched, divalent C₁-C₂₀-alkylene group,
- R₇ and R₈ independently represent a hydrogen atom, a C₁ -C₆ -alkyl group, a hydroxy-C₁-C₆-alkyl group, a C₂-C₆-alkenyl group, an amino-C₁-C₆-alkyl group or a group of formula (III)
- (Aʺʺ)-Si(R₆")_{d}"(OR₅")_{c}" (III),
- c represents an integer from 1 to 3,
- d represents the integer 3 - c,
- c' is an integer from 1 to 3,
- d' represents the integer 3 - c',
- c" represents an integer from 1 to 3,
- d" stands for the integer 3 - c",
- e stands for 0 or 1,
- f stands for 0 or 1,
- g stands for 0 or 1,
- h stands for 0 or 1,
provided that at least one of the residues e, f, g, and h is not equal to 0.

3. Method according to one of claims 1 to 2, **characterized in that** the agent (a) contains at least one organic silicon compound (a1) of formula (I),
R₁ R₂ N-L-Si(OR₃)ₐ (R₄)_{b} (I),
where
- R₁ and R₂ both represent a hydrogen atom, and
- L represents a linear, divalent C₁-C₆alkylene group, preferably a propylene group (-CH₂-CH₂-CH₂) or an ethylene group (-CH₂-CH₂-),
- R₃ and R₄ independently of one another represent a methyl group or an ethyl group,
- a represents the number 3, and
- b represents the number 0.

4. Method according to one of claims 1 to 3, **characterized in that** the agent (a) contains at least one organic silicon compound (a1) of formula (I) selected from the group consisting of
- (3-aminopropyl)triethoxysilane
- (3-aminopropyl)trimethoxysilane
- 1-(3-aminopropyl)silantriol
- (2-aminoethyl)triethoxysilane
- (2-aminoethyl)trimethoxysilane
- 1-(2-aminoethyl)silane triol
- (3-Dimethylaminopropyl)triethoxysilane
- (3-Dimethylaminopropyl)trimethoxysilane
- 1-(3-dimethylaminopropyl)silane triol
- (2-Dimethylaminoethyl)triethoxysilane.
- (2-Dimethylaminoethyl)trimethoxysilane and/or
- 1-(2-dimethylaminoethyl)silantriol.

5. Method according to one of claims 2 to 4, **characterized in that** the agent (a) contains at least one organic silicon compound (a1) of formula (II),
(R₅O)_{c}(R₆)_{d}Si-(A)ₑ-[NR₇-(A')]_{f} -[O-(A")]g -[NR₈ -(A‴)]ₕ -Si(R₆')_{d}(OR₅')_{c'} (II),
where
- e and f both represent the number 1,
- g and h both represent the number 0,
- A and A' independently represent a linear, divalent C₁-C₆alkylene group, and
- R7 represents a hydrogen atom, a methyl group, a 2-hydroxyethyl group, a 2-alkenyl group, a 2-aminoethyl group or a group of formula (III).

6. Method according to one of claims 2 to 5, **characterized in that** the agent (a) contains at least one organic silicon compound (a1) of formula (II) selected from the group consisting of
- 3-(trimethoxysilyl)-N-[3-(trimethoxysilyl)propyl]-1-propanamine
- 3-(triethoxysilyl)-N-[3-(triethoxysilyl)propyl]-1-propanamine
- N-methyl-3-(trimethoxysilyl)-N-[3-(trimethoxysilyl)propyl]-1-propanamine
- N-Methyl-3-(triethoxysilyl)-N-[3-(triethoxysilyl)propyl]-1-propanamine
- 2-[Bis[3-(trimethoxysilyl)propyl]amino]ethanol
- 2-[Bis[3-(triethoxysilyl)propyl]amino]-ethanol
- 3-(trimethoxysilyl)-N,N-bis[3-(trimethoxysilyl)propyl]-1-propanamine
- 3-(Triethoxysilyl)-N,N-bis[3-(triethoxysilyl)propyl]-1-propanamine
- N1,N1-Bis[3-(trimethoxysilyl)propyl]-1,2-ethanediamine,
- N1,N1-Bis[3-(triethoxysilyl)propyl]-1,2-ethanediamine,
- N,N-Bis[3-(trimethoxysilyl)propyl]-2-propen-1-amine and/or
- N,N-Bis[3-(triethoxysilyl)propyl]-2-propen-1-amine.

7. Method according to one of claims 1 to 6, **characterized in that** the agent (a) contains at least one organic silicon compound (a1) of formula (IV), which is selected from the group consisting of
- methyltrimethoxysilane
- Methyltriethoxysilane
- Ethyltrimethoxysilane
- Ethyltriethoxysilane
- Propyltrimethoxysilane
- Propyltriethoxysilane
- Hexyltrimethoxysilane
- Hexyltriethoxysilane
- Octyltrimethoxysilane
- Octyltriethoxysilane
- Dodecyltrimethoxysilane,
- Dodecyltriethoxysilane,
- Octadecyltrimethoxysilane,
- Octadecyltriethoxysilane and
- mixtures thereof.

8. Method according to one of claims 1 to 7, **characterized in that** the substrate wafer comprises aluminum.

9. Method according to one of claims 1 to 8, **characterized in that** the metal alkoxide comprises a silicon alkoxide, which is preferably selected from the group consisting of tetramethyl orthosilicate, tetraethyl orthosilicate, tetraisopropyl orthosilicate, and mixtures thereof.

10. Method according to one of claims 1 to 9, **characterized in that** the organosilicon compound with a basic group comprises a silane with one, two, or three silicon atoms, which also comprises one or more basic chemical groups and one or more hydroxyl groups or hydrolyzable groups per molecule.

11. Method according to one of claims 1 to 10, **characterized in that** the organosilicon compound with a basic group is selected from the group consisting of
- (3-aminopropyl)triethoxysilane
- (3-aminopropyl)trimethoxysilane
- 1-(3-aminopropyl)silantriol
- (2-aminoethyl)triethoxysilane
- (2-aminoethyl)trimethoxysilane
- 1-(2-aminoethyl)silane triol
- (3-Dimethylaminopropyl)triethoxysilane
- (3-Dimethylaminopropyl)trimethoxysilane
- 1-(3-dimethylaminopropyl)silane triol
- (2-Dimethylaminoethyl)triethoxysilane
- (2-Dimethylaminoethyl)trimethoxysilane
- 1-(2-dimethylaminoethyl)silane triol and
- mixtures thereof.

12. Method according to one of claims 1 to 11, **characterized in that** the agent (a) contains at least one further coloring compound (a2), which is preferably selected from the group of colored metal oxides, metal hydroxides, metal oxide hydrates, silicates, metal sulfides, complex metal cyanides, metal sulfates, bronze pigments and/or colored pigments based on mica or glimmer, which are coated with at least one metal oxide and/or one metal oxychloride.

13. Multi-component packaging unit (kit-of-parts) for coloring keratinous material, comprising separately packaged
- a first container with an agent (a'), wherein the agent (a') contains:
(a1) at least one organic silicon compound of formula (I) and at least one organic silicon compound of formula (IV),
wherein, in the organic silicon compound of formula (I)
R₁ R₂ N-L-Si(OR₃)ₐ(R₄)_{b} (I),
- R₁ and R₂ independently represent a hydrogen atom or a C₁ -C₆ alkyl group,
- L represents a linear or branched, divalent C₁-C₂₀alkylene group,
- R₃ represents a hydrogen atom or a C₁ -C₆ alkyl group,
- R₄ represents a C₁ -C₆ -alkyl group,
- a is an integer from 1 to 3, and
- b is the integer 3 - a, and
wherein, in the organic silicon compound of formula (IV)
R₉ Si(OR₁₀)ₖ (R₁₁ )ₘ (IV),
- R₉ represents a C₁ -C₁₈ alkyl group,
- R₁₀ represents a hydrogen atom or a C₁ -C₆ alkyl group,
- R₁₁ represents a C₁ -C₆ alkyl group
- k is an integer from 1 to 3, and
- m represents the integer 3 - k, and
- a second container with an agent (a"), wherein the agent (a") contains:
(a2) at least one coloring compound comprising at least one effect pigment comprising α) a substrate platelet and β) a coating, wherein the coating comprises at least one layer which has been produced by wet chemistry using a metal alkoxide and an organosilicon compound with a basic group,
- a third container containing an agent (b), wherein the agent (b) comprises:
(b1) at least one sealing reagent comprising a compound selected from the group consisting of film-forming polymers, alkalizing agents, acidifying agents, and mixtures thereof.

## Revendications

1. Procédé de coloration de matière kératinique, en particulier de cheveux humains, comprenant les étapes suivantes :
- application d'un agent (a) sur la matière kératinique, l'agent (a) contenant :
(a1) au moins un composé organique du silicium de formule (I) et au moins un composé organique du silicium de formule (IV),
dans lesquels, dans le composé organique du silicium de formule (I)
R₁R₂N-L-Si(OR₃)ₐ(R₄)_{b} (I),
- R₁, R₂ représentent indépendamment l'un de l'autre un atome d'hydrogène ou un groupe alkyle en C₁ -C₆,
- L représente un groupe alkylène en C₁ -C₂₀ divalent, linéaire ou ramifié,
- R₃ représente un atome d'hydrogène ou un groupe alkyle en C₁ -C₆,
- R₄ représente un groupe alkyle en C₁ -C₆,
- a représente un nombre entier compris entre 1 et 3, et
- b représente le nombre entier 3 - a, et
où, dans le composé organique du silicium de formule (IV)
R₉Si(OR₁₀)ₖ(R₁₁)ₘ (IV),
- R₉ représente un groupe alkyle en C₁ -C₁₈,
- R₁₀ représente un atome d'hydrogène ou un groupe alkyle en C₁ -C₆,
- R₁₁ représente un groupe alkyle en C₁ -C₆
- k représente un nombre entier compris entre 1 et 3, et
- m représente le nombre entier 3 - k, et
(a2) au moins un composé colorant contenant au moins un pigment à effet, comprenant α) une plaquette de substrat et β) un revêtement, le revêtement comportant au moins une couche qui a été préparée par voie chimique humide à l'aide d'un alcoxyde métallique et d'un composé organosilicié comportant un groupe basique , et
- application d'un agent (b) sur le matériau kératinique, l'agent (b) contenant :
(b1) au moins un réactif de scellement comprenant un composé choisi dans le groupe constitué par les polymères filmogènes, les agents alcalinisants, les agents acidifiants et leurs mélanges.

2. Procédé selon la revendication 1, **caractérisé en ce que** l'agent (a) contient en outre au moins un composé organique du silicium (a1) de formule (II)
dans lequel, dans le composé organique du silicium de formule (II)
(R₅ O)_{c} (R₆ )_{d} Si-(A)ₑ -[NR₇ -(A')]_{f} -[O-(A")]_{g} -[NR₈ -(A‴)]ₕ -Si(R₆')_{d'}(OR₅')_{c'} (II),
- R5, R5', R5", R6, R6' et R6" représentent indépendamment les uns des autres un groupe alkyle en C₁ -C₆,
- A, A', A", A‴ et Aʺʺ représentent indépendamment les uns des autres un groupe alkylène C₁ -C₂₀ divalent, linéaire ou ramifié,
- R₇ et R₈ représentent indépendamment l'un de l'autre un atome d'hydrogène, un groupe alkyle ₑₙ C₁ -C₆, un groupe hydroxy-alkyle en C₍₂) -C(6₎ , un groupe amino-alkyle en C₁ - C₆, un groupe alcényle en C(2) -C(6) , un groupe aryle, un groupe aralkyle, un groupe aryloxyalkyle, un groupe arylalkyle, un groupe aryloxyalkyle, un groupe arylalkyle, un groupe aryloxyalkyle₁ -C₆ -alkyle, un groupe alcényle ₑₙ C₂ -C₆, un groupe amino-alkyle ₑₙ C₁ -C₆ ou un groupement de formule (III)
- (Aʺʺ)-Si(R₆")_{d}"(OR₅'')_{c}'' (III),
- c représente un nombre entier compris entre 1 et 3,
- d représente le nombre entier 3 - c,
- c' représente un nombre entier compris entre 1 et 3,
- d" représente le nombre entier 3 - c'
- c" représente un nombre entier compris entre 1 et 3,
- d" représente le nombre entier 3 - c",
- e représente 0 ou 1,
- f représente 0 ou 1,
- g représente 0 ou 1,
- h représente 0 ou 1,
à condition qu'au moins l'un des restes e, f, g et h soit différent de 0.

3. Procédé selon l'une des revendications 1 à 2, **caractérisé en ce que** l'agent (a) contient au moins un composé organique du silicium (a1) de formule (I)
R₁R₂N-L-Si(OR₃)ₐ(R₄)_{b} (I),
où
- R₁ et R₂ représentent tous deux un atome d'hydrogène, et
- L représente un groupe alkylène linéaire divalent en C₁à C₆, de préférence un groupe propylène (-CH₂-CH₂-CH₂-) ou un groupe éthylène (-CH₂-CH₂-),
- R₃ et R₄ représentent indépendamment l'un de l'autre un groupe méthyle ou un groupe éthyle,
- a représente le nombre 3 et
- b représente le nombre 0.

4. Procédé selon l'une des revendications 1 à 3, **caractérisé en ce que** l'agent (a) contient au moins un composé organique du silicium (a1) de formule (I) choisi dans le groupe constitué par
- (3-aminopropyl)triéthoxysilane
- (3-aminopropyl)triméthoxysilane
- 1-(3-aminopropyl)silantriol
- (2-aminoéthyl)triéthoxysilane
- (2-aminoéthyl)triméthoxysilane
- 1-(2-aminoéthyl)silantriol
- (3-diméthylaminopropyl)triéthoxysilane
- (3-diméthylaminopropyl)triméthoxysilane
- 1-(3-diméthylaminopropyl)silantriol
- (2-diméthylaminoéthyl)triéthoxysilane.
- (2-diméthylaminoéthyl)triméthoxysilane et/ou
- 1-(2-diméthylaminoéthyl)silantriol.

5. Procédé selon l'une des revendications 2 à 4, **caractérisé en ce que** l'agent (a) contient au moins un composé organique du silicium (a1) de formule (II):
(R₅O)_{c}(R₆)_{d}Si-(A)ₑ-[NR₇ -(A')]_{f}-[O-(A")]_{g}-[NR₈ -(A‴)]ₕ-Si(R₆')_{d'}(OR₅')_{c'} (II),
où
- e et f représentent tous deux le chiffre 1,
- g et h représentent tous deux le chiffre 0,
- A et A' représentent indépendamment l'un de l'autre un groupe alkylène linéaire divalent en C₁ -C₆ et
- R7 représente un atome d'hydrogène, un groupe méthyle, un groupe 2-hydroxyéthyle, un groupe 2-alcényle, un groupe 2-aminoéthyle ou un groupement de formule (III).

6. Procédé selon l'une des revendications 2 à 5, **caractérisé en ce que** l'agent (a) contient au moins un composé organique du silicium (a1) de formule (II) choisi dans le groupe constitué par
- 3-(triméthoxysilyl)-N-[3-(triméthoxysilyl)propyl]-1-propanamine
- 3-(triéthoxysilyl)-N-[3-(triéthoxysilyl)propyl]-1-propanamine
- N-méthyl-3-(triméthoxysilyl)-N-[3-(triméthoxysilyl)propyl]-1-propanamine
- N-méthyl-3-(triéthoxysilyl)-N-[3-(triéthoxysilyl)propyl]-1-propanamine
- 2-[bis[3-(triméthoxysilyl)propyl]amino]-éthanol
- 2-[bis[3-(triéthoxysilyl)propyl]amino]-éthanol
- 3-(triméthoxysilyl)-N,N-bis[3-(triméthoxysilyl)propyl]-1-propanamine
- 3-(triéthoxysilyl)-N,N-bis[3-(triéthoxysilyl)propyl]-1-propanamine
- N1,N1-bis[3-(triméthoxysilyl)propyl]-1,2-éthanediamine,
- N1,N1-bis[3-(triéthoxysilyl)propyl]-1,2-éthanediamine,
- N,N-bis[3-(triméthoxysilyl)propyl]-2-propène-1-amine et/ou
- N,N-bis[3-(triéthoxysilyl)propyl]-2-propène-1-amine.

7. Procédé selon l'une des revendications 1 à 6, **caractérisé en ce que** l'agent (a) contient au moins un composé organique du silicium (a1) de formule (IV) choisi dans le groupe constitué par
- méthyltriméthoxysilane
- méthyltriéthoxysilane
- éthyl triméthoxysilane
- éthyl triéthoxysilane
- Propyltriméthoxysilane
- Propyltriethoxysilane
- Hexyltriméthoxysilane
- Hexyltriethoxysilane
- Octyltriméthoxysilane
- Octyltriethoxysilane
- Dodécyltriméthoxysilane,
- Dodécyltriéthoxysilane,
- octadécyltriméthoxysilane,
- octadécyltriéthoxysilane et
- leurs mélanges.

8. Procédé selon l'une des revendications 1 à 7, **caractérisé en ce que** la plaquette de substrat comprend de l'aluminium.

9. Procédé selon l'une des revendications 1 à 8, **caractérisé en ce que** l'alcoxyde métallique comprend un alcoxyde de silicium, de préférence choisi dans le groupe constitué par l'orthosilicate de tétraméthyle, l'orthosilicate de tétraéthyle, l'orthosilicate de tétraisopropyle et leurs mélanges.

10. Procédé selon l'une des revendications 1 à 9, **caractérisé en ce que** le composé organosilicié comportant un groupe basique comprend un silane comportant un, deux ou trois atomes de silicium, qui comprend en outre un ou plusieurs groupes chimiques basiques et un ou plusieurs groupes hydroxyle ou groupes hydrolysables par molécule.

11. Procédé selon l'une des revendications 1 à 10, **caractérisé en ce que** le composé organosilicié à groupe basique est choisi dans le groupe constitué par
- (3-aminopropyl)triéthoxysilane
- (3-aminopropyl)triméthoxysilane
- 1-(3-aminopropyl)silantriol
- (2-aminoéthyl)triéthoxysilane
- (2-aminoéthyl)triméthoxysilane
- 1-(2-aminoéthyl)silantriol
- (3-diméthylaminopropyl)triéthoxysilane
- (3-diméthylaminopropyl)triméthoxysilane
- 1-(3-diméthylaminopropyl)silantriol
- (2-diméthylaminoéthyl)triéthoxysilane
- (2-diméthylaminoéthyl)triméthoxysilane
- 1-(2-diméthylaminoéthyl)silantriol et
- leurs mélanges.

12. Procédé selon l'une des revendications 1 à 11, **caractérisé en ce que** l'agent (a) contient au moins un autre composé colorant (a2) qui est de préférence choisi dans le groupe des oxydes métalliques colorés, des hydroxydes métalliques, des hydrates d'oxydes métalliques, des silicates, sulfures métalliques, cyanures métalliques complexes, sulfates métalliques, pigments de bronze et/ou de pigments colorés à base de mica ou de micacée, qui sont recouverts d'au moins un oxyde métallique et/ou un oxychlorure métallique.

13. Unité d'emballage à plusieurs composants (kit de pièces) pour la coloration de matière kératinique, comprenant, confectionnés séparément les uns des autres
- un premier récipient contenant un agent (a'), l'agent (a') contenant :
(a1) au moins un composé organique du silicium de formule (I) et au moins un composé organique du silicium de formule (IV),
dans lequel, dans le composé organique du silicium de formule (I)
R₁R₂N-L-Si(OR₃)ₐ(R₄)_{b} (I),
- R₁ et R₂ représentent indépendamment l'un de l'autre un atome d'hydrogène ou un groupe alkyle en C₁ -C₆,
- L représente un groupe alkylène en C₁ -C₂₀ divalent, linéaire ou ramifié,
- R₃ représente un atome d'hydrogène ou un groupe alkyle en C₁ -C₆ ,
- R₄ représente un groupe alkyle en C₁ -C₆,
- a représente un nombre entier de 1 à 3, et
- b représente le nombre entier 3 - a, et
où, dans le composé organique du silicium de formule (IV)
R₉Si(OR₁₀)ₖ (R₁₁)ₘ (IV),
- R₉ représente un groupe alkyle en C₁-C₁₈,
- R₁₀ représente un atome d'hydrogène ou un groupe alkyle en C₁ -C₆,
- R₁₁ représente un groupe alkyle en C₁-C₆
- k représente un nombre entier compris entre 1 et 3, et
- m représente le nombre entier 3 - k, et
- un deuxième récipient contenant un agent (a"), l'agent (a") contenant :
(a2) au moins un composé colorant comprenant au moins un pigment à effet comprenant α) une plaquette de substrat et β) un revêtement, le revêtement d' comprenant au moins une couche qui a été préparée par voie chimique humide à l'aide d'un alcoxyde métallique et d'un composé organosilicié comportant un groupe basique,
- un troisième récipient contenant un agent (b), l'agent (b) contenant :
(b1) au moins un réactif de scellement comprenant un composé choisi dans le groupe constitué par les polymères filmogènes, les agents alcalinisants, les agents acidifiants et leurs mélanges.
